(19) **Europäisches Patentamt**
**European Patent Office**
**Office européen des brevets**

(11) **EP 1 438 571 B1**

(12) **EUROPÄISCHE PATENTSCHRIFT**

(45) Veröffentlichungstag und Bekanntmachung des Hinweises auf die Patenterteilung:
**08.09.2010 Patentblatt 2010/36**

(21) Anmeldenummer: **02774406.9**

(22) Anmeldetag: **25.09.2002**

(51) Int Cl.:
***G01N 27/414*** (2006.01)

(86) Internationale Anmeldenummer:
**PCT/DE2002/003613**

(87) Internationale Veröffentlichungsnummer:
**WO 2003/038420 (08.05.2003 Gazette 2003/19)**

(54) **BIOSENSOR-SCHALTKREIS UND EIN AUS EINER MEHRZAHL DIESER BIOSENSOR-SCHALTKREISE BESTEHENDER SENSOR-ARRAY UND BIOSENSOR-ARRAY**

BIOSENSOR CIRCUIT AND SENSOR ARRAY CONSISTING OF A PLURALITY OF SAID BIOSENSOR CIRCUITS AND BIOSENSOR ARRAY

CIRCUIT BIOCAPTEUR, ENSEMBLE DE CAPTEURS CONSTITUE D'UNE PLURALITE DE CIRCUITS BIOCAPTEURS, ET ENSEMBLE DE BIOCAPTEURS

(84) Benannte Vertragsstaaten:
**DE FR GB**

(30) Priorität: **16.10.2001 DE 10151020**

(43) Veröffentlichungstag der Anmeldung:
**21.07.2004 Patentblatt 2004/30**

(73) Patentinhaber: **Infineon Technologies AG**
**85579 Neubiberg (DE)**

(72) Erfinder:
• **EVERSMANN, Björn-Oliver**
**80336 München (DE)**
• **JENKNER, Martin**
**82152 Planegg (DE)**
• **PAULUS, Christian**
**82362 Weilheim (DE)**
• **THEWES, Roland**
**82194 Gröbenzell (DE)**

(74) Vertreter: **Kühn, Armin**
**Viering, Jentschura & Partner**
**Postfach 22 14 43**
**80504 München (DE)**

(56) Entgegenhaltungen:
WO-A-01/75462     DE-A- 3 926 657
US-A- 4 701 253     US-A- 5 309 085
US-A- 5 602 467

• KRAUSE M ET AL: "Extended gate electrode arrays for extracellular signal recordings" SENSORS AND ACTUATORS B, ELSEVIER SEQUOIA S.A., LAUSANNE, CH, Bd. 70, Nr. 1-3, 1. November 2000 (2000-11-01), Seiten 101-107, XP004224587 ISSN: 0925-4005
• BAUMANN W H ET AL: "Microelectronic sensor system for microphysiological application on living cells" SENS ACTUATORS, B CHEM; SENSORS AND ACTUATORS, B: CHEMICAL 1999 ELSEVIER SEQUOIA SA, LAUSANNE, SWITZERLAND, Bd. 55, Nr. 1, 1999, Seiten 77-89, XP002228955

**Beschreibung**

Die Erfindung betrifft eine Schaltkreis-Anordnung, ein Sensor-Array und ein Biosensor-Array.

Die Netzwerkstruktur von Gehirnen höherer Tiere ist von hoher Komplexität und ist Gegenstand aktueller Forschung in der Neurobiologie. Ein Aspekt dieser Komplexität ist darin zu sehen, dass äußerlich erkennbare Funktionalitäten wie das Gedächtnis oder die Objektbildung erst durch die Vernetzung einzelner Nervenzellen miteinander realisiert wird. Für die neurobiologische Analyse hat dies zur Folge, dass zum Verständnis des Gehirns die Aktivität einer sehr großen Anzahl von Nervenzellen zu berücksichtigen ist. Aus experimentellen bzw. apparativen Gründen stoßen daher klassische Techniken zum Ableiten neuronaler Aktivität an ihre Grenzen. Beispielsweise ist das Erfassen elektrischer Signale einer Nervenzellen mittels einer Penetration in einzelne Zellen unter Verwendung von Glasmikroelektroden bei großen Zellverbänden mit zehntausenden oder gar Millionen von Neuronen selbst bei in-vitro Versuchen nicht mehr möglich.

Aus dem Stand der Technik sind Verfahren bekannt, um nichtinvasiv, das heißt anschaulich ohne Eindringen in die zu untersuchende Nervenzelle, das elektrische Potential einer Zelle oder eines Zellverbands sensoriell zu erfassen. Ein solches Konzept ist beispielsweise in [1] beschrieben. Gemäß diesem Konzept kann eine Vielzahl von Sensoren zeitlich simultan betrieben werden, um die Aktivität einer neurobiologischen Substanz aufzuzeichnen. Dabei wird ein Metallkontakt aus einem inerten Material wie beispielsweise Gold oder Platin als Sensor-Elektrode verwendet. Als Substrat wird gemäß dem Stand der Technik häufig Glas eingesetzt, da es optisch durchlässig ist und daher eine Überwachung der experimentellen Anordnung mit einem Durchlichtmikroskop ermöglicht. Allerdings weist die Verwendung von Glas als Substrat für ein Sensor-Array den Nachteil auf, dass die erreichbaren Strukturdimensionen nicht ausreichend gering sind und dass daher eine ausreichend hohe Ortsauflösung der Aktivität von Nervenzellen nicht erreichbar ist.

Eine Sensor-Anordnungen mit metallischen Elektroden wird häufig als Multi-Elektroden-Array (MEA) bezeichnet.

Ein Multi-Elektroden-Array weist einen bekannten und häufig konstanten Abstand zwischen benachbarten Sensor-Elektroden des Arrays auf, so dass es Neurobiologen ermöglicht ist, eine sogenannte "Karte" von neuronaler Aktivität anzufertigen. Dabei können biologische Proben wie zum Beispiel Hirnproben verwendet werden, in denen die Vernetzung der Neuronen durch die Präparation nicht verändert ist. Prinzipiell weist ein Multi-Elektroden-Array den Vorteil auf, dass die Anzahl der aufzeichnenden Elektroden ausreichend hoch gewählt werden kann, so dass sich beispielsweise bei zueinander ähnlichen, aber nicht miteinander vernetzten Zellen statistische Eigenschaften von Nervenzellen erfassen lassen.

Die Funktion einer Nervenzelle ist für praktische Anwendungen als biochemisch-elektrischer Signalwandler interessant. Die Aktivität neuronaler Zellen wird durch bestimmte Stoffe selektiv beeinflusst, wobei vorteilhaft ist, dass viele solcher Stoffe wasserlöslich sind. Zu den die Aktivität eines Neurons beeinflussenden Molekülen gehören insbesondere Neurotransmitter, die Gegenstand vieler pharmakologischer Untersuchungen sind. Insbesondere sind Multi-Elektroden-Arrays mit darauf kultivierten Nervenzellen, insbesondere aus einem Rattenhirn, zu idealen Experimentierobjekten für die Entwicklung von Pharmaka geworden. Vorteile liegen in der guten experimentellen Handhabung und in vielversprechenden Perspektiven für Langzeitstudien. Wesentlich ist für eine solche Anwendung die zweidimensionale Struktur eines Pharma-Sensors aus Nervenzellen und einer Sensor-Anordnung.

Eine andere Anwendung eines gekoppelten Nervenzellen-SensorSystems ist es, toxische Substanzen zu detektieren. Biosensoren zeichnen sich durch einen hohen Grad von Spezifität auf. Nervenzellen beispielsweise sind vorwiegend auf solche Stoffe sensitiv, die für ihren Metabolismus

(Stoffwechsel) relevant sind. Ein wichtiger Einsatzbereich von Biosensoren ist daher das Umweltmonitoring, das heißt das Erfassen von Umweltparametern, insbesondere von Giftstoffen. Aber auch auf militärischem und sicherheitstechnischem Gebiet sind Biosensoren aufgrund des beschriebenen Aspekts einsatzfähig. Ein solches Konzept ist beispielsweise in [2] beschrieben.

Es ist allerdings zu betonen, dass das Einsatzgebiet von Biosensoren, insbesondere der erfindungsgemäßen Schaltkreis-Anordnung, des erfindungsgemäßen Sensor-Arrays und des erfindungsgemäßen Biosensor-Arrays nicht auf Anwendungen mit Nervenzellen beschränkt sind.

Auf den beschriebenen Einsatzgebieten bzw. auf anderen Einsatzgebieten sind die folgenden Anforderungen an Sensor-Anordnungen zu stellen: Es sollen ausreichend viele Sensoren simultan zu betreiben sein, um zu ermöglichen, eine Momentanaufnahme der Potentialverhältnisse auf der aktiven Oberfläche der Sensor-Anordnung zu erhalten. Ferner ist der Abstand von Sensor-Elementen voneinander bzw. die räumliche Ausdehnung eines Sensor-Elements ausreichend klein zu wählen (typischerweise 10•m bis einige 10•m), um eine ausreichend gute Ortsauflösung zu erhalten. Eine weitere wichtige Anforderung an solche Sensoren besteht darin, dass die Ausgangssignale zweier beliebiger Sensoren eines Multi-Elektroden-Arrays bei gleichen Eingangssignalen ebenfalls gleich sein müssen. Dies bedeutet insbesondere, dass keine statischen Differenzen in den Ausgangssignalen der Sensor-Elemente (Offsets) auftreten dürfen, die beispielsweise auf Prozessschwankungen während des Herstellens der Sensor-Elemente beruhen können.

Lösungsansätze zum Ausbilden von Sensoren mit den gewünschten Eigenschaften sind einerseits Sensor-Anordnungen mit sogenannten IGFETs (Insulated Gate-Feldeffekttransistoren) und andererseits die bereits angesprochenen Multi-Elektroden-Arrays (MEA).

Ein solcher FET ist von seinem Grundprinzip her ähnlich wie ein Metall-Isolator-Halbleiter-Feldeffekttransistor (MISFET) aufgebaut. Er unterscheidet sich von einem herkömmlichen MISFET dadurch, dass die Leitfähigkeit des Kanalbereichs des Transistors nicht mittels einer Metallelektrode, sondern mittels elektrischer oder elektrochemischer Vorgänge innerhalb eines Elektrolyten oberhalb des Dielektrikums gesteuert wird, wobei das Dielektrikum optional auch Ladungen aus dem Elektrolyten aufnehmen kann. Mit anderen Worten sind elektrisch geladene, zu erfassende Partikel (beispielsweise durch die Ionenkanäle von Nervenzellen hindurchtretende Ionen) über den Elektrolyten in Kontakt mit einer dielektrischen Schicht an der Oberfläche des Dielektrikums, wodurch eine rein kapazitive Kopplung zwischen den elektrisch geladenen, zu erfassenden Partikeln und dem Kanalbereich des FETs oder aber zwischen den elektrisch geladenen zu erfassenden Partikeln und der unterhalb der dielektrischen Schicht des FETs angeordneten Gate-Elektrode des FETs bewirkt wird. Mit anderen Worten wirkt die dielektrische Schicht wie das Dielektrikum eines Kondensators, der zwischen den elektrisch geladenen Ionen und direkt dem Kanalbereich des FETs oder zwischen den elektrisch geladenen Ionen der Gate-Elektrode des FETs ausgebildet ist, wobei mittels dieser kapazitiven Kopplung (ohne ohmsche Anteile) der geladenen Partikel an der Oberfläche des FET-Sensors die Leitfähigkeit des FETs infolge eines Sensor-Ereignisses verändert wird, so dass der Wert des Stromflusses zwischen Source- und Drain-Anschluss des FETs ein Maß für das Sensor-Ereignis ist. Ein direkter ohmscher Kontakt, das heißt ein direktes Vordringen der Partikel an einen elektrisch leitfähigen Bereich des FETs, ist nicht möglich. Die Kopplung ist somit rein kapazitiv.

Ein alternatives Lösungskonzept zum Bereitstellen von Sensor-Anordnungen, die den oben genannten Anforderungen gerecht werden, sind die Multi-Elektroden-Arrays (MEAs). Multi-Elektroden-Arrays weisen eine elektrisch leitfähige Oberfläche, zumeist eine Metallelektrode, in direktem Wirkkontakt mit den ein Sensor-Ereignis auslösenden elektrisch geladenen Partikeln. Mit anderen Worten sind die elektrisch geladenen Partikel bei Multi-Elektroden-Arrays in direktem Wirkkontakt mit der Oberfläche einer Elektrode, so dass die Kopplung zwischen den zu erfassenden Partikeln und einer Sensor-Elektrode zumindest teilweise ohmscher Art ist. Zwar kann bei Multi-Elektroden-Arrays die Kopplung zwischen den zu erfassenden Partikeln und der Elektrode auch kapazitive Anteile aufweisen (an der Oberfläche einer Elektrode können sich sogenannte Helmholtz-Schichten, das heißt Schichten von Partikeln mit alternierend positiven bzw. negativen Ladungen) ausbilden, wichtig sind jedoch die ohmschen Anteile. Bei Multi-Elektroden-Arrays wird also der Ladungszustand eines Knotens direkt unterhalb der Metallelektrode direkt durch zu erfassende Partikel verändert.

Bei den Multi-Elektroden-Arrays sind wiederum zwei Konzepte zu unterscheiden: Optisch und elektrisch ansteuerbare MEAs.

Bei der Verwendung optisch adressierbarer Multi-Elektroden-Arrays sind Metallelektroden eines Multi-Elektroden-Arrays matrixförmig angeordnet. Dimensionen von aus dem Stand der Technik bekannten optisch adressierbaren Multi-Elektroden-Arrays weisen typischerweise 60 Zeilen und 60 Spalten auf, wobei die Anzahl der Sensor-Elemente sich aus dem Produkt der Zeilen mit den Spalten ergibt. Die Elektroden einer Spalte sind jeweils über einen Fotowiderstand an eine gemeinsame Spalten-Leitung angeschlossen. Die Auswahl einer Position innerhalb des Sensor-Feldes erfolgt beispielsweise, indem unter Verwendung eines Lasers der mit dieser Position assoziierte Fotowiderstand mittels eines Lichtpulses in einen elektrisch leitenden Zustand versetzt wird. Allerdings weist dieses Konzept den Nachteil auf, dass zu einem Zeitpunkt jeweils nur ein Sensor-Feld ausgewählt werden kann. Ferner weisen optische MEAs den Nachteil auf, dass sie teure und aufwendige Komponenten aufweisen. Auch ist der Aufbau derartiger Sensor-Anordnungen aufgrund der Verwendung makroskopischer Bauelemente, wie beispielsweise einer Laseranordnung, oft hoch, was einer angestrebten Miniaturisierung entgegen wirkt. Da optische MEAs gemäß dem Stand der Technik vorwiegend auf Basis eines Glas-Substrates ausgebildet werden, ist beispielsweise die Verwendung von aktiven Schalt- oder Verstärker-Einheiten, beispielsweise von Vorverstärkern direkt unterhalb der Elektrode, technologisch nicht möglich. Auch ist bei der Glas-Substrat-Technologie eine ausreichend geringe Dimensionierung der Sensor-Elemente und ein ausreichend geringer Abstand zwischen den Sensor-Elementen nicht möglich, so dass sowohl die zeitliche als auch die räumliche Auflösung der Sensor-Elemente verbesserungsbedürftig ist.

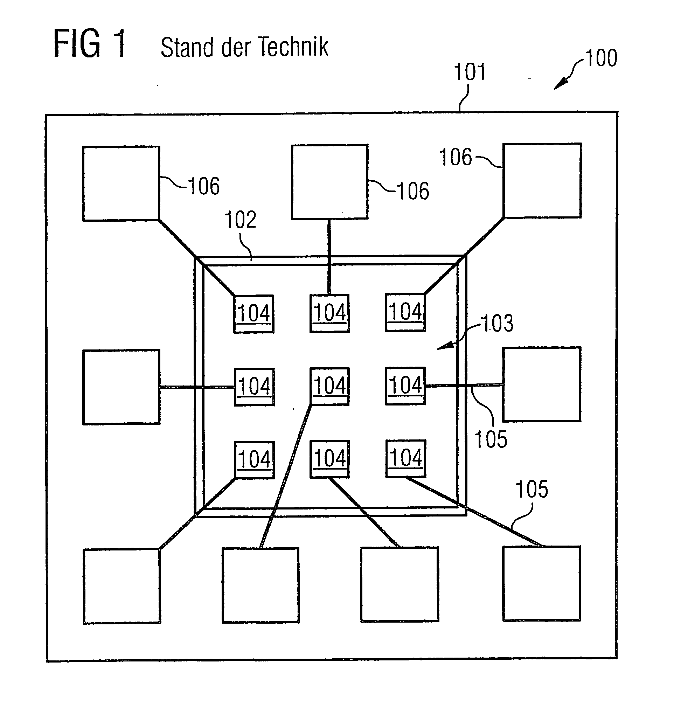

Ein Beispiel für einen aus dem Stand der Technik bekannten elektrisch adressierbaren MEA ist in **Fig.1** gezeigt. Der dort gezeigte elektrisch adressierbare MEA 100 ist auf einem Glassubstrat 101 ausgebildet. Mittels einer Begrenzungswand 102 ist ein aktiver Sensorbereich 103 im zentralen Bereich des elektrisch adressierbaren MEAs 100 ausgebildet. In dem aktiven Sensorbereich 103 ist eine Vielzahl von Sensor-Feldern 104 im Wesentlichen matrixförmig angeordnet, wobei die Sensor-Felder 104 derart eingerichtet sind, dass sie ein Sensor-Ereignis eines darüber angeordneten zu untersuchenden Objekts, beispielsweise einer darauf aufgebrachten Nervenzelle, erfassen können. Über elektrische Zuleitungen 105 werden die elektrischen Signale zu Kontaktflächen 106 im Randbereich des elektrisch adressierbaren MEAs 100 abgeleitet. Der Platzbedarf für die elektrischen Zuleitungen 105 ist sehr hoch. Dadurch ist die maximal erreichbare Anzahl von Sensor-Feldern 104 stark eingeschränkt. Die derzeitige technologische Grenze von bekannten MEAs liegt bei 64 Sensor-Feldern. Neben der stark beschränkten Anzahl maximal erreichbarer Sensor-Felder 104 infolge der separaten elektrischen Kontaktierung jedes einzelnen Sensor-Feldes 104 mittels elektrischer Zuleitungen 105 ist eine ausreichend gute räumliche Auflösung nicht erreichbar. Ferner ist zur Auswertung der an den Kontaktflächen 106 bereitgestellten Signale eine in Fig.1 nicht gezeigte aufwendige externe Auswerte-Elektronik erforderlich, die den Platzbedarf des elektrisch adressierbaren Multi-Elektroden-Arrays 100 erhöht. Ferner ist ein wesentlicher Nachteil der aus dem Stand der Technik bekannten elektrisch adressierbaren MEAs 100 darin zu sehen, dass das Erfordernis, dass die Ausgangssignale zweier unterschiedlicher Sensoren eines MEAs 100 bei gleichen Eingangssignalen ebenfalls gleich sind, oft nicht erfüllt ist. Dies beruht unter anderem auf Schwankungen in der Prozesstechnologie beim Ausbilden der einzelnen Sensor-Felder 104 und hat zur Folge, dass die Nachweissensitivität und die Verlässlichkeit der erhaltenen Sensor-Signale verbesserungsbedürftig ist. Mit anderen Worten weisen unterschiedliche Sensor-Felder 104 eines elektrisch adressierbaren MEAs 100 Schwankungen bezüglich des Wertes eines oder mehrere physikalischer Parameter der Sensor-Elemente 104 auf, beispielsweise als Folge von schwankenden Prozessbedingungen bei deren Herstellung, so dass eine eindeutige Zuordnung eines elektrischen Ausgangssignals zu einem Sensor-Signal an einem zugehörigen Sensor-Feld 104 nicht möglich ist.

Ferner ist aus [3] ein vollelektronisches Multi-Elektroden-Array mit einer elektronischen Positionsauswahl offenbart, das jedoch die genannte Anforderung, dass bei unterschiedlichen Sensor-Feldern einem definierten Eingangssignal ein eindeutiges Ausgangssignal zugeordnet sein soll, ebenfalls nicht erfüllt.

In [5] ist eine Kombination aus einer beheizten Lambda-Sonde mit sprungförmiger bzw. binärer Sensorcharakteristik mit einer weiteren beheizten Lambda-Sonde für die Bestimmung des Lambdawertes in einem Gasgemisch offenbart, wobei das Ausgangssignal der einen Lambda-Sonde zur Kalibrierung der anderen Lambda-Sonde dient.

In WO0175462 wird eine Biosensor-Array-Anordnung zum kapazitiven Erfassen eines Zustands eines Feld-Effekt-Transistors offenbart.

Krause, M et al. : "Extended gate electrode arrays for extracellular signal recordings" Sensor and Actuators B 70: 101-107 offenbart einen Bioelectrode-Array basierend auf Feld-Effekt-Transistoren, bei denen extrazelluläre Signale, beispielsweise von Neuronen oder Muskeln, abgegriffen werden können.

US5602467 offenbart eine Schaltungsanordnung zur kapazitiven Messung von Ionen in Lösungen, bei der die Schwellenspannung des Messtransistors kompensiert wird.

Bauman, W. H. et al. (1999) " Microelectronic sensor system for microphysiological application on living cells" Sensors and Actuators B 55: 77-89 offenbart einen Multi-ISFET-Chip zur Untersuchung von Zellen, bei dem die Schwellenspannung der einzelnen Feld-Effekt-Transistoren kompensiert werden kann.

Der Erfindung liegt das Problem zugrunde, ein elektronisches Multi-Elektroden-Array bereitzustellen, bei dem auch bei einer Veränderung bzw. einer Abweichung des Wertes eines physikalischen Parameters eines Sensor-Elements von einem Referenzwert, d.h. einem bei normalen, vorzugsweise vorgegebenen, Prozessbedingungen auftretendem Wert des physikalischen Parameters, ein von dem Sensor-Element generiertes Sensor-Signal von der Veränderung des Werts unabhängig ist.

Die Erfindung wird durch eine Schaltkreis-Anordnung, ein Sensor-Array und ein Biosensor-Array mit den Merkmalen gemäß den unabhängigen Patentansprüchen gelöst.

Die erfindungsgemäße Schaltkreis-Anordnung weist eine Schaltkreis-Anordnung gemäss Anspruch 1 auf.

Ferner ist erfindungsgemäß ein Sensor-Array mit einer Mehrzahl von in Kreuzungsbereichen von Zeilen- und Spalten-Leitungen im Wesentlichen matrixförmig angeordneten und mit in Zeilen- und Spalten-Leitungen verschalteten Schaltkreis-Anordnungen mit den oben genannten Merkmalen geschaffen.

Ferner ist ein Biosensor-Array mit einem Sensor-Array mit den oben genannten Merkmalen bereitgestellt.

Die erfindungsgemäße Schaltkreis-Anordnung weist insbesondere das Merkmal auf, dass die Komponenten der Schaltkreis-Anordnung, insbesondere die Sensor-Elemente und die Kalibrier-Einrichtung in ein Substrat, vorzugsweise ein halbleitertechnologisches Substrat integrierbar sind. Dies bewirkt eine Miniaturisierung der Anordnung, wodurch die räumliche Auflösung eines auf der erfindungsgemäßen Schaltkreis-Anordnung basierenden Sensor-Arrays verbessert ist. Ferner können übliche und damit ausgereifte Prozesse der Halbleitertechnologie erfindungsgemäß verwendet werden, um die Schaltkreis-Anordnung auszubilden. Dadurch ist ein wenig aufwendiges Herstellen der Schaltkreis-Anordnung möglich. Ein wichtiger Vorteil der erfindungsgemäßen Schaltkreis-Anordnung gegenüber dem Stand der Technik besteht darin, dass infolge der Kompensierung einer Veränderung des Werts des physikalischen Parameters des Sensor-Elements mittels der in und/oder auf dem Substrat ausgebildeten Kalibrier-Einrichtung eine eindeutige Zuordnung eines Sensor-Signals zu einem an dem zugehörigen Sensor-Element erfolgten Sensor-Ereignisses möglich ist, selbst wenn zwischen unterschiedlichen Sensor-Elementen beispielsweise infolge von Prozessschwankungen der jeweilige physikalische Parameter oder mehrere derartige physikalische Parameter einen unterschiedlichen Wert aufweisen. Das Sensor-Elemement weist einen Mess-Transistor auf, dessen Schwellenspannung oder andere Parameter infolge von Prozessschwankungen beim Ausbilden der Sensor-Elemente um einen Mittel- oder Referenz-Wert herum streuen. Sensor-Elemente mit einem unterschiedlichen Wert dieses physikalischen Parameters liefern gemäß dem Stand der Technik bei dem gleichen Sensor-Ereignis unterschiedliche Sensor-Signale. Die erfindungsgemäße Kalibrier-Einrichtung ist derart eingerichtet, dass dieser nachteilige Effekt gerade vermieden ist, indem eine Veränderung des Werts des physikalischen Parameters des Sensor-Elements zumindest teilweise kompensiert wird. Dadurch ist die Messgenauigkeit bzw. die Reproduzierbarkeit eines auf der erfindungsgemäßen Schaltkreis-Anordnung basierenden Sensor-Arrays erhöht, und eine all zu hohe Exaktheit ist bei der Herstellung der Sensor-Elemente des Sensor-Arrays entbehrlich.

Unter einem physikalischen Parameter wird im Rahmen dieser Beschreibung die Schwellenspannung eines Mess-Transistors des Sensor-Elements, verstanden, welcher Parameter bei einem bestimmten Sensor-Ereignis das daraus resultierende Sensor-Signal beeinflusst, wobei mittels der Kalibrier-Einrichtung diese Beeinflussung zumindest teilweise vermieden wird.

Das Sensor-Element der Schaltkreis-Anordnung weist eine mit der zu untersuchenden Substanz koppelbare elektrisch leitfähige Sensor-Elektrode auf.

Das Sensor-Element weist ferner einen Mess-Transistor auf, dessen Gate-Anschluss mit der elektrisch leitfähigen Sensor-Elektrode gekoppelt ist.

Der Mess-Transistor ist also vorzugsweise ein Feldeffekttransistor, insbesondere ein MOSFET. Es ist jedoch alternativ möglich, als Mess-Transistor des Sensor-Elements einen Bipolar-Transistor zu verwenden, wobei der Basis-Anschluss des Bipolar-Transistors die Funktionalität des Gate-Anschlusses eines Feldeffekttransistors und wobei die Emitter- und Kollektor-Anschlüsse des Bipolar-Transistors die Funktionalität der Source-/Drain-Anschlüsse des Feldeffekttransistors wahrnehmen. Indem als aktives Element des Sensor-Elements ein Mess-Transistor verwendet wird, ist ein hochsensitives und wenig aufwendiges Bauelement einer geringen Strukturdimension verwendet.

Weiter kann die erfindungsgemäße Schaltkreis-Anordnung eine Einrichtung zum Erfassen eines ein erfolgtes Sensor-Ereignis charakterisierenden elektrischen Parameters aufweisen, die mit einem ersten Source-/Drain-Anschluss des Mess-Transistors koppelbar ist. Diese Einrichtung kann insbesondere ein Voltmeter zum Erfassen einer elektrischen Spannung oder ein Amperemeter zum Erfassen eines elektrischen Stroms sein, es ist jedoch auch das Erfassen eines anderen elektrischen Parameters wie beispielsweise eines elektrischen Widerstands oder einer Frequenz möglich. Anschaulich wird infolge eines Sensor-Ereignisses der Ladungszustand des Gate-Anschlusses des Mess-Transistors beeinflusst, wodurch ein elektrischer Strom zwischen den beiden Source-/Drain-Anschlüssen des Mess-Transistors verändert wird. Ein mit diesem Stromfluss korrespondierendes elektrisches Signal, entweder der Stromfluss selbst oder eine damit zusammenhängende elektrische Spannung oder ein anderes elektrisches Signal, wird dann mittels der Einrichtung zum Erfassen dieses elektrischen Parameters erfasst.

Ferner kann die Kalibrier-Einrichtung derart eingerichtet sein, dass mit ihr das an den ersten oder einen zweiten Source-/Drain-Anschluss des Mess-Transistors angelegte elektrische Potential derart steuerbar ist, dass durch sie ein von einem Sensor-Ereignis bewirktes Sensor-Signal des Sensor-Elements auf einen Wert einstellbar ist, der von dem Wert des physikalischen Parameters des Sensor-Elements unabhängig ist.

Anschaulich wird einer der beiden Source-/Drain-Anschlüsse des Mess-Transistors infolge der Funktionalität der Kalibrier-Einrichtung auf ein derartiges elektrisches Potential gebracht, dass eine Veränderung des Werts des physikalischen Parameters dieses Sensor-Elements dadurch zumindest teilweise kompensiert wird und unterschiedliche Sensor-Elemente mit unterschiedlichen Werten dieses physikalischen Parameters bei einem gleichen Sensor-Ereignis ein gleiches oder im Wesentlichen gleiches Sensor-Signal erzeugen.

Die Kalibrierung erfolgt also über einen der beiden Source-/Drain-Anschlüsse des Mess-Transistors. Alternativ kann die Kalibrierung über den Substrat-Anschluss (Bulk-Anschluss) des Mess-Transistors erfolgen. In diesem Falle erhält jeder Mess-Transistor eine eigene, separat kontaktierbare Wanne, deren Potential beispielsweise mittels eines Source-Folgers eingestellt werden kann, wodurch die Übertragungseigenschaften des darin bzw. darauf ausgebildeten Mess-Transistors beeinflusst werden. Mit anderen Worten können alle elektrischen Knoten eines MOSFETs (erster Source-/Drain-Anschluss, zweiter Source-/Drain-Anschluss, Bulk-Anschluss) bis auf den Gate-Anschluss dazu dienen, auf ein solches Potential gebracht zu werden, dass dadurch das zugehörige Sensor-Element kalibriert ist bzw. die Veränderung des Wertes des physikalischen Parameters des Sensor-Elements zumindest teilweise kompensiert ist.

Insbesondere kann die Kalibrier-Einrichtung der Schaltkreis-Anordnung derart eingerichtet sein, dass mit ihr das an dem ersten Source-/Drain-Anschluss des Mess-Transistors anliegende elektrische Potential steuerbar ist.

Dies ist insbesondere bei einer Schaltkreis-Anordnung realisiert, bei der an den zweiten Source-/Drain-Anschluss des Mess-Transistors ein erstes elektrisches Referenz-Potential, beispielsweise das Massepotential, anlegbar ist, und bei der die Kalibrier-Einrichtung einen Kalibrier-Transistor und einen ersten und einen zweiten Source-/Drain-Anschluss aufweist, welche Source-/Drain-Anschlüsse zwischen den ersten Source-/Drain-Anschluss des Mess-Transistors und die Einrichtung zum Erfassen eines elektrischen Parameters geschaltet sind, und an dessen Gate-Anschluss ein derartiges elektrisches Signal anlegbar ist, dass das an den ersten Source-/Drain-Anschluss des Mess-Transistors anlegbare elektrische Potential derart einstellbar ist, dass die Veränderung des Wertes des physikalischen Parameters des Sensor-Elements zumindest teilweise kompensierbar ist.

Mit anderen Worten wird gemäß dieser Ausgestaltung der Kalibrier-Transistor als Gate-gesteuerter Source-Folger betrieben, wobei der zweite Source-/Drain-Anschluss des Kalibrier-Transistors mit dem ersten Source-/Drain-Anschluss des Mess-Transistors gekoppelt ist, und wobei über den Gate-Anschluss des Kalibrier-Transistors ein Knoten zwischen dem zweiten Source-/Drain-Anschluss des Kalibrier-Transistors und den ersten Source-/Drain-Anschluss des Mess-Transistors auf ein solches elektrisches Potential gebracht ist, dass dadurch die Veränderung des Wertes des physikalischen Parameters des Sensor-Elements zumindest teilweise kompensiert ist.

Alternativ kann die Kalibrier-Einrichtung der Schaltkreis-Anordnung derart eingerichtet sein, dass mit ihr das an dem zweiten Source-/Drain-Anschluss des Mess-Transistors anliegende elektrische Potential steuerbar ist.

Dies kann realisiert sein, indem der erste Source-/Drain-Anschluss des Mess-Transistors mit der Einrichtung zum Erfassen eines elektrischen Parameters gekoppelt ist, und indem die Kalibrier-Einrichtung einen Kalibrier-Transistor aufweist, der einen mit dem zweiten Source-/Drain-Anschluss des Mess-Transistors gekoppelten ersten Source-/Drain-Anschluss und einen zweiten Source-/Drain-Anschluss aufweist, an den ein zweites elektrisches Referenz-Potential anlegbar ist, und an dessen Gate-Anschluss ein derartiges elektrisches Signal anlegbar ist, dass das an den zweiten Source-/Drain-Anschluss des Mess-Transistors anlegbare elektrische Potential derart einstellbar ist, dass die Veränderung des Werts des physikalischen Parameters des Sensor-Elements zumindest teilweise kompensierbar ist.

In diesem Fall wird die Kalibrierung mittels einer Source-Gegenkopplung des Kalibrier-Transistors gegen den Mess-Transistor realisiert, wobei auf dem Gate-Anschluss des Kalibrier-Transistors eine Ladung verbleibt. Anschaulich wird in diesem Fall der Kalibrier-Transistor als steuerbarer Widerstand betrieben, wobei der Widerstand auf einen Wert eingestellt wird, durch den bewirkt ist, dass eine Veränderung des Wertes des physikalischen Parameters des Sensor-Elements zumindest teilweise kompensiert ist.

Die Kalibrier-Einrichtung der Schaltkreis-Anordnung kann alternativ einen Kalibrier-Transistor, eine erste Konstantstromquelle, die mit jeweiligen zweiten Source-/Drain-Anschlüssen der zueinander parallel geschalteten Mess- und Kalibrier-Transistoren gekoppelt ist, zum Bereitstellen einer vorgegebenen elektrischen Stromstärke, und eine Stromspiegel-Schaltung aufweisen, die mit jeweiligen ersten Source-/Drain-Anschlüssen der zueinander parallel geschalteten Mess- und Kalibrier-Transistoren gekoppelt ist, die derart verschaltet ist, dass mit ihr zum zumindest teilweisen Kompensieren der Veränderung des Wertes des physikalischen Parameters des Sensor-Elements das elektrische Potential am Gate-Anschluss des Kalibrier-Transistors derart einstellbar ist, dass in Abwesenheit eines Sensor-Ereignisses der Stromfluss zwischen den beiden Source-/Drain-Anschlüssen des Mess-Transistors und der Stromfluss zwischen den beiden Source-/Drain-Anschlüssen des Kalibrier-Transistors im Wesentlichen gleich ist.

Gemäß dieser Ausgestaltung der erfindungsgemäßen Schaltkreis-Einrichtung werden ein Mess-Transistor und ein Kalibrier-Transistor in zwei zueinander parallel geschalteten Stromzweigen betrieben, und es wird unter Verwendung der Funktionalität und einer geeigneten Verschaltung der Stromspiegel-Schaltung sichergestellt, dass in den beiden Stromzweigen in Abwesenheit eines Sensor-Ereignisses ein im Wesentlichen gleicher Strom fließt. Ein gleicher Strom in den beiden parallel geschalteten Zweigen mit Mess- bzw. Kalibrier-Transistor bewirkt eine derartige Einstellung der Potentiale an den Knoten des Mess- bzw. Kalibrier-Transistors, dass auch bei einer Abweichung des Werts des physikalischen Parameters des Mess- bzw. Kalibrier-Transistors bei einem Sensor-Ereignis ein von dem Wert des physikalischen Parameters unabhängiger Sensor-Strom fließt.

Alternativ kann bei der erfindungsgemäßen Schaltkreis-Anordnung an den ersten Source-/Drain-Anschluss des Mess-Transistors ein drittes elektrisches Potential angelegt sein, und die Kalibrier-Einrichtung kann einen Kalibrier-Transistor mit einem ersten und einem zweiten Source-/Drain-Anschluss, eine zweite Konstantstromquelle, die mit den jeweiligen zweiten Source-/Drain-Anschlüssen der zueinander parallel geschalteten Mess- und Kalibrier-Transistoren gekoppelt ist, zum Bereitstellen einer vorgebbaren elektrischen Stromstärke, und eine dritte Konstantstromquelle aufweisen, die mit dem ersten Source-/Drain-Anschluss des Kalibrier-Transistors koppelbar ist, zum Bereitstellen einer weiteren vorgebbaren elektrischen Stromstärke, welche dritte Konstantstromquelle derart verschaltet ist, dass mit ihr zum zumindest teilweisen Kompensieren der Veränderung des Werts des physikalischen Parameters die an den Anschlüssen der Transistoren anlegbaren Potentiale derart einstellbar sind, dass in Abwesenheit eines Sensor-Ereignisses die Stromflüsse zwischen den beiden Source-/Drain-Anschlüssen des Mess-Transistors einerseits und zwischen den beiden Source-/Drain-Anschlüssen des Kalibrier-Transistors andererseits gleich groß sind.

Gemäß einer alternativen Realisierung ist die Kalibrier-Einrichtung in der Schaltkreis-Anordnung derart eingerichtet, dass mit ihr ein von einem Sensor-Ereignis bewirktes Sensor-Signal des Sensor-Elements unter Verwendung des Prinzips der korrelierten Doppelabtastung (Correlated Double Sampling, CDS) in einen Wert umwandelbar ist, der von dem Wert des physikalischen Parameters des Sensor-Elements unabhängig ist.

Insbesondere kann gemäß dem CDS-Prinzip bei der Schaltkreis-Anordnung an einen zweiten Source-/Drain-Anschluss des Mess-Transistors ein viertes elektrisches Referenz-Potential angelegt sein, und die Kalibrier-Einrichtung kann eine elektrische Subtrahier-Einrichtung mit zwei Eingängen und einem Ausgang aufweisen, welcher Ausgang mit der Einrichtung zum Erfassen eines elektrischen Parameters koppelbar ist, welcher erste Eingang mit dem ersten Source-/Drain-Anschluss des Mess-Transistors gekoppelt ist, und welche elektrische Subtrahier-Einrichtung derart eingerichtet ist, dass an ihrem Ausgang die Differenz zwischen zwei an den beiden Eingängen angelegten elektrischen Signalen bereitstellbar ist. Ferner kann die Kalibrier-Einrichtung ein zwischen den ersten Source-/Drain-Anschluss des Mess-Transistors und den zweiten Eingang der elektrischen Subtrahier-Einrichtung geschaltetes Abtast-Halte-Glied (Sample and Hold) aufweisen. Die Kalibrier-Einrichtung ist derart eingerichtet, dass in einem ersten Betriebszustand in das Abtast-Halte-Glied ein von dem physikalischen Parameter des Sensor-Elements abhängiges Sensor-Signal einprägbar ist und dem zweiten Eingang der elektrischen Subtrahier-Einrichtung bereitstellbar ist. In einem zweiten Betriebszustand ist dem ersten Eingang der elektrischen Subtrahier-Einrichtung ein für den physikalischen Parameter des Sensor-Elements charakteristisches Signal bereitstellbar. Nach dem zweiten Betriebszustand ist an dem Ausgang der elektrischen Subtrahier-Einrichtung ein von dem Wert des physikalischen Parameters des Sensor-Elements unabhängiges Sensor-Signal bereitgestellt, wodurch die Veränderung des Werts des physikalischen Parameters zumindest teilweise kompensiert ist.

Mit anderen Worten wird zunächst in einem ersten Schritt ein Sensor-Ereignis in dem Sensor-Element detektiert und dem Abtast-Halte-Glied ein Sensor-Signal bereitgestellt und darin abgespeichert. Dieses Sensor-Signal ist von der Veränderung des Werts des physikalischen Parameters des ersten Sensor-Elements abhängig, und kann ferner von physikalischen Parametern weiterer Komponenten, beispielsweise eines Verstärkers zum Verstärken des Sensor-Signals abhängig sein. Das in dem Abtast-Halte-Glied gespeicherte Signal ist daher für unterschiedliche Sensor-Elemente mit unterschiedlichen Werten des physikalischen Parameters unterschiedlich. In dem zweiten Betriebszustand wird ein Sensor-Signal nicht erfasst, so dass ein Hilfs-Signal, das dem ersten Eingang der elektrischen Subtrahier-Einrichtung bereitgestellt ist, von dem Sensor-Ereignis unabhängig ist, und das von dem Wert des physikalischen Parameters bzw. von der Veränderung des Wertes des physikalischen Parameters abhängt. Die elektronische Subtrahier-Einrichtung ist derart eingerichtet, dass sie die Differenz zwischen dem von dem Wert des physikalischen Parameters abhängigen Signal an dem ersten Eingang und dem an dem zweiten Eingang bereitgestellten Sensor-Signal inklusive einem von dem physikalischen Parameter abhängigen Teilsignal bilden kann, so dass an dem Ausgang der elektronischen Subtrahier-Einrichtung ein von dem Wert des physikalischen Parameters im Wesentlichen unabhängiges Sensor-Signal bereitgestellt werden kann.

Anschaulich weist die Kalibrier-Einrichtung gemäß den beschriebenen Ausgestaltungen Bauelemente (insbesondere Transistoren und Kapazitäten, zum Beispiel ausgebildet durch die Gate-Kapazität eines Transistors) auf, mit deren Hilfe während einer Kalibrierphase für ein jeweiliges Sensor-Element spezifische Werte des physikalischen Parameters abgespeichert werden können, was im Kombination mit einer geeigneten Verschaltung mit den übrigen Komponenten der Schaltkreis-Anordnung bzw. des Sensor-Arrays dazu führt, dass alle Sensor-Elemente unabhängig von der jeweiligen Veränderung des Wertes des physikalischen Parameters eine identische Übertragungscharakteristik aufweisen, bzw. dass deren Offset-Größen zumindest teilweise abgeglichen sind.

Insbesondere kann der elektronische Parameter der Schaltkreis-Anordnung eine elektrische Spannung oder ein elektrischer Strom sein.

In der Regel wird ein ohmsch gekoppeltes Sensor-Element als Sensor-Signal einen elektrischen Strom zwischen den beiden Source-/Drain-Anschlüssen des darin vorzugsweise enthaltenen Mess-Transistors aufweisen. Dieser elektrische Strom kann beispielsweise direkt als Sensor-Ereignis erfasst werden. Alternativ kann, was in bestimmten Anwendungsfällen weniger aufwendig sein kann, eine von dem beschriebenen elektrischen Strom abhängige elektrische Spannung als zu erfassender elektrischer Parameter verwendet werden. Um einen elektrischen Sensor-Strom in eine elektrische Sensor-Spannung umzuwandeln, kann beispielsweise ein Strom-Spannungs-Wandler verwendet werden, beispielsweise ein Widerstand, an dem infolge des Sensor-Stroms eine Sensor-Spannung abfällt.

Vorzugsweise weist die Sensor-Elektrode der erfindungsgemäßen Schaltkreis-Anordnung eines oder eine Kombination der Materialien Titan, Titannitrid, Gold und Platin auf. Diese Materialien haben alle die Eigenschaft, elektrisch gut leitfähig zu sein und chemisch inert zu sein. Die geringe Empfindlichkeit gegenüber möglicherweise chemisch aggressiven Elektrolyten in Wirkkontakt mit der Sensor-Elektrode und die Kompatibilität der beschriebenen Materialien mit häufig empfindlichen biologischen Substanzen führt dazu, dass diese Materialien gut geeignet sind als Material für die Sensor-Elektrode. Insbesondere ist eine mit einer dünnen Titannitrid-Schicht bedeckte Titan-Sensor-Elektrode besonders vorteilhaft, da Titannitrid in dünnen Schichten ein guter elektrischer Leiter ist, chemisch inert ist, biokompatibel ist und eine hohe aktive Oberfläche aufweist. Diese Sensor-Elektrode steht in Kontakt mit dem Elektrolyten und dient der Ableitung der Signale, beispielsweise der zellulären Signale von Nervenzellen.

Ferner kann die erfindungsgemäße Schaltkreis-Anordnung mindestens ein integriertes Verstärker-Element zum Verstärken eines Sensor-Signals aufweisen.

Mittels Verstärkens der oft kleinen elektrischen Sensor-Signale wird die Sensitivität erhöht. Indem die Verstärkung in der Nähe des Sensor-Signals, das heißt nach einem kurzen Übertragungsweg des elektrischen Signals, erfolgt, ist das Signal-Rausch-Verhältnis erhöht. Als Verstärker sind beispielsweise Spannungsverstärker oder Transkonduktanzverstärker geeignet.

Gemäß einer bevorzugten Weiterbildung weist die Schaltkreis-Anordnung eine Schalt-Einrichtung auf, die derart eingerichtet ist, dass mit dieser das Sensor-Element wahlweise mit einem fünften elektrischen Referenz-Potential koppelbar oder von diesem entkoppelbar ist, um das Sensor-Element vor einer Schädigung zu schützen und/oder um an das Sensor-Element ein definiertes elektrisches Potential anzulegen.

Eine derartige Schalt-Einrichtung kann eine zu- oder abschaltbare elektrische Kopplung des Eingangs des Mess-Transistors mit einer schaltungsintern vorgegebenen elektrischen Spannung bewirken. Dies ist insbesondere in einem Betriebszustand vorteilhaft, in dem ein auf der erfindungsgemäßen Schaltkreis-Anordnung basierendes Sensor-Array mit einem Elektrolyten aufgefüllt wird, da an dem Eingang des Mess-Transistors während dieses Vorgangs elektrische Überschläge auftreten könnten, wenn der Eingang sehr hochohmig ist, zum Beispiel, wenn der Eingang mit dem Gate-Anschluss eines MOS-Transistors gekoppelt ist. Indem an den Eingang des Mess-Transistors mittels der Schalt-Einrichtung ein vorgebbares elektrisches Referenz-Potential angelegt wird, ist der empfindliche, als integriertes Bauelement ausgebildete, Mess-Transistor vor elektrischen Überschlägen geschützt. Ferner kann bei der Herstellung eines Multi-Elektroden-Arrays an dem Eingang eines Mess-Transistors eine prozessbedingte elektrische Aufladung (sogenannte Antenneneffekte) auftreten, wenn der Eingang als Gate-Anschluss eines MOS-Transistors ausgeführt ist und dieser Anschluss keine weitere Verbindung zu einem anderen Schaltungsknoten aufweist. Der Mess-Transistor ist vor solchen nachteiligen Effekten geschützt, wenn der Gate-Anschluss des Mess-Transistors mit dem elektrischen Referenz-Potential gekoppelt wird. Ferner kann es für bestimmte Anwendungen erforderlich sein, zumindest einen Teil der Gate-Anschlüsse der Mess-Transistoren der Schaltkreis-Anordnungen eines Sensor-Arrays an ein Referenz-Potential anzulegen, beispielsweise während einer Kalibrierphase.

Vorzugsweise ist das Substrat der Sensor-Anordnung ein Silizium-Substrat, insbesondere ein Silizium-Wafer oder ein Silizium-Chip. In diesem Falle können die Vorteile und ausgereiften üblichen Prozesse der Silizium-Mikroelektronik verwendet werden, um die erfindungsgemäße Sensor-Anordnung herzustellen.

Die Art der Kopplung zwischen dem Sensor-Element und der zu untersuchenden Flüssigkeit kann zusätzlich einen kapazitiven Anteil aufweisen.

Im Weiteren werden Ausgestaltungen des erfindungsgemäßen Sensor-Arrays, das erfindungsgemäße Schaltkreis-Anordnungen aufweist, beschrieben. Ausgestaltungen der Schaltkreis-Anordnung gelten auch für das Schaltkreis-Anordnungen aufweisende Sensor-Array.

Indem ein Sensor-Array mit einer Mehrzahl von in Kreuzungsbereichen von Zeilen- und Spalten-Leitungen im Wesentlichen matrixförmig angeordneten und mit den Zeilen- und Spalten-Leitungen verschalteten Schaltkreis-Anordnungen erfindungsgemäß bereitgestellt ist, ist eine hohe Integrationsdichte von Sensor-Elementen und ein hoher Grad an Miniaturisierung erreichbar, wodurch die räumliche Auflösung des Sensor-Arrays hoch ist.

Vorzugsweise weist bei dem Sensor-Array zumindest ein Teil der Schaltkreis-Anordnungen ein mit der jeweils zugehörigen Zeilen-Leitung und/oder Spalten-Leitung gekoppeltes Auswahl-Element zum Auswählen der jeweiligen Sensor-Anordnung auf, um ein Sensor-Signal des Sensor-Elements der ausgewählten Schaltkreis-Anordnung zu erfassen und/oder bei der ausgewählten Schaltkreis-Anordnung die Veränderung des Wertes des physikalischen Parameters zumindest teilweise zu kompensieren und/oder um an das Sensor-Element der ausgewählten Schaltkreis-Anordnung das fünfte elektrische Potential anzulegen.

Eine derartige Schaltung ermöglicht die Auswahl eines Sensor-Elements bzw. einer Schaltkreis-Anordnung des Sensor-Arrays und ist derart eingerichtet, Signale zum Ansteuern von Auswahl-Elementen beispielsweise an einem Endabschnitt von Zeilen- und Spalten-Leitungen bereitzustellen.

Auch kann eine Schaltung mit einem Multiplexer zum sukzessiven Auswählen von Sensor-Elementen, welche Schaltung beispielsweise mittels eines Steuer-Signals steuerbar ist, bereitgestellt sein.

Gemäß einer bevorzugten Weiterbildung des erfindungsgemäßen Sensor-Arrays kann zumindest ein Teil der einer jeweiligen Zeilen- und/oder Spalten-Leitung zugeordneten Schaltkreis-Anordnungen eine gemeinsame Einrichtung zum Erfassen eines ein erfolgtes Sensor-Ereignis charakterisierenden elektrischen Parameters, eine gemeinsame Konstantstromquelle, eine gemeinsame Schalt-Einrichtung, ein gemeinsames Referenz-Potential, einen gemeinsamen Strom-Spannungs-Wandler, einen gemeinsamen Analog-Digital-Wandler, einen gemeinsamen Stromspiegel, eine gemeinsame Differenzstufe, eine gemeinsame Subtrahier-Einrichtung, ein gemeinsames Abtast-Halte-Glied und/oder einen gemeinsamen Verstärker aufweisen.

Indem eine gemäß obiger Beschreibung einer jeweiligen Schaltkreis-Anordnung zugeordnete Komponente nicht jeder einzelnen Schaltkreis-Anordnung, sondern einem Teil oder aller Schaltkreis-Anordnungen einer Zeile bzw. einer Spalte zugeordnet werden, sind Komponenten eingespart, wodurch der Platzbedarf und der Herstellungsaufwand reduziert sind. Mit anderen Worten kann beispielsweise ein gemeinsamer Verstärker am Endabschnitt einer Spalten-Leitung bereitgestellt sein, so dass nicht jede einzelne Schaltkreis-Anordnung der Spalten-Leitung einen separaten Verstärker aufweisen muss. Insbesondere ist zu betonen, dass ein von einem Sensor-Element abgeleitetes verstärktes analoges Signal bereits On-Chip in eine digitale Größe umgewandelt werden kann (mittels eines Analog-Digital-Wandlers), wodurch die Fehlerrobustheit erhöht und die Nachweisempfindlichkeit verbessert ist. Auch kann das Sensor-Array Schaltungen bzw. Schaltungskomponenten aufweisen, die mit den Komponenten der einzelnen Schaltkreis-Anordnungen Regelkreise bilden, die während der Kalibrierphase aktiviert werden und der Bereitstellung von im Inneren der Schaltkreis-Anordnungen (beispielsweise an geeigneten Knoten oder Anschlüssen von Transistoren) abzuspeichernden Kalibriergrößen dienen.

Ferner kann bei dem Sensor-Array zumindest ein Teil der Zeilen- und oder Spalten-Leitungen jeweils eine Einrichtung zum Erfassen eines ein erfolgtes Sensor-Ereignis charakterisierenden elektrischen Parameters aufweisen, wobei das Sensor-Array derart eingerichtet ist, dass mittels der einer jeweiligen Zeilen- oder Spalten-Leitung zugeordneten Einrichtung zum Erfassen eines elektrischen Parameters entweder ein Sensor-Signal genau einer Sensor-Anordnung der jeweiligen Zeilen- oder Spalten-Leitung oder eine Summe von Sensor-Signalen von zumindest einem Teil der Sensor-Anordnung in der jeweiligen Zeilen- oder Spalten-Leitungen erfassbar ist. Mit anderen Worten kann wahlweise beispielsweise entlang einer Spalten-Leitung entweder das Sensor-Signal von genau einem Sensor-Element der Spalten-Leitung oder von einem Teil der Sensor-Elemente der Spalten-Leitung oder von allen Sensor-Elementen der Spalten-Leitung erfasst werden. Im letzteren Falle wird ein Summenstrom-Signal der Sensor-Elemente einer Spalten-Leitung erfasst und ausgewertet. Selbstverständlich ist ein derartiges Erfassen von Summenstrom-Signalen auch entlang einer Zeilen-Leitung möglich.

Zumindest ein Teil der Spalten-Leitungen des erfindungsgemäßen Sensor-Arrays kann mit einer Potentialsteuer-Einrichtung gekoppelt sein, welche derart eingerichtet ist, dass sie das elektrische Potential der zugehörigen Spalten-Leitung auf einem im Wesentlichen konstanten Wert hält. Indem mittels einer Potentialsteuer-Einrichtung das elektrische Potential von einer Spalten-Leitung auf einem im Wesentlichen konstanten Wert gehalten werden kann, ist eine verbesserte Reproduzierbarkeit und eine Erhöhung der Messgenauigkeit erreicht.

Wie oben angesprochen ist erfindungsgemäß auch ein Biosensor-Array mit einem Sensor-Array mit den oben genannten Merkmalen bereitgestellt. Mit anderen Worten kann das erfindungsgemäße Sensor-Array ohne weiteres als Sensor zum Erfassen von solchen Signalen verwendet werden, die von biologischen Systemen stammen, wie beispielsweise Nervenzellen, die auf das Biosensor-Array aufgebracht sind oder darauf aufgewachsen sind. Aufgrund der hohen räumlichen Auflösung des Biosensor-Arrays, der Bio-Kompatibilität der verwendeten Materialien und der Nachweisempfindlichkeit von Sensor-Elementen des Biosensor-Arrays ist dieses für biologische Anwendungen ideal.

Ausführungsbeispiele der Erfindung sind in den Figuren dargestellt und werden im Weiteren näher erläutert.

Es zeigen:

Figur 1 ein elektrisch adressierbares Multi-Elektroden-Array gemäß dem Stand der Technik,

Figuren 2 bis 19 Sensor-Arrays gemäß bevorzugten Ausführungsbeispielen der Erfindung.

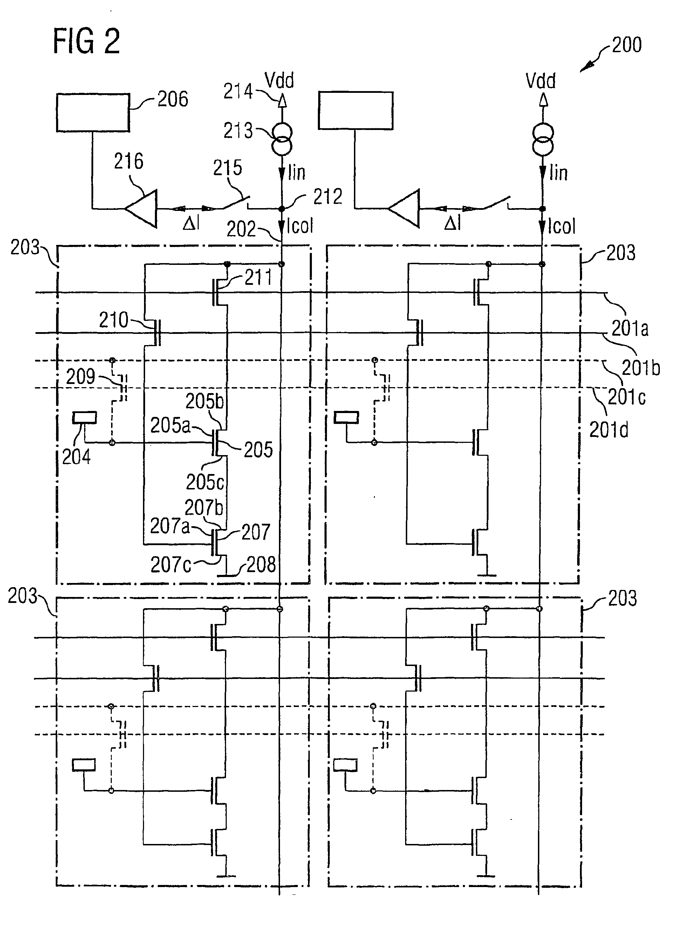

Es sei angemerkt, dass bei den im Weiteren bezugnehmend auf Fig.2 bis Fig.19 beschriebenen Ausführungsbeispielen des erfindungsgemäßen Sensor-Arrays viele der beschriebenen Komponenten in unterschiedlichen Ausführungsbeispielen auftreten. Solche Komponenten sind in unterschiedlichen Ausführungsbeispielen jeweils mit den gleichen Bezugsziffern versehen. Ferner ist auch die Funktionalität von Teil-Schaltkreisen der unterschiedlichen Ausführungsbeispiele des erfindungsgemäßen Sensor-Arrays teilweise gleich, so dass diese Funktionalität nicht für jedes Ausführungsbeispiel im Detail beschrieben wird und daher auf andere Ausführungsbeispiele zurückverwiesen wird.

In **Fig.2** ist ein Sensor-Array 200 gemäß einem ersten bevorzugten Ausführungsbeispiel der Erfindung gezeigt.

Das Sensor-Array 200 weist eine Mehrzahl von in Kreuzungsbereichen von Zeilen-Leitungen 201a, 201b, 201c, 201d und Spalten-Leitungen 202 matrixförmig angeordneten und mit den Zeilen- und Spalten-Leitungen 201a, 201b, 201c, 201d, 202 verschalteten Biosensor-Schaltkreis-Anordnungen 203 auf. In Fig.2 sind insgesamt vier Biosensor-Schaltkreis-Anordnungen 203 gezeigt, die jedoch nur einen Ausschnitt der matrixförmigen Anordnung von Biosensor-Schaltkreis-Anordnungen 203 zeigen. In gemäß Fig.2 horizontaler Richtung sind Biosensor-Schaltkreis-Anordnungen 203 einer Zeile angeordnet, in gemäß Fig.2 vertikaler Richtung sind die Biosensor-Schaltkreis-Anordnungen 203 einer Spalte angeordnet. Gemäß dem in Fig.2 gezeigten Ausführungsbeispiel weist jeder der Biosensor-Schaltkreis-Anordnungen 203 dieselbe interne Verschaltung auf, so dass im Weiteren nur der Aufbau und die Verschaltung einer Biosensor-Schaltkreis-Anordnung 203 näher beschrieben wird.

Jede Biosensor-Schaltkreis-Anordnung 203 weist eine mit einer zu untersuchenden Substanz, beispielsweise einer Nervenzelle (nicht gezeigt in den Figuren), gekoppelte elektrisch leitfähige Sensor-Elektrode 204 aus Gold auf. Ferner weist das in der Biosensor-Schaltkreis-Anordnung 203 enthaltenen Sensor-Element einen Mess-Transistor 205 auf, dessen Gate-Anschluss 205a mit der elektrisch leitfähigen Sensor-Elektrode 204 gekoppelt ist. Das Sensor-Element der Biosensor-Schaltkreis-Anordnung 203 ist von der Sensor-Elektrode 204 und dem Mess-Transistor 205 gebildet. Die Kopplung des Sensor-Elements mit der in Fig.2 nicht gezeigten zu untersuchenden Substanz weist einen ohmschen Anteil auf, d.h., es besteht ein direkter elektrischer Kontakt zwischen elektrisch geladenen Partikeln in der zu untersuchenden Flüssigkeit und dem mit der Sensor-Elektrode 204 gekoppelten Gate-Anschluss 205a des Mess-Transistors 205. Ferner ist in Fig.2 ein Amperemeter 206 zum Erfassen eines ein erfolgtes Sensor-Ereignis charakterisierenden elektrischen Stroms gezeigt, welches Amperemeter 206 über mehrere andere Bauelemente mit dem ersten Source-/Drain-Anschluss 205b des Mess-Transistors 205 koppelbar ist. Die Biosensor-Schaltkreis-Anordnungen 203 sind in ein Silizium-Substrat (nicht gezeigt) integriert. Insbesondere weist jede der Biosensor-Schaltkreis-Anordnungen 203 eine in oder auf dem Substrat ausgebildete Kalibrier-Einrichtung auf, die derart eingerichtet ist, dass mit ihr eine Veränderung des Werts eines physikalischen Parameters des Sensor-Elements zumindest teilweise kompensierbar ist. Mit anderen Worten weist jedes der Sensor-Elemente 204 der Biosensor-Schaltkreis-Anordnung 203 einen Wert des physikalischen Parameters, nämlich der Schwellenspannung des Mess-Transistors 205, auf, der bei den unterschiedlichen Sensor-Elementen um einen Referenzwert streut. Die Kalibrier-Einrichtung ist, wie im Weiteren beschrieben wird, dazu in der Lage, eine elektrische Ladung als Korrekturgröße an einem Knoten des zugehörigen Sensor-Elements zu speichern, so dass anschaulich alle Mess-Transistoren 205 bei demselben Arbeitspunkt betreibbar sind, unabhängig von dem Wert des tatsächlichen physikalischen Parameters des jeweiligen Transistors. Bei der Biosensor-Schaltkreis-Anordnung 203 ist die Kalibrier-Einrichtung derart eingerichtet, dass mit ihr das an einem zweiten Source-/Drain-Anschluss 205c des Mess-Transistors 205 anliegende elektrische Potential steuerbar ist. Der erste Source-/Drain-Anschluss 205b des Mess-Transistors 205 ist, wie in Fig.2 gezeigt, mit dem Amperemeter 206 gekoppelt, und die Kalibrier-Einrichtung weist einen Kalibrier-Transistor 207 auf, der einen mit dem zweiten Source-/Drain-Anschluss 205c des Mess-Transistors 205 gekoppelten ersten Source-/Drain-Anschluss 207b aufweist, und der einen zweiten Source-/Drain-Anschluss 207c aufweist, an den ein elektrisches Massepotential 208 angelegt ist, und an dessen Gate-Anschluss 207a ein derartiges elektrisches Signal anlegbar ist, dass das an den zweiten Source-/Drain-Anschluss 205c des Mess-Transistors 205 anlegbare elektrische Potential derart einstellbar ist, dass die Veränderung des Werts der Schwellenspannung (physikalischer Parameter) des jeweiligen Sensor-Elements zumindest teilweise kompensierbar ist. Ferner weist jede Biosensor-Schaltkreis-Anordnung 203 einen ersten Schalt-Transistor 209 auf, dessen Source-/Drain-Anschlüsse zwischen den Gate-Anschluss 205a des Mess-Transistors 205 und die zugehörige dritte Zeilen-Leitung 201c geschaltet ist. Ferner ist der Gate-Anschluss des ersten Schalt-Transistors 209 mit der zugehörigen vierten Zeilen-Leitung 201d gekoppelt. Der erste Schalt-Transistor 209 sowie die dritte und vierte Zeilen-Leitung 201c, 201d bilden eine Schalt-Einrichtung, die derart eingerichtet ist, dass mit dieser das zugeordnete Sensor-Element wahlweise mit einem an die dritte Zeilen-Leitung 201c angelegten elektrischen Referenz-Potential koppelbar oder von diesem entkoppelbar ist, um das zugehörige Sensor-Element vor einer Schädigung zu schützen und/oder um an das zugehörige Sensor-Element ein definiertes elektrisches Potential, nämlich das an der dritten Zeilen-Leitung 201c angelegte Potential, bereitzustellen. An den Gate-Anschluss 205a des Mess-Transistors 205 ist das an der dritten Zeilen-Leitung 201c anliegende elektrische Potential dann angelegt, wenn an die vierte Zeilen-Leitung 201c ein entsprechendes Schalt-Signal angelegt wird, mittels welchem der erste Schalt-Transistor 209 leitend wird. Der Gate-Anschluss 207a des Kalibrier-Transistor 207 ist mit einem Source-/Drain-Anschluss eines zweiten Schalt-Transistor 210 gekoppelt, dessen Gate-Anschluss mit der zugehörigen zweiten Zeilen-Leitung 201b gekoppelt ist. Der erste Source-/Drain-Anschluss 205b des Mess-Transistors 205 ist mit einem Source-/Drain-Anschluss eines dritten Schalt-Transistors 211 gekoppelt, dessen Gate-Anschluss mit der zugehörigen ersten Zeilen-Leitung 201a gekoppelt ist. Die jeweils anderen Source-/Drain-Anschlüsse des zweiten und des dritten Schalt-Transistors 210, 211 sind mit einer zugehörigen Spalten-Leitung 202 gekoppelt. Am Endabschnitt jeder Spalten-Leitung 202 ist ein elektrischer Knoten 212 angeordnet. Der erste elektrische Knoten 212 ist mit einer Konstantstromquelle 213 gekoppelt, an die eine Versorgungsspannung 214 angelegt ist. Ferner ist der erste elektrische Knoten 212 über einen ersten Schalter 215 und einen ersten Verstärker 216 und mit dem Amperemeter 206 gekoppelt. Der erste Verstärker 216 dient zum Verstärken eines Sensor-Signals.

Im Weiteren wird die Funktionalität des Sensor-Arrays 200 beschrieben. Bei dem Sensor-Array 200 aus Fig. 2 wird mittels Anlegens eines elektrischen Signals an die erste Zeilen-Leitung 201a genau eine Sensor-Anordnung 203 in jeder Spalten-Leitung 202 aktiviert. Das an einem Endabschnitt der Spalten-Leitung 202 bereitgestellte Sensor-Signal eines Sensor-Elements einer Biosensor-Schaltkreis-Anordnung 203 enthält einen Sensorstrom •I, welcher einem Bias-Strom $I_{in}$ überlagert ist.

Mittels der Konstantstromquelle 213 wird in jede Spalten-Leitung 202 der Strom $I_{in}$ eingespeist, und zwar sowohl während einer Kalibrierphase als auch während einer Messphase (siehe Beschreibung unten). Die Auswahl einer bestimmten Zeile von Biosensor-Schaltkreis-Anordnungen 203 erfolgt dadurch, dass an eine zugehörige erste Zeilen-Leitung 201a ein elektrisches Signal angelegt wird, wodurch der dritte Schalt-Transistor 211 leitend wird.

In einer Kalibrierphase sind die ersten Schalter 215 geöffnet, so dass der Strom $I_{in}$ identisch ist mit dem in einer Spalten-Leitung 202 fließenden Spaltenstrom $I_{col}$. Ferner wird an die zweiten Spalten-Leitungen 201b ein elektrisches Signal angelegt, so dass der damit gekoppelte zweite Schalt-Transistor 210 leitend wird. Dann fließt auch zwischen den jeweiligen Source-/Drain-Anschlüssen des dritten Schalt-Transistors 211, des Mess-Transistors 205 und des Kalibrier-Transistors 207 die Stromstärke $I_{col}$.

Wird der Kalibrier-Transistor 207 in Sättigung betrieben, das heißt, ist die Differenz zwischen der Spannung zwischen dem Gate-Anschluss 205a und dem zweiten Source-/Drain-Anschluss 205c des Mess-Transistors 205 und der Schwellenspannung des Mess-Transistors 205 kleiner als die Spannung zwischen den beiden Source-/Drain-Anschlüssen 205b, 205c des Mess-Transistors 205, so gilt für den Stromfluss $I_{DS}$ durch den ersten Source-/Drain-Anschluss 205b des Mess-Transistors 205 näherungsweise:

$$I_{DS} = 1/2 \ W/L \ k \ [V(E) - (V_t - \bullet V_t) - V_{12})^2 \qquad (1)$$

Dabei ist k eine technologieabhängige Konstante, auch Transistorkonstante genannt, W/L ist das Verhältnis zwischen Weite W und Länge L des Transistors, V(E) ist die an der Sensor-Elektrode 204 anliegende (oder angelegte) elektrische Spannung, die identisch mit dem elektrischen Potential des Gate-Anschlusses 205a des Mess-Transistors 205 ist, $V_t$ ist ein Mittel- oder Referenzwert der Schwellenspannungen unterschiedlicher Mess-Transistoren 205 der Biosensor-Schaltkreis-Anordnungen 203 des Sensor-Arrays 200, $\bullet V_t$ ist die individuelle Abweichung bzw. Veränderung der Schwellenspannung eines bestimmten Mess-Transistors 205 des Sensor-Arrays 200 von dem Mittelwert $V_t$ (allgemein ausgedrückt die Veränderung des physikalischen Parameters), und $V_{12}$ ist die gemeinsame elektrische Spannung an dem zweiten Source-/Drain-Anschluss 205c des Mess-Transistors 205 und an dem ersten Source-/Drain-Anschluss 207b des Kalibrier-Transistors 207. Da die Abhängigkeit des Stroms durch den ersten Source-/Drain-Anschluss von der Spannung zwischen den beiden Source-/Drain-Anschlüssen bei einem Transistor mit einem Arbeitspunkt im Sättigungsbereich gering ist, ist dieser Effekt vernachlässigbar (vgl. allgemeine Transistorkennlinie).

Aufgrund der sich bei der Verschaltung gemäß Fig.2 in der Kalibrierphase (erster Schalter 215 offen) ergebenden schaltungstechnischen Zwangsbedingung

$$I_{in} = I_{col} = I_D \qquad (2)$$

stellt sich an dem Gate-Anschluss 207a des Kalibrier-Transistors 207 eine bestimmte elektrische Spannung ein. Mittels dieser ist der Leitfähigkeitszustand des Kanalbereichs des Kalibrier-Transistors 207 definiert, welcher Kalibrier-Transistors 207 daher anschaulich als steuerbarer Widerstand betrieben wird und somit eine Source-Gegenkopplung des Mess-Transistors 205 bewirkt. Da die schaltungstechnische Konfiguration von Fig.2 einen geschlossenen Regelkreis darstellt, ergibt sich genau der Spannungsabfall $V_{12}$ über dem Kalibrier-Transistor 207, bei dem die Gleichungen (1) und (2) gleichzeitig erfüllt sind. Insbesondere bedeutet dies, dass für jede Biosensor-Schaltkreis-Anordnung 203 ein individueller Wert $V_{12}$ erhalten wird, der von der Veränderung der Schwellenspannung des zugehörigen Mess-Transistors 205 abhängt:

$$V_{12}nm = \bullet V_t nm + const. \qquad (3)$$

Dabei bezeichnet n die Ordnungszahl der Zeile und m die Ordnungszahl der Spalte der jeweiligen Biosensor-Schaltkreis-Anordnung 203 in der matrixförmigen Anordnung von Biosensor-Schaltkreis-Anordnungen 203.

Wenn sich die beschriebenen elektrischen Ladungszustände an den Anschlüssen des Mess-Transistor 205 bzw. des Kalibrier-Transistor 207 eingestellt haben, wird das an der zugehörigen zweiten Zeilen-Leitung 201b zuvor angelegte elektrische Signal abgeschaltet, so dass der zweite Schalt-Transistor 210 nicht mehr leitet. Auf der Kapazität des Gate-Anschlusses 207a des Kalibrier-Transistors 207 verbleibt die Ladungsmenge, die während der Kalibrierphase aufgebracht wurde, das heißt die Gate-Spannung des Kalibrier-Transistors 207 verbleibt unverändert und das Pixel ist kalibriert.

Der beschriebene Regelungs- und Kalibriermechanismus funktioniert auch, wenn nicht nur ein einziger physikalischer Parameter, im beschriebenen Ausführungsbeispiel die Schwellenspannung des Mess-Transistors 205 Schwankungen unterworfen ist, sondern auch dann, wenn mehrere (beispielsweise zusätzlich der Faktor k in Gleichung (1) oder weitere Parameter in einer detaillierteren Beschreibung des Transistors) physikalische Parameter beispielsweise infolge von Prozessschwankungen einen Wert aufweisen, der von einem Mittelwert abweicht. Ferner ist anzumerken, dass der beschriebene Regelungs- und Kalibriermechanismus ebenso funktioniert, wenn, was aufgrund des Sättigungsbetriebs des Mess-Transistors 205 vernachlässigt wurde, die Abhängigkeit des Stromflusses zwischen den beiden Source-/Drain-Anschlüssen 205b, 205c des Mess-Transistors 205 von der Spannung zwischen den beiden Source-/Drain-Anschlüssen 205b, 206c des Mess-Transistors 205 berücksichtigt wird, etwa in einem Szenario, in dem diese Abhängigkeit stärker ist als beim Betrieb des Transistors in Sättigung.

In einer Messphase ist der erste Schalter 215 geschlossen. Änderungen des Potentials der zugehörigen Sensor-Elektrode 204 führen zu einer Änderung des Stroms durch den ersten Source-/Drain-Anschluss 205b des Mess-Transistors 205 und somit zu einer Änderung des Spaltenstromes $I_{col}$. Die Differenz aus $I_{in}$ und $I_{col}$ fließt über den geschlossenen ersten Schalter 215 in den Eingang des ersten Verstärkers 216. Der erste Verstärker 216 kann ein Stromverstärker sein oder er kann den Eingangsstrom in eine Ausgangsspannung umsetzen und daher als Strom-Spannungs-Wandler fungieren.

Es ist zu beachten, dass der von dem Amperemeter 206 nach erfolgter Kalibrierungs- und Messphase detektierte Sensor-Strom unabhängig von dem einer Variation der Schwellenspannung oder eines anderen physikalischen Parameters unterworfenen, aus Sensor-Elektrode 204 und Mess-Transistor 205 gebildeten Sensor-Element ist, so dass infolge der Kalibrierung sichergestellt ist, dass jede der in Fig.2 gezeigten Biosensor-Schaltkreis-Anordnungen 203 bei einem bestimmten Sensor-Signal einen bestimmten Sensor-Strom an dem Amperemeter 206 bewirken.

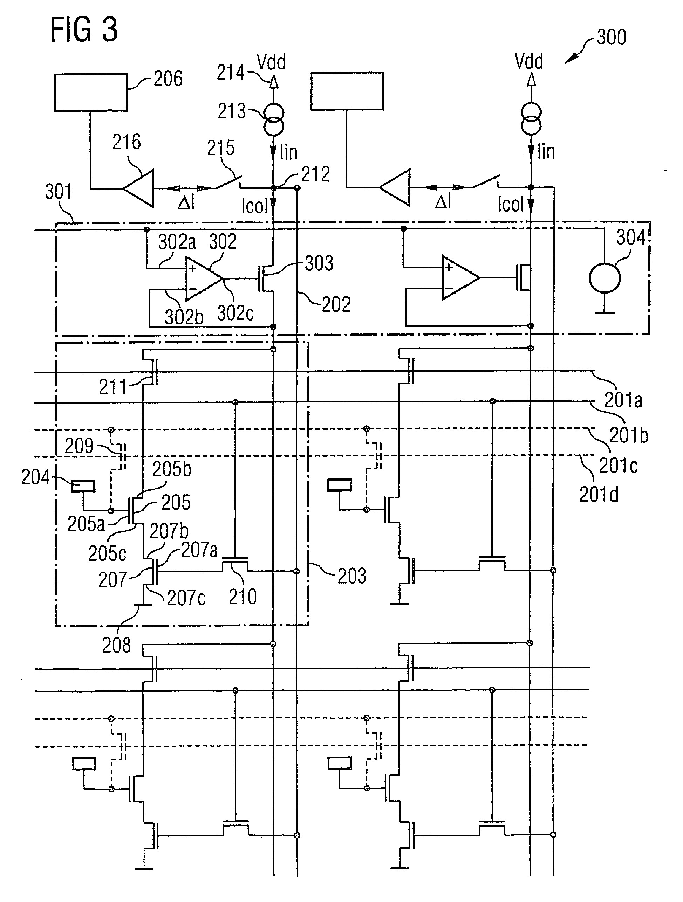

Im Weiteren wird bezugnehmend auf **Fig.3** ein zweites bevorzugtes Ausführungsbeispiel des erfindungsgemäßen Sensor-Arrays beschrieben.

Das in Fig.3 gezeigte Sensor-Array 300 unterscheidet sich von dem in Fig.2 gezeigten Sensor-Array 200 dadurch, dass die Spalten-Leitungen 202 mit einer Potentialsteuer-Einrichtung 301 gekoppelt sind, welche Potentialsteuer-Einrichtung 301 derart eingerichtet ist, dass sie das elektrische Potential der dazugehörigen Spalten-Leitung 202 auf einem im Wesentlichen konstanten Wert hält.

Die in Fig.3 gezeigte Potentialsteuer-Einrichtung 301 weist einen Operationsverstärker 302 mit einem nicht-invertierten Eingang 302a und einem invertierten Eingang 302b und einem Ausgang 302c auf und weist einen vierten Schalt-Transistor 303 auf, dessen Gate-Anschluss mit dem Ausgang 302c des Operationsverstärkers 302 gekoppelt ist. Der eine Source-/-Drain-Anschluss des vierten Schalt-Transistor 303 ist mit dem ersten elektrischen Knoten 212 gekoppelt, und der andere Source-/Drain-Anschluss des vierten Schalt-Transistors 303 ist sowohl mit dem invertierten Eingang 302b des Operationsverstärkers 302 als auch mit einem Source-/Drain-Anschluss des dritten Schalt-Transistors 211 gekoppelt. Ferner weist die Potentialsteuer-Einrichtung 301 eine erste Spannungsquelle 304 auf, mittels derer der nicht-invertierte Eingang 302a des Operationsverstärkers 302 auf ein vorgebbares elektrisches Potential gebracht werden kann.

Bei dem Sensor-Array 300 aus Fig.3 kann das elektrische Potential der Spalten-Leitungen 202 und daher das elektrische Potential an dem ersten Source-/Drain-Anschluss 205b des Mess-Transistors 205 über die mittels des Operationsverstärkers 302 und des vierten Schalt-Transistors 303 aufgebaute Steuer-Schaltung sowohl in der Kalibrierphase als auch in der Messphase auf einem konstanten Potential gehalten werden. Dadurch kann die Genauigkeit der Kalibrierung weiter erhöht werden. Unterschiedliche Potentiale des ersten Source-/Drain-Anschlusss 205b während der Kalibrierphase und während der Messphase des Mess-Transistors 205 können zu geringen unerwünschten Differenzströmen •I_führen, welche an dem ersten Verstärker 216 ein parasitäres Eingangssignal bewirken können. Bei dem in Fig.3 gezeigten Ausführungsbeispiel des Sensor-Arrays 300 ist dieses Problem vermieden.

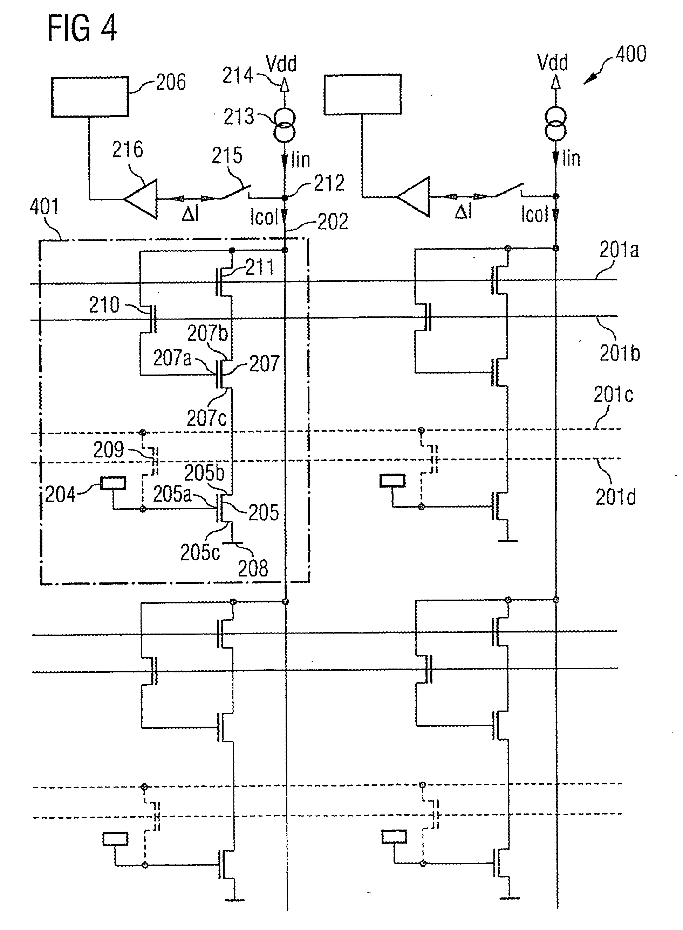

Im Weiteren wird Bezug nehmend auf **Fig.4** ein drittes bevorzugtes Ausführungsbeispiel des erfindungsgemäßen Sensor-Arrays beschrieben.

Das in Fig.4 gezeigte Sensor-Array 400 weist dieselben Komponenten auf wie das bezugnehmend auf Fig.2 beschriebene Sensor-Array 200. Allerdings ist die Verschaltung innerhalb der Biosensor-Schaltkreis-Anordnungen des Sensor-Arrays 400 anders als im Falle des Sensor-Arrays 200. Daher wird im Weiteren die Verschaltung und die Funktionalität der Biosensor-Schaltkreis-Anordnung 401 beschrieben.

Bei dem Sensor-Array 400 ist die Sensor-Elektrode 204 mit dem Gate-Anschluss 205a des Mess-Transistors 205 gekoppelt. Der zweite Source-/Drain-Anschluss 205c des Mess-Transistors 205 ist mit dem Massepotential 208 gekoppelt. Der erste Source-/-Drain-Anschluss 205b des Mess-Transistors 205 ist mit dem zweiten Source-/Drain-Anschluss 207c des Kalibrier-Transistors 207 gekoppelt. Der erste Source-/Drain-Anschluss 207b des Kalibrier-Transistors 207 ist mit einem Source-/-Drain-Anschluss des dritten Schalt-Transistors 211 gekoppelt, und der Gate-Anschluss 207a des Kalibrier-Transistors 207 ist mit einem Source-/Drain-Anschluss des zweiten Kalibrier-Transistors 210 gekoppelt. Der andere Source-/Drain-Anschluss des zweiten Schalt-Transistors 210 ist mit dem ersten elektrischen Knoten 212 gekoppelt, genauso wie der andere Source-/Drain-Anschluss des dritten Schalt-Transistors 211.

Bei der Biosensor-Schaltkreis-Anordnung 401 ist an den zweiten Source-/-Drain-Anschluss 205c des Mess-Transistors 205 ein Massepotential 208 angelegt, und die beiden Source-/Drain-Anschlüsse 207b, 207c des Kalibrier-Transistors 207 der Kalibrier-Einrichtung sind zwischen den ersten Source-/Drain-Anschluss 205b des Mess-Transistors 205 und (über mehrere andere Komponenten) das Amperemeter 206 geschaltet, und an den Gate-Anschluss 207a des Kalibrier-Transistors 207 ist ein derartiges elektrisches Signal anlegbar, dass das an den ersten Source-/Drain-Anschluss 205b des Mess-Transistors 205 anlegbare elektrische Potential derart einstellbar ist, dass die Veränderung des Werts des physikalischen Parameters, nämlich der Schwellenspannung des Mess-Transistors 205 des Sensor-Elements, zumindest teilweise kompensierbar ist.

Anschaulich wird bei dem in Fig.4 gezeigten Ausführungsbeispiel des Sensor-Arrays 400 in der Biosensor-Schaltkreis-Anordnung 401 die Kalibrierung des Stroms durch den ersten Source-/Drain-Anschluss 205b des Mess-Transistors 205 nicht, wie in Fig.2, über eine Source-Gegenkopplung durch den Kalibrier-Transistor 207 erreicht, sondern der Kalibrier-Transistor 207 wird in Fig.4 als Element zum Einstellen des elektrischen Potentials des ersten Source-/Drain-Anschlusses 205b des Mess-Transistors 205 genutzt. Damit dieser SteuerMechanismus effizient genutzt werden kann, ist es vorteilhaft, für den Mess-Transistor 205 einen Arbeitspunkt zu wählen, bei dem die Stärke des Stromflusses durch den ersten Source-/Drain-Anschluss 205b des Mess-Transistors 205 ausreichend stark von dem elektrischen Potential an dem ersten Source-/Drain-Anschluss 205b des Mess-Transistors 205 abhängt. Dies ist für Arbeitspunkte im Triodengebiet oder im linearen Bereich der Transistorkennlinie gut erfüllt. Mit anderen Worten arbeitet die Biosensor-Schaltkreis-Anordnung 401 besonders effizient als Kalibrier-Einrichtung, wenn die Spannung zwischen dem Gate-Anschluss 205a und dem zweiten Source-/Drain-Anschluss 205c des Mess-Transistors 205 abzüglich der Schwellenspannung größer als die Spannung zwischen den beiden Source-/Drain-Anschlüssen 205b, 205c des Mess-Transistors 205 ist. Ferner ist dies gut für Transistoren mit einer ausreichend kurzen Kanallänge selbst im Sättigungsbetrieb erfüllt, was eine Folge von Kurzkanaleffekten ist. Was den Kalibrier-Transistor 207 anbetrifft, so ist es sinnvoll, für diesen einen Arbeitspunkt im Sättigungsbereich zu wählen. Gemäß der in Fig.4 gezeigten Konfiguration der Biosensor-Schaltkreis-Anordnung 401 wird der Kalibrier-Transistor 207 als Source-Folger betrieben, das heißt, dass das Potential des zweiten Source-/Drain-Anschlusses 207c des Kalibrier-Transistors 207 gegenüber dem Potential an dem Gate-Anschluss 207a um einen definierten Betrag versetzt ist (das heißt anschaulich dem Potential an dem Gate-Anschluss folgt) und ist weitgehend unabhängig von dem elektrischen Potential an dem ersten Source-/Drain-Anschluss 207b des Kalibrier-Transistors 207.

Es ist anzumerken, dass für alle hier beschriebenen Ausführungsbeispiele des erfindungsgemäßen Sensor-Arrays bzw. der erfindungsgemäßen Biosensor-Schaltkreis-Anordnung die Arbeitspunkte der Transistoren mittels Justierens von Parametern, insbesondere der geometrischen Parameter (z.B. Weite W und Länge L) der Mess-Transistoren und der Kalibrier-Transistoren sowie des in die jeweilige Spalten-Leitung 202 eingeprägten Stromes $I_{in}$ erfolgt.

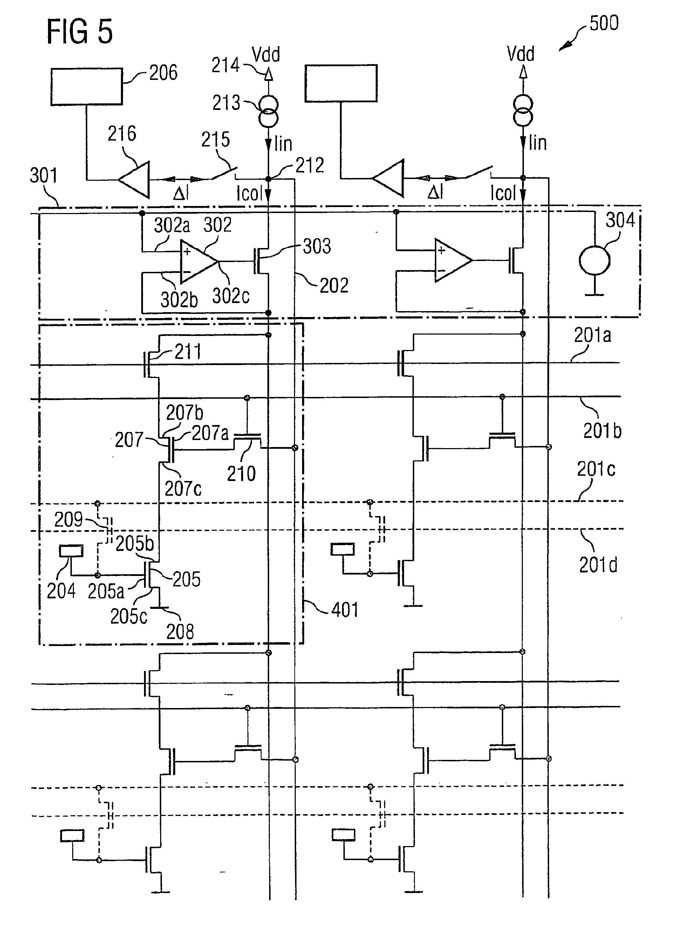

Im Weiteren wird bezugnehmend auf **Fig.5** das dort gezeigte Sensor-Array 500 gemäß einem vierten bevorzugten Ausführungsbeispiel der Erfindung beschrieben.

Das in Fig.5 gezeigte Sensor-Array 500 unterscheidet sich von dem in Fig.4 gezeigten Sensor-Array 400 lediglich darin, dass das Sensor-Array 500 zusätzlich eine Potentialsteuer-Einrichtung 301 aufweist, die oben bezugnehmend auf Fig.3 detailliert beschrieben ist. Diese weist die Funktionalität auf, dass das elektrische Potential der Spalten-Leitungen 202 auf einen konstanten Wert eingeregelt werden kann. Die Funktionalität und die Verschaltung der Potentialsteuer-Einrichtung 301 und der Biosensor-Schaltkreis-Anordnung 401 des Sensor-Arrays 500 ist analog zu der Verschaltung der Potentialsteuer-Einrichtung 301 und der Biosensor-Schaltkreis-Anordnung 203 aus Fig.3 realisiert.

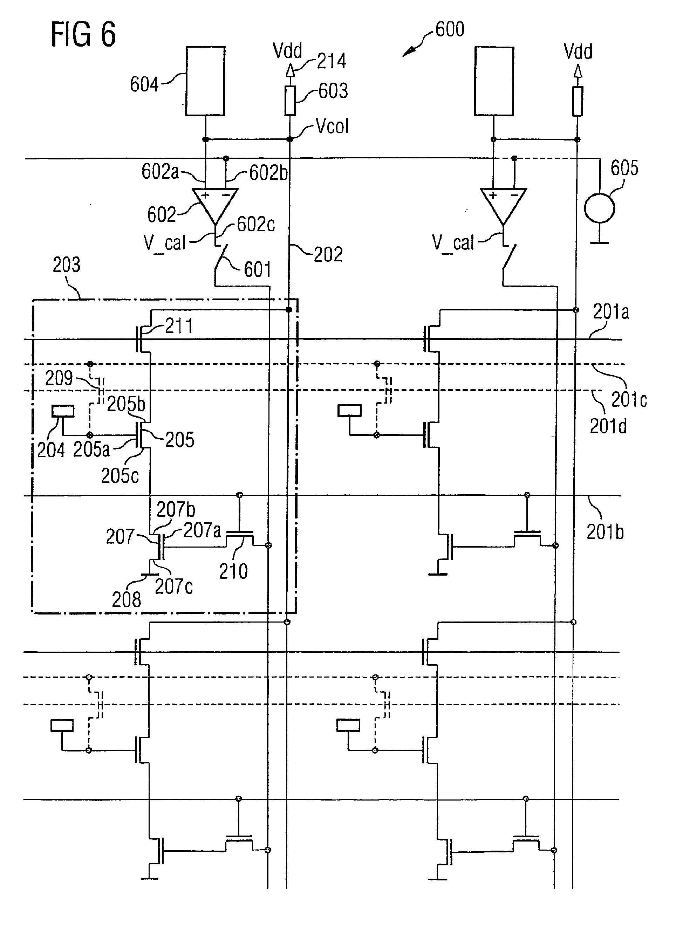

Im Weiteren wird bezugnehmend auf **Fig.6** ein fünftes bevorzugtes Ausführungsbeispiel des erfindungsgemäßen Sensor-Arrays beschrieben.

Das in Fig.6 gezeigte Sensor-Array 600 weist Biosensor-Schaltkreis-Anordnungen 203 auf, von denen jede so ausgebildet ist wie die in Fig.2 gezeigten Biosensor-Schaltkreis-Anordnungen 203. Allerdings ist das im Falle des Sensor-Arrays 600 erfasste Sensor-Signal eine elektrische Spannung und nicht wie bei dem Sensor-Array 200 ein elektrischer Strom. Die Modifikationen, die erforderlich sind, um von dem in Fig.2 gezeigten Sensor-Array 200 auf das in Fig.6 gezeigte Sensor-Array 600 zu gelangen, werden im Weiteren beschrieben.

Wie bereits angesprochen ist die Verschaltung innerhalb der Biosensor-Schaltkreis-Anordnung 203 analog wie in Fig.2 beschrieben. Wie in Fig.6 gezeigt, ist derjenige Source-/Drain-Anschluss des zweiten Schalt-Transistors 210, der mit dem Gate-Anschluss 207a des Kalibrier-Transistors 207 nicht gekoppelt ist, mit einem zweiten Schalter 601 gekoppelt. Der zweite Schalter 601 ist mit dem Ausgang 602c eines Operationsverstärkers 602 gekoppelt, dessen nicht-invertierter Eingang 602a sowohl mit einem Voltmeter 604 als auch mit demjenigen Source-/Drain-Anschluss des dritten Auswahltransistors 211 gekoppelt ist, der nicht mit dem ersten Source-/Drain-Anschluss 205b des Mess-Transistors 205 gekoppelt ist. Der invertierte Eingang 602b des Operationsverstärkers 602 ist mit einer zweiten Spannungsquelle 605 gekoppelt, mittels welcher der invertierte Eingang 602b des Operationsverstärkers 602 auf ein definiertes elektrisches Potential gebracht ist. Ferner ist der nicht-invertierte Eingang 602a des Operationsverstärkers 602 mit einem Anschluss eines Lastelements 603, vorzugsweise einem elektrischen Widerstand, gekoppelt, an dessen anderen Anschluss die Versorgungsspannung 214 angelegt ist. Mit anderen Worten ist bei der in Fig.6 gezeigten Konfiguration verglichen mit der in Fig.2 gezeigten Konfiguration des Sensor-Arrays die Konstantstromquelle 213 durch das Lastelement 603 ersetzt. Ferner ist das Amperemeter 206 durch ein Voltmeter 604 ersetzt. Der Sensor-Strom wird durch den Spannungsabfall an dem Lastelement 603 in eine zu erfassende Spannung umgewandelt. Mit anderen Worten ist bei dem Sensor-Array 600 das Sensor-Signal eine elektrische Spannung.

Der elektrische Strom, der durch eine mittels der ersten Zeilen-Leitung 201a und des dritten Schalt-Transistors 211 ausgewählte Biosensor-Schaltkreis-Anordnung 203 fließt, bewirkt in der in Fig.6 gezeigten Schaltung sowohl während einer Kalibrierphase als auch während einer Messphase einen Spannungsabfall an dem Lastelement 603. Das Ausgangssignal einer Spalten-Leitung 202 ist die elektrische Spalten-Spannung $V_{col}$. Auch bei dem Sensor-Array 600 erfolgt die Auswahl der Biosensor-Schaltkreis-Anordnungen 203 einer Zeile, indem mittels eines elektrischen Signals an einer der ersten Zeilen-Leitungen 201a die zugehörigen dritten Schalt-Transistoren 211 in einen leitenden Zustand gebracht werden. Bei dem Sensor-Array 600 wird vorzugsweise genau eine Zeile von Biosensor-Schaltkreis-Anordnungen 203 zugeschaltet, das heißt, dass an die zugehörige erste Zeilen-Leitung 201a ein die damit gekoppelten dritten Schalt-Transistoren 211 in einen leitenden Zustand versetzendes elektrisches Signal anliegt, wohingegen an den anderen ersten Zeilen-Leitungen 201a (der anderen Zeilen von Biosensor-Schaltkreis-Anordnungen 203) in diesem Falle ein derartiges elektrisches Signal nicht anliegt.

In einer Kalibrierphase zum Kalibrieren der Biosensor-Schaltkreis-Anordnung 203 einer Zeile von Biosensor-Schaltkreis-Anordnungen wird bei der auszuwählenden Zeile an die entsprechende Zeilen-Leitung 201b ein solches elektrisches Signal angelegt, dass dadurch die zugehörigen zweiten Schalt-Transistoren 210 leitend werden, wohingegen an die zweiten Zeilen-Leitungen 201b einer nicht auszuwählenden Zeilen von Biosensor-Schaltkreis-Anordnungen 203 ein derartiges elektrisches Signal nicht angelegt wird. Der zweite Schalter 601 ist in der Kalibrierphase geschlossen. Die Verschaltung der Biosensor-Schaltkreis-Anordnungen 203 mit dem Operationsverstärker 602 und dem Lastelement 603 in der oben beschriebenen Weise bewirkt wiederum einen geschlossenen Steuerkreis. An dem Ausgang 602c des Operationsverstärkers 602 ist eine Spannung bereitgestellt, die über den leitenden zweiten Schalt-Transistor 210 an den Gate-Anschluss 207a des Kalibrier-Transistors 207 angelegt wird. Mittels dieser Spannung ist der Leitfähigkeitszustand des Kalibrier-Transistors 207 festgelegt, der wie bei dem Sensor-Array 200, als steuerbarer Widerstand betrieben wird. Dadurch wird eine Source-Gegenkopplung des Mess-Transistors 205 bewirkt, so dass der elektrische Strom durch den Mess-Transistor 205 und durch den Kalibrier-Transistor 207 derart eingestellt wird, dass sich an dem Lastelement 603 ein solcher Spannungsabfall ergibt, dass sich die Differenzspannung an den Eingängen 602a, 602b des Operationsverstärkers 602 zu Null ergibt, wodurch $V_{col}$ gleich dem mittels der zweiten Spannungsquelle 605 an den invertierten Eingang 602b angelegten elektrischen Potential ist. Dadurch ist bewirkt, dass die Ausgangs-Spannung eines Sensor-Elements bzw. einer Biosensor-Schaltkreis-Anordnung 203, das heißt die elektrische Spannung auf der signalführenden Spalten-Leitung 202, unabhängig von der Schwellenspannung des Mess-Transistors 205 (bzw. eines weiteren oder mehrerer weiterer physikalischer Parameter des Mess-Transistors 205 oder anderer Bauelemente) einer bestimmten Biosensor-Schaltkreis-Anordnung 203 und unabhängig von dem genauen Widerstandswert des Lastelements 603 auf einen vorgegebenen Wert eingestellt ist. Hat sich dieser Zustand in dem Sensor-Array 600 stabil ausgebildet, wird das elektrische Signal an der zweiten Spalten-Leitung 201b entfernt, so dass der zweite Schalt-Transistor 210 nicht leitend wird. Auf der Gate-Kapazität, das heißt auf dem Gate-Anschluss 207a des Kalibrier-Transistors 207 verbleibt jedoch die während der oben beschriebenen Kalibrierphase aufgebrachte Ladung, das heißt, dass das elektrische Potential des Gate-Anschlusses 207a unverändert bleibt, so dass die zugehörige Biosensor-Schaltkreis-Anordnung 203 kalibriert ist.

Nach Abschluss der Kalibrierphase kann der zweite Schalter 601 geöffnet werden. Zwar wird die Öffnung bzw. Deaktivierung des Regelkreises auch dadurch bewirkt, dass das elektrische Signal an der zweiten Zeilen-Leitung 201b abgeschaltet wird, allerdings kann der Betrieb eines nicht geschlossenen Regelkreises zu unerwünscht großen Spannungshüben an dem Ausgang 602c des Operationsverstärkers 602 führen, wodurch Störsignale von während der Kalibrierphase zum Bereitstellen des elektrischen Gate-Potentials des Kalibrier-Transistors 207 genutzten Leitungen auf die signalführenden Leitungen 202 überkoppeln können. Dieser unerwünschte Effekt wird als

Übersprechen bezeichnet und kann durch Öffnen des zweiten Schalters 601 nach Beendigung der Kalibrierphase vermieden werden.

Bei dem Sensor-Array 600 ist der Arbeitspunkt des Mess-Transistors 205 vorzugsweise im Sättigungsbereich zu wählen. Allerdings funktioniert der beschriebene Regelungs- und Kalibriermechanismus auch dann, wenn der Arbeitspunkt des Mess-Transistors 205 außerhalb des Sättigungsbereichs gewählt wird.

In der Messphase führen wiederum Änderungen des Potentials V(E) an der Sensor-Elektrode 204 infolge eines Sensor-Ereignisses, beispielsweise infolge eines Stromes elektrisch geladener Partikel durch die Ionenkanäle der Membran einer Nervenzelle auf die aktive Sensor-Oberfläche des Sensor-Arrays 600, zu einer Änderung des Stromflusses durch den ersten Source-/Drain-Anschluss 205b des Mess-Transistors 205 und somit zu einer Änderung des Stromflusses durch die zugehörige Spalten-Leitung 202, wodurch ein entsprechender Spannungsabfall an dem Lastelement 603 erfolgt.

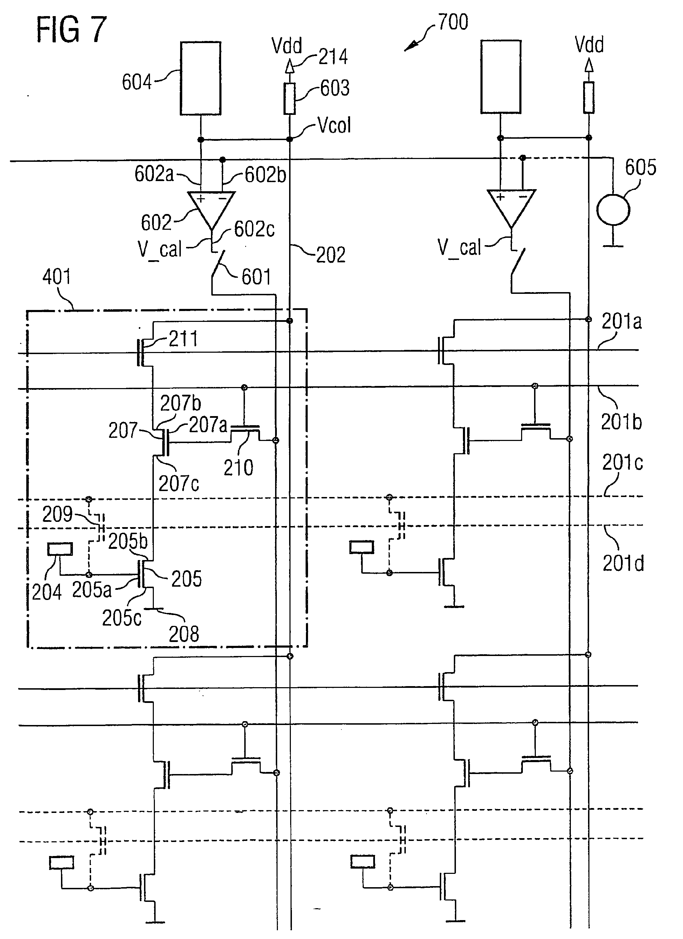

Im Weiteren wird bezugnehmend auf **Fig.7** ein sechstes bevorzugtes Ausführungsbeispiel des erfindungsgemäßen Sensor-Arrays beschrieben.

Das in Fig.7 gezeigte Sensor-Array 700 weist eine Biosensor-Schaltkreis-Anordnung 401 auf, wie dies oben bezugnehmend auf Fig.4 für das Sensor-Array 400 beschrieben worden ist. Ferner ist das Sensor-Signal im Falle des Sensor-Arrays 700 eine elektrische Spannung, wie im Falle des Sensor-Arrays 600. Dabei ist das Lastelement 603 mit demjenigen Source-/Drain-Anschluss des dritten Schalt-Transistors 211 gekoppelt, der nicht mit dem ersten Source-/Drain-Anschluss 207b des Kalibrier-Transistors 207 gekoppelt ist, und der zweite Schalter 601 ist mit demjenigen Source-/Drain-Anschluss des zweiten Schalt-Transistors 210 gekoppelt, der nicht mit dem Gate-Anschluss 207a des Kalibrier-Transistors 207 gekoppelt ist.

Die Funktionalität des Sensor-Arrays 700 ergibt sich aus einer Kombination der obigen Beschreibungen des Sensor-Arrays 400 und des Sensor-Arrays 600. Anschaulich sind die Positionen des Mess-Transistors 205 und des Schalt-Transistors 207 bei dem Sensor-Array 700 gegenüber dem Sensor-Array 600 gegeneinander "vertauscht", das heißt, die Kalibrierung wird bei dem Sensor-Array 700 nicht über eine Source-Gegenkopplung des Mess-Transistors 205 durch den Kalibrier-Transistor 207 erreicht, sondern der Kalibrier-Transistor 207 wird hier als Element zum Einstellen des elektrischen Potentials des ersten Source-/Drain-Anschlusses 205b des Mess-Transistors 205 genutzt. Die Arbeitspunkte der beteiligten Transistoren, insbesondere des Mess-Transistors 205 und des Kalibrier-Transistors 207 sind daher so zu wählen, wie oben für das Sensor-Array 400 beschrieben.

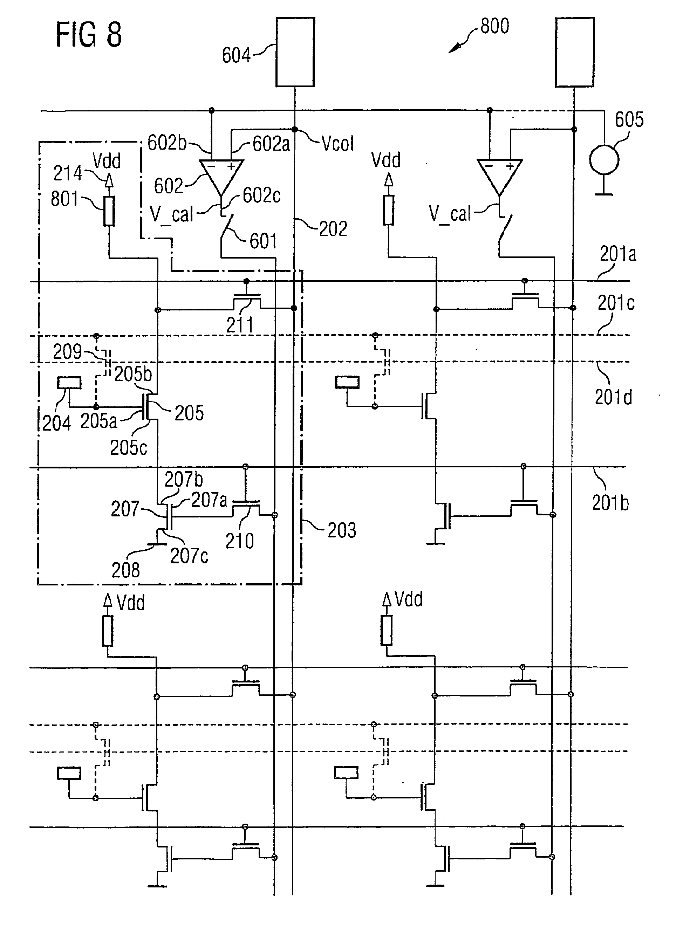

Bezugnehmend auf **Fig.8** wird im Weiteren ein siebtes Ausführungsbeispiel des erfindungsgemäßen Sensor-Arrays beschrieben.

Das in Fig.8 gezeigte Sensor-Array 800 unterscheidet sich nur punktuell von dem in Fig.6 gezeigten Sensor-Array 600. Insbesondere sind die Biosensor-Schaltkreis-Anordnungen 203 in beiden Ausführungsbeispielen der Sensor-Arrays 600, 800 weitgehend identisch ausgebildet, wobei die in Fig.8 gezeigte Biosensor-Schaltkreis-Anordnung 203 verglichen mit der in Fig.6 gezeigten Biosensor-Schaltkreis-Anordnung 203 zusätzlich ein eigenes Lastelement 801 aufweist. Auch wird in beiden Fällen ein Spannungssignal $V_{col}$ als Sensor-Signal erfasst. Auch weist das Sensor-Array 800 den Operationsverstärker 602 auf, der mit der Biosensor-Schaltkreis-Anordnung 203 wie im Falle des Sensor-Arrays 600 verschaltet ist. Allerdings ist bei dem Sensor-Array 800 jeder Biosensor-Schaltkreis-Anordnung 203 ein darin enthaltenes Lastelement 801 zugeordnet, dessen einer Anschluss mit dem ersten Source-/Drain-Anschluss 205b des zugehörigen Mess-Transistors 205 gekoppelt ist, und an dessen anderen Anschluss das elektrische Potential der Versorgungsspannung 214 angelegt ist.

Mit anderen Worten ist das Sensor-Array 800 gegenüber dem Sensor-Array 600 dahingehend verändert, dass nicht ein gemeinsames Lastelement 603 pro Spalten-Leitung 202 von allen Biosensor-Schaltkreis-Anordnungen 203 einer Spalten-Leitung 202 genutzt wird, sondern jede Biosensor-Schaltkreis-Anordnung 203 weist bei dem Sensor-Array 800 ein individuelles Lastelement 801 auf. Es ist zu betonen, dass Parameterschwankungen bei den individuellen Lastelementen 801 durch die Kalibrierung ebenfalls kompensiert werden. Mit anderen Worten kann der ohmsche Widerstand des Lastelements 801 als weiterer physikalischer Parameter angesehen werden, dessen Wert bei unterschiedlichen Biosensor-Schaltkreis-Anordnungen 203 unterschiedlich ist, wobei dieser Unterschied bzw. diese Veränderung des Werts diesen physikalischen Parameters durch die Kalibrierung zumindest teilweise kompensiert wird. Hinsichtlich der Kalibrierphase, der Messphase und des Einstellens bzw. Wählens der Arbeitspunkte der Transistoren, insbesondere des Mess-Transistors 205 und des Kalibrier-Transistors 207, sei auf die obige Beschreibung des Sensor-Arrays 200 verwiesen.

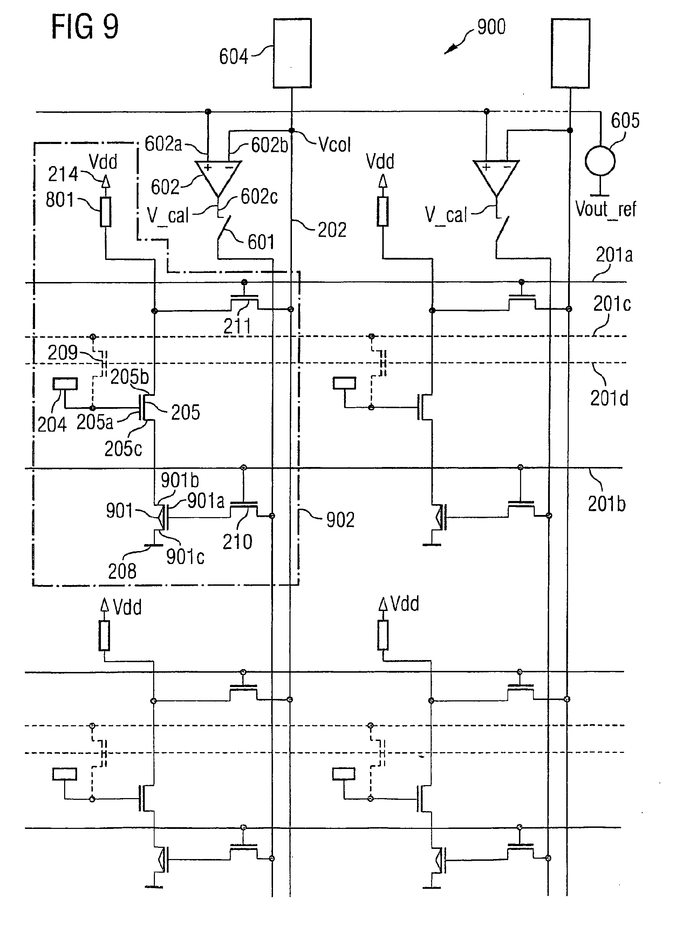

Es sei darauf hingewiesen, dass die Transistoren in den Sensor-Arrays 200 bis 800 alle als n-MOS-Feldeffekttransistoren ausgebildet sind. Dass anstatt eines n-MOS-Transistors mit geringfügigen konstruktiven Änderungen jeder der Transistoren, insbesondere der Mess-Transistor und der Kalibrier-Transistor, als p-MOS-Transistor ausgebildet werden kann, ist exemplarisch in dem in **Fig.9** gezeigten Sensor-Array 900 gezeigt, das im Weiteren beschrieben wird.

Das Sensor-Array 900 ist ähnlich ausgebildet wie das Sensor-Array 800. Im Gegensatz zu dem Sensor-Array 800 weist das Sensor-Array 900 allerdings einen als p-MOS-Transistor ausgebildeten p-MOS-Kalibrier-Transistor 901 auf. Im Weiteren wird bezugnehmend auf Fig.9 beschrieben, wie die Verschaltung der Komponenten des Sensor-Arrays 900 verändert werden muss, um als Kalibrier-Transistor einen p-MOS-Transistor 901 zu verwenden. Bei der Biosensor-Schaltkreis-Anordnung 902 ist der p-MOS-Kalibrier-Transistor 901, der einen Gate-Anschluss 901a, einen ersten Source-/Drain-Anschluss 901b und einen zweiten Source-/Drain-Anschluss 901c aufweist, bezüglich der unmittelbar daran angrenzenden benachbarten Bauelemente verschaltet wie der in Fig.8 gezeigte n-MOS-Kalibrier-Transistor 207. Allerdings ist die Verschaltung des Operationsverstärkers 602 modifiziert, um der Tatsache Rechnung zu tragen, dass der gemäß Fig.8 als n-MOS-Transistor ausgebildete Kalibrier-Transistor im Falle des Sensor-Arrays 900 als p-MOS-Kalibrier-Transistor 901 ausgebildet ist. Wie in Fig.9 gezeigt, ist der invertierte Eingang 602b des Operationsverstärkers 602 (anders als in Fig.8) mit demjenigen Source-/Drain-Anschluss des dritten Schalt-Transistors 211 gekoppelt, der nicht mit dem ersten Source-/Drain-Anschluss 205b des Mess-Transistors 205 gekoppelt ist. Dagegen ist der nicht-invertierte Eingang 602a des Operationsverstärkers 602 mit der zweiten Spannungsquelle 605 gekoppelt. Abgesehen von diesem Aspekt ist die Biosensor-Schaltkreis-Anordnung 902 mit dem p-MOS-Kalibrier-Transistor 901 identisch aufgebaut und verschaltet wie die Biosensor-Schaltkreis-Anordnung 209 aus Fig.8. Mit anderen Worten ist infolge des Ersetzens des n-MOS-Kalibrier-Transistors 207 aus Fig.8 durch den p-MOS-Kalibrier-Transistor 901 aus Fig.9 der invertierte Eingang 602b mit dem nicht-invertierten Eingang 602a des Operationsverstärkers 602 jeder Spalten-Leitung 202 zu vertauschen. Die Möglichkeit, als Transistor für das erfindungsgemäße Sensor-Array wahlweise p-MOS- oder n-MOS-Transistoren verwenden zu können, ermöglicht es, das Sensor-Array auf die Bedürfnisse des Einzelfalls flexibel einstellen zu können. Insbesondere kann Parametern wie dem zur Verfügung stehenden Platzbedarf und anderer Rahmenbedingungen dadurch flexibel Rechnung getragen werden.

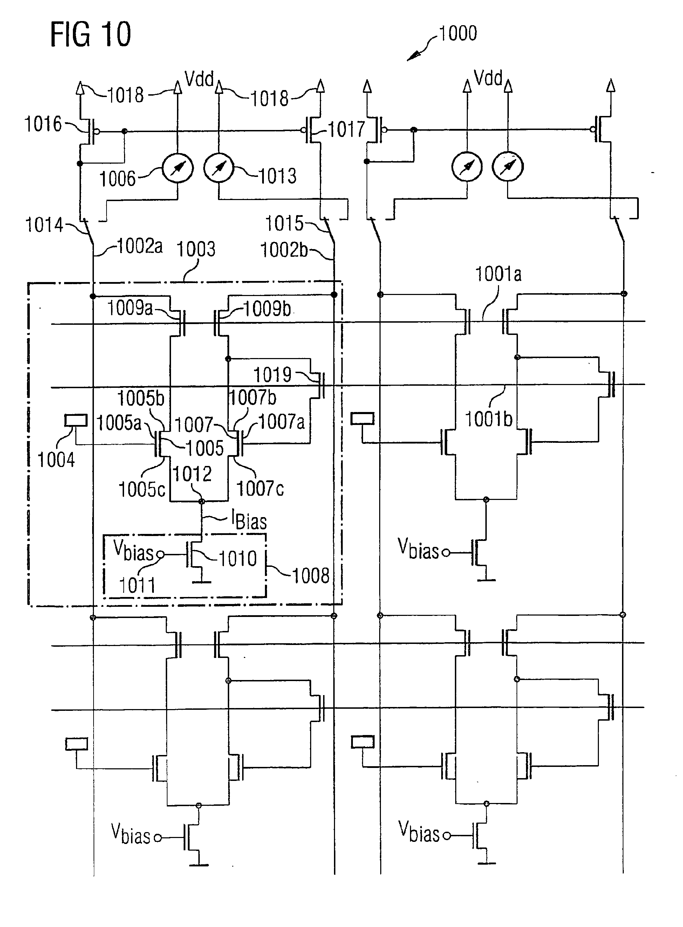

Im Weiteren wird Bezug nehmend auf **Fig.10** ein neuntes bevorzugtes Ausführungsbeispiel des erfindungsgemäßen Sensor-Arrays beschrieben.

Das Sensor-Array 1000 weist eine Mehrzahl von in Kreuzungsbereichen von Zeilen-Leitungen 1001a, 1001b und Spalten-Leitungen 1002a, 1002b im Wesentlichen matrixförmig angeordneten und mit den Zeilen- und Spalten-Leitungen 1001a, 1001b, 1002a, 1002b verschalteten Biosensor-Schaltkreis-Anordnungen 1003 auf. Eine Biosensor-Schaltkreis-Anordnung 1003 ist auf bzw. in einem Substrat (nicht gezeigt in Fig.10) ausgebildet und weist ein Sensor-Element gemäss Anspruch 1 auf.

Das Sensor-Element ist aus einer mit der zu untersuchenden Substanz koppelbaren elektrisch leitfähigen Sensor-Elektrode 1004 und aus einem Mess-Transistor 1005 aufgebaut, dessen Gate-Anschluss 1501 mit der elektrisch leitfähigen Sensor-Elektrode 1004 aus Platin gekoppelt ist. Ferner ist ein erster Source-/Drain-Anschluss 1005b des Mess-Transistors 1005 (über andere Bauelemente) mit einem ersten Amperemeter 1006 koppelbar. Die Kalibrier-Einrichtung jeder Biosensor-Schaltkreis-Anordnung 1003 ist derart eingerichtet, dass mit ihr das an einem zweiten Source-/Drain-Anschluss 1005c des Mess-Transistors 1005 anliegende elektrische Potential steuerbar ist. Ferner weist die Kalibrier-Einrichtung jeder Biosensor-Schaltkreis-Anordnung 1003 einen Kalibrier-Transistor 1007, eine erste Konstantstromquelle 1008, die mit jeweiligen zweiten Source-/-Drain-Anschlüssen 1005c, 1007c der zueinander parallel geschalteten Mess- und Kalibrier-Transistoren 1005, 1007 gekoppelt ist, zum Bereitstellen einer vorgegebenen elektrischen Stromstärke $I_{Bias}$, und eine Stromspiegel-Schaltung auf, die mit jeweiligen ersten Source-/Drain-Anschlüssen 1005b, 1007b der zueinander parallel geschalteten Mess- und Kalibrier-Transistoren 1005, 1007 gekoppelt ist, die derart verschaltet ist, dass mit ihr zum zumindest teilweisen Kompensieren der Veränderung des Wertes des physikalischen Parameters (der Schwellenspannung des Mess-Transistors 1005) das elektrische Potential am Gate-Anschluss 1007a des Kalibrier-Transistors 1007 derart einstellbar ist, dass in Abwesenheit eines Sensor-Ereignisses die Stromflüsse zwischen den beiden Source-/Drain-Anschlüssen dessen Mess-Transistors 1005b, 1005c bzw. Kalibrier-Transistors 1007b, 1007c gleich sind. Es ist darauf hinzuweisen, dass gemäß dem beschriebenen Ausführungsbeispiel jeder Spalte von Biosensor-Schaltkreis-Anordnungen 1003 eine gemeinsame Stromspiegel-Schaltung und weitere gemeinsame Elemente gemeinsam bereitgestellt sind, wie im Weiteren beschrieben.

Im Weiteren wird die Funktionalität des Sensor-Arrays 1000 beschrieben. Wie auch in den zuvor beschriebenen Ausführungsbeispielen kann eine Biosensor-Schaltkreis-Anordnung 1003 ausgewählt werden, indem an die erste Zeilen-Leitung 1001a ein solches elektrisches Signal angelegt wird, dass dadurch ein erster und ein zweiter Auswahltransistor 1009a, 1009b leitend werden. Der erste und der zweite Schalt-Transistor 1009a, 1009b fungieren als Schalter und schließen, wenn an die erste Zeilen-Leitung 1001a ein entsprechendes elektrisches Signal angelegt wird. Die erste Konstantstromquelle 1008 ist aus einem ersten Hilfs-Transistor 1010 und einer Bias-Spannungsquelle 1011 gebildet. Dabei wird der erste Hilfstransistor 1010 in Sättigung betrieben. An den Gate-Anschluss des ersten Hilfs-Transistors 1010 ist das elektrische Potential der Bias-Spannungsquelle 1011 angelegt, ein erster Source-/Drain-Anschluss des ersten Hilfs-Transistors 1008 ist geerdet, und ein zweiter Source-/-Drain-Anschluss des ersten Hilfs-Transistors 1008 ist mit den zweiten Source-/Drain-Anschlüssen 1005c, 1007c des Mess-Transistors 1005 und des Kalibrier-Transistors 1007 gekoppelt. Der von der Konstantstromquelle 1008 einem elektrischen Knoten 1012 bereitgestellte Konstantstrom wird als $I_{Bias}$ bezeichnet. Der Mess-Transistor 1005 wird vorzugsweise in Sättigung betrieben. Ändert sich die Spannung an der Sensor-Elektrode 1004 und somit an dem Gate-Anschluss 1005a des Mess-Transistors 1005, so wird der Stromfluss zwischen den beiden Source-/Drain-Anschlüssen 1005b, 1005c dadurch charakteristisch beeinflusst. Da der Gesamtstrom $I_{Bias}$ durch die Source-/Drain-Anschlüsse 1005b, 1005c des Mess-Transistors 1005 bzw. durch die Source-/ Drain-Anschlüsse 1007b, 1007c des Kalibrier-Transistors 1007 konstant ist, tritt ein Sensor-Signal sowohl im Strompfad des Mess-Transistors als auch im Strompfad des Kalibrier-Transistors 1007 auf. Allerdings führt das Sensor-Signal in einem der beiden parallelen Strompfade zu einer Erhöhung, und in dem anderen Strompfad zu einer entsprechenden Erniedrigung der Stromstärke um einen für das Sensor-Ereignis charakteristischen Differenzwert. Die beiden Stromflüsse durch den Mess-Transistor 1005 bzw. durch den Kalibrier-Transistor 1007 werden durch die leitenden ersten und zweiten Schalt-Transistoren 1009a, 1009b, von denen jeweils ein Source-/Drain-Anschluss mit dem ersten Source-/Drain-Anschluss des Messtransistors bzw. des Kalibrier-Transistors 1005b bzw. 1007b gekoppelt ist, hindurch geleitet und können von dem mit dem anderen Source-/Drain-Anschluss des ersten Schalt-Transistors 1009a gekoppelten ersten Amperemeter 1006 bzw. von einem mit dem anderen Source-/Drain-Anschluss des zweiten Schalt-Transistors 1009b gekoppelten zweiten Amperemeter 1013 erfasst werden, falls weder ein erster Schalter 1014 noch ein zweiter Schalter 1015 in der in Fig.10 gezeigten Stellung (sondern in der dazu komplementären Stellung) sind. Dann kann der Stromfluss durch den Mess-Transistor 1005 mittels des ersten Amperemeters 1006 und der Stromfluss durch den Kalibrier-Transistor 1007 mittels des zweiten Amperemeters 1013 erfasst werden. Das Bilden der Differenz dieser beiden erfassten Stromstärken, das heißt eine differenzielle Signalverarbeitung, bietet wegen einer verbesserten Fehlerrobustheit eine erhöhte Messgenauigkeit, was den bei biologischen Proben zu erwartenden kleinen Messsignalen besonders wichtig ist.

Die Kalibrierung der Biosensor-Schaltkreis-Anordnungen 1003 erfolgt bei dem Sensor-Array 1000 unter Verwendung einer Stromspiegel-Schaltung, die in der in Fig.10 gezeigten Weise verschaltet ist, und die in einen ersten und einen zweiten Stromspiegel-Transistor 1016, 1017 aufweist. Ist der erste Schalter 1014 in der in Fig.10 gezeigten Schalterstellung, so ist derjenige Source-/Drain-Anschluss des ersten Schalt-Transistors 1009a, der mit dem ersten Source-/Drain-Anschluss 1005b des Mess-Transistors 1005 nicht gekoppelt ist, sowohl mit einem Source-/Drain-Anschluss als auch mit dem Gate-Anschluss des ersten Stromspiegel-Transistors 1016 gekoppelt. Ferner ist der Gate-Anschluss des ersten Stromspiegel-Transistors 1016 mit dem Gate-Anschluss des zweiten Stromspiegel-Transistors 1017 gekoppelt. In der in Fig.10 gezeigten Schalterstellung des zweiten Schalters 1015 ist derjenige Source-/Drain-Anschluss des zweiten Hilfs-Transistors 1009b, der mit dem ersten Source-/Drain-Anschluss des Kalibrier-Transistors 1007 nicht gekoppelt ist, mit einem Source-/Drain-Anschluss 1007b des zweiten Stromspiegel-Transistors 1017 gekoppelt. Ferner ist bei dem Sensor-Array 1000 an die jeweils anderen Source-/Drain-Anschlüsse des ersten Stromspiegel-Transistors 1016 und des zweiten Stromspiegel-Transistors 1017 sowie an jeweils einen Anschluss der beiden Amperemeter 1006, 1013 das elektrische Potential einer Versorgungsspannung 1018 angelegt.

Im Weiteren wird beschrieben, wie unter Verwendung der beiden Stromspiegel-Transistoren 1016, 1017, bzw. deren Verschaltung mit den Biosensor-Schaltkreis-Anordnungen 1003 in der in Fig.10 gezeigten Weise eine Kalibrierung durchgeführt wird. Im Kalibrierbetrieb ist die Schalterstellung des ersten Schalters 1014 und des zweiten Schalters 1015 so, wie in Fig.10 gezeigt. Um eine bestimmte Biosensor-Schaltkreis-Anordnung 1003 zum Kalibrieren auszuwählen, wird an eine entsprechende erste Zeilen-Leitung 1001a ein entsprechendes elektrisches Signal angelegt, so dass der erste und der zweite Schalt-Transistor 1009a, 1009b leitend sind. Aufgrund von Schwankungen der elektrischen Transistorparameter des Mess-Transistors 1005 und des Kalibrier-Transistors 1007 (beispielsweise die Schwellenspannung) wird der Stromfluss durch die beiden Zweige des Differenzpaares, das heißt zwischen den beiden Source-/Drain-Anschlüssen 1005b, 1005c des Mess-Transistors 1005 einerseits und zwischen den beiden Source-/Drain-Anschlüssen 1007b, 1007c des Kalibrier-Transistors 1007 andererseits selbst dann im Allgemeinen nicht gleich sein, wenn an die Gate-Anschlüsse 1005a bzw. 1007a des Mess-Transistors 1005 bzw. des Kalibrier-Transistors 1007 ein identisches elektrisches Potential angelegt ist. Mittels der Stromspiegel-Schaltung wird der Ausgangsstrom durch die Source-/Drain-Anschlüsse des Mess-Transistors 1005 invertiert und mit dem Strom durch die Source-/Drain-Anschlüsse des Kalibrier-Transistors 1007 aus dem anderen Pfad verglichen. Tritt zwischen diesen beiden Stromstärken eine Differenz auf, die von Null verschieden ist, so ändert sich bei einem infolge eines elektrischen Signals an einer der zweiten Spalten-Leitungen 1001b leitenden dritten Schalt-Transistors 1019 das Potential am Gate-Anschluss 1007a des Kalibrier-Transistors 1007, bis in beiden Pfaden des Differenzpaares der gleiche Strom fließt. Der Gate-Anschluss des dritten Schalt-Transistors 1019 ist mit der zweiten Zeilen-Leitung 1001b gekoppelt, seine beiden Source-/Drain-Anschlüsse sind zwischen den Gate-Anschluss 1007a des Kalibrier-Transistors 1007 und den zweiten Schalt-Transistor 1009b geschaltet. Entfernt man das elektrische Signal an der zweiten Spalten-Leitung 1000b, so wird der dritte Schalt-Transistor 1019 nicht-leitend und die Kalibrierspannung bzw. die Kalibrierladung verbleibt an dem Gate-Anschluss 1007a des Kalibrier-Transistors 1007, so dass auf beiden Pfaden, das heißt, durch den Mess-Transistor 1005 einerseits und den Kalibrier-Transistor 1007 andererseits, der gleiche Strom $I_{Bias}/2$ fließt. Dadurch ist die Biosensor-Schaltkreis-Anordnung 1003 kalibriert.

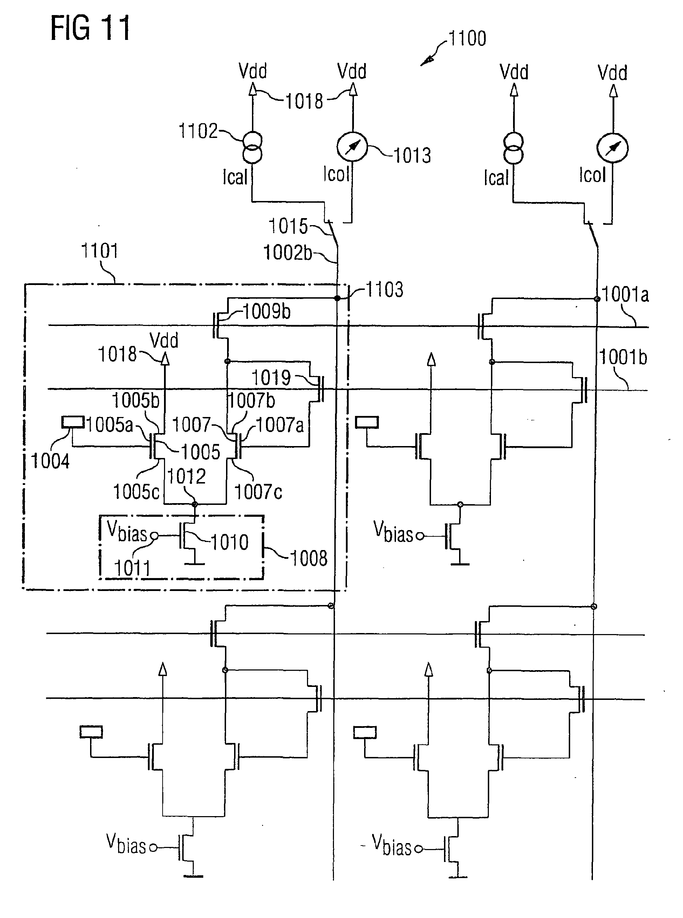

In **Fig.11** ist ein zehntes Ausführungsbeispiel des erfindungsgemäßen Sensor-Arrays gezeigt.

Das Sensor-Array 1100 weist wiederum eine Mehrzahl von im Wesentlichen matrixförmig angeordneten Biosensor-Schaltkreis-Anordnungen 1101 auf, die verglichen mit den Biosensor-Schaltkreis-Anordnungen 1003 in Fig.10 modifiziert sind. Insbesondere ist an den ersten Source-/Drain-Anschluss 1005b des Mess-Transistors 1005 das elektrische Potential einer Versorgungsspannung 1018 angelegt. Die Komponenten der Stromspiegel-Schaltung in dem Strompfad des Mess-Transistors 1005 sind im Falle des Sensor-Arrays 1100 entbehrlich. Die Verschaltung des Strompfads, in dem der Kalibrier-Transistor 1007 angeordnet ist, entspricht innerhalb der Biosensor-Schaltkreis-Anordnung 1101 der Konfiguration aus Fig.10. Mit anderen Worten ist ein Sensor-Signal der Biosensor-Schaltkreis-Anordnung 1101 an den zweiten Schalter 1015 anlegbar, der entweder in der in Fig.11 gezeigten Stellung ist, wenn das Sensor-Array 1100 in der Kalibrierphase betrieben wird, oder in der dazu entgegengesetzten Stellung ist, wenn das Sensor-Array 1100 in der Messphase betrieben wird.

In der Kalibrierphase ist derjenige Source-/Drain-Anschluss des zweiten Schalt-Transistors 1009b, der mit dem ersten Source-/Drain-Anschluss 1007b des Kalibrier-Transistors 1007 nicht gekoppelt ist, mit einem Anschluss der zweiten Konstantstromquelle 1102 gekoppelt, deren anderer Anschluss auf das elektrische Potential der Versorgungsspannung 1018 gebracht ist. In der Messphase hingegen ist der beschriebene Source-/Drain-Anschluss des zweiten Schalt-Transistors 1009b mit einem Anschluss des zweiten Amperemeters 1013 gekoppelt.

Bei der Biosensor-Schaltkreis-Anordnung 1101 ist an den ersten Source-/-Drain-Anschluss 1005b des Mess-Transistors 1005 also das Potential der Versorgungsspannung 1018 angelegt, und die Kalibrier-Einrichtung weist auf: den Kalibrier-Transistor 1007 mit dem ersten Source-/Drain-Anschluss 1007b und dem zweiten Source-/Drain-Anschluss 1007c, die erste Konstantstromquelle 1008, die mit den zweiten Source-/Drain-Anschlüssen 1005c bzw. 1007c der zueinander parallel geschalteten Mess- und Kalibrier-Transistoren 1005, 1007 gekoppelt ist, zum Bereitstellen einer vorgebbaren elektrischen Stromstärke, und eine zweite Konstantstromquelle 1102, die mit dem ersten Source-/Drain-Anschluss 1007b des Kalibrier-Transistors 1007 koppelbar ist, zum Bereitstellen einer weiteren vorgebbaren elektrischen Stromstärke, welche zweite Konstantstromquelle 1102 derart verschaltet ist, dass mit ihr zum zumindest teilweisen Kompensieren der Veränderung des Werts des physikalischen Parameters die an den Anschlüssen der Transistoren 1005, 1007 anlegbaren Potentiale derart einstellbar sind, dass in Abwesenheit eines Sensor-Ereignisses an der Sensor-Elektrode 1004 die Stromflüsse zwischen den beiden Source-/Drain-Anschlüssen 1005b, 1005c des Mess-Transistors 1005 und zwischen den beiden Source-/Drain-Anschlüssen 1007b, 1007c des Kalibrier-Transistors 1007 gleich sind.

Anschaulich ist bei dem Sensor-Array 1100 im Gegensatz zu dem Sensor-Array 1000 nur einer der beiden Strompfade des Mess-Transistors 1005 und des Kalibrier-Transistors 1007, nämlich gemäß Fig.11 nur der Strompfad zwischen den Source-/Drain-Anschlüssen 1007b, 1007c des Kalibrier-Transistors 1007 aus der Biosensor-Schaltkreis-Anordnung 1101 herausgeführt. Eine Zeile von Biosensor-Schaltkreis-Anordnungen 1101 wird wiederum ausgewählt, indem an die erste Zeilen-Leitung 1001a ein derartiges elektrisches Signal angelegt wird, dass dadurch der zweite Schalt-Transistor 1009b leitend wird. An dem gemäß Fig.11 oberen Randabschnitt des Sensor-Arrays 1100 wird dann mittels des zweiten Amperemeters 1013 in der entsprechenden Schalterstellung (entgegengesetzte Schalterstellung des zweiten Schalters 1015 wie in Fig.11) der Stromfluss durch den Strompfad des Kalibrier-Transistors 1007 gemessen und ausgewertet.

Die Kalibrierung erfolgt bei dem Sensor-Array 1100 durch Einprägen eines Referenzstromes $I_{cal}$. Vorzugsweise ist dieser Kalibrierstrom halb so groß wie der Strom der ersten Konstantstromquelle 1008 der Biosensor-Schaltkreis-Anordnung 1101, das heißt $I_{cal} = I_{Bias}/2$. Dann ist sichergestellt, dass auch in dem Messzweig des Differenzpaares mit dem Mess-Transistor 1005 ein Strom $I_{Bias}/2$ fließt. Um auch statistische Schwankungen des Konstantstroms $I_{Bias}$ (beispielsweise aufgrund einer Variation der Schwellenspannung des ersten Hilfs-Transistors 1010) zu kompensieren, kann dieser zunächst an einem Randabschnitt des Sensor-Arrays 1100 gemessen werden. Hierzu ist in der Kalibrierphase die elektrische Spannung an einem elektrischen Ausgangsknoten 1103 einer Biosensor-Schaltkreis-Anordnung 1101 und somit an dem Gate-Anschluss 1007a des Kalibrier-Transistors 1007 so hoch gewählt, dass der gesamte oder annähernd der gesamte Strom $I_{Bias}$ der ersten Konstantstromquelle 1008 durch diesen Pfad fließt. Im Anschluss prägt man diesem Pfad einen Strom $I_{cal}<I_{Bias}$ ein, wodurch sich ein Strom durch den Mess-Transistor 1005 des Werts $I_{Bias}-I_{cal}$ ergibt. Nach Beendigung der Kalibrierphase, das heißt nach Entfernen des elektrischen Signals an der zweiten Zeilen-Leitung 1001b, aufgrund welchen Signals der dritte Schalt-Transistor 1019 zuvor leitend war, wird der dritte Schalt-Transistor 1019 nicht leitend und daher bleibt der zuvor eingeprägte Ladungszustand an dem Gate-Anschluss 1007a des Kalibrier-Transistors 1007 gespeichert und die Biosensor-Schaltkreis-Anordnung 1101 ist kalibriert.

Das in Fig.11 gezeigte Sensor-Array 1100 weist gegenüber dem in Fig.10 gezeigten Sensor-Array den Vorteil auf, dass einige Komponenten eingespart sind, so dass das Sensor-Array 1100 in der Fertigung weniger aufwendig ist. Dagegen weist das in Fig.10 gezeigte Sensor-Array 1100 in Folge der differenzierten Strommessung eine besonders hohe Nachweisempfindlichkeit auf.

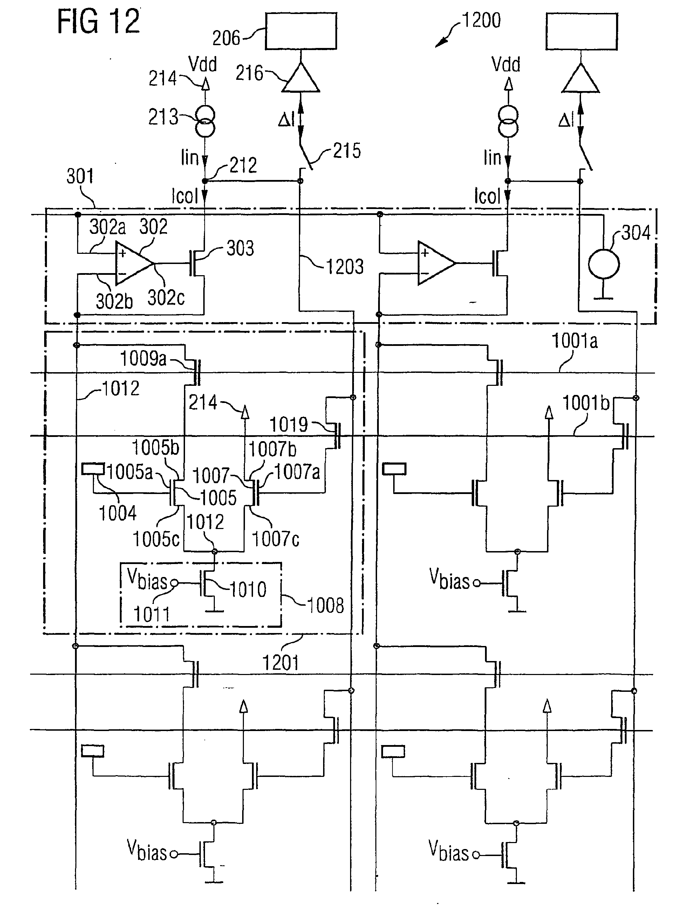

In **Fig.12** ist ein Sensor-Array 1200 gemäß einem elften bevorzugten Ausführungsbeispiel der Erfindung gezeigt.

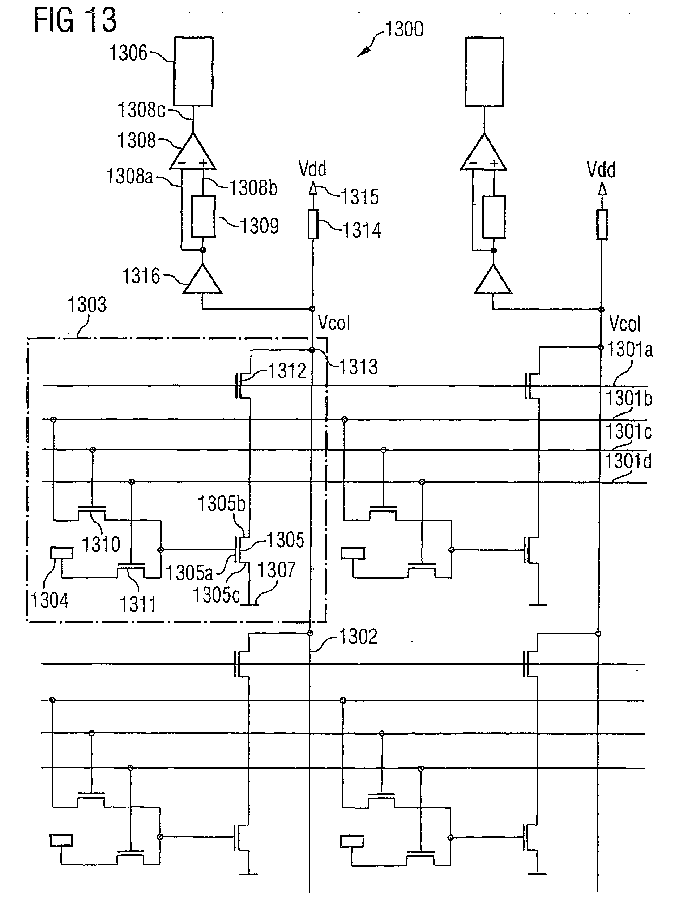

Das Sensor-Array 1200 weist eine Vielzahl von matrixförmig angeordneten Biosensor-Schaltkreis-Anordnungen 1201 auf, die teilweise so aufgebaut und verschaltet sind, wie die in Fig.11 gezeigten Biosensor-Schaltkreis-Anordnungen 1101. Allerdings ist bei dem Sensor-Array 1200 eine Potentialsteuer-Einrichtung 301 zum Konstanthalten des Potentials der Spalten-Leitungen 1202 bereitgestellt. Bei dem Sensor-Array 1200 ist eine Kalibrierspannung ähnlich wie in Figuren 6 bis 9 über eine zusätzliche Spalten-Leitung 1203 zugeführt, und das Potential der Spalten-Leitung 1202 wird analog zu dem Konzept von Fig.3 mittels einer Potentialsteuer-Einrichtung 301 auf einem konstanten elektrischen Potential gehalten, das von der ersten Spannungsquelle 304 bereitgestellt ist. An den ersten Source-/Drain-Anschluss 1007b des Kalibrier-Transistors 1007 ist das elektrisch Potential der Versorgungsspannung 214 angelegt. Im Weiteren wird Bezug genommen auf Fig.13 bis Fig.15, anhand derer ein anderes Konzept beschrieben wird, mit dem eine Veränderung des Werts eines physikalischen Parameters eines Sensor-Elements zumindest teilweise kompensierbar ist.

Zunächst wird das in **Fig.13** gezeigte Sensor-Array 1300 beschrieben.

Das Sensor-Array 1300 weist eine Mehrzahl von in Kreuzungsbereichen von Zeilen-Leitungen 1301a, 1301b, 1301c, 1301d und Spalten-Leitungen 1302 im Wesentlichen matrixförmig angeordneten und mit den Zeilen- und Spalten-Leitungen 1301a, 1301b, 1301c, 1301d, 1302 verschalteten Biosensor-Schaltkreis-Anordnungen 1303 auf. Wie bereits bei den oben beschriebenen Ausführungsbeispielen weist jede Biosensor-Schaltkreis-Anordnung 1303 ein Sensor-Element mit einem physikalischen Parameter und eine Kalibrier-Einrichtung auf, die derart eingerichtet ist, dass mit ihr eine Veränderung des Werts des physikalischen Parameters des Sensor-Elements zumindest teilweise kompensierbar ist. Das Sensor-Element der Biosensor-Schaltkreis-Anordnung 1303 weist eine mit einer zu untersuchenden Substanz (nicht gezeigt in der Figur) koppelbare elektrisch leitfähige Elektrode 1304 auf. Ferner weist das Sensor-Element der Biosensor-Schaltkreis-Anordnung 1303 einen Mess-Transistor 1305 auf, dessen Gate-Anschluss 1305a mit der elektrisch leitfähigen Sensor-Elektrode 1304 (über einen weiteren Transistor 1311, der weiter unten beschrieben wird) gekoppelt ist. Ferner ist in Fig.13 für jede Spalten-Leitung 1302 ein Voltmeter 1306 zum Erfassen einer elektrischen Sensor-Spannung bereitgestellt, welches Voltmeter 1306 mit einem ersten Source-/Drain-Anschluss 1305b des Mess-Transistors 1305 koppelbar ist.

Die Kalibrier-Einrichtung der Biosensor-Schaltkreis-Anordnung 1303 ist derart eingerichtet, dass mit ihr ein von dem Sensor-Ereignis bewirktes Sensor-Signal des Sensor-Elements unter Verwendung des Prinzips der korrelierten Doppelabtastung (Correlated Double Sampling, CDS) in einen Wert umwandelbar ist, der von dem Wert des physikalischen Parameters des Sensor-Elements unabhängig ist.

Bei der Biosensor-Schaltkreis-Anordnung 1303 ist an einen zweiten Source-/Drain-Anschluss 1305c des Mess-Transistors 1305 ein elektrisches Massepotential 1307 angelegt. Die Kalibrier-Einrichtung weist einen Differenzverstärker 1308 mit einem ersten Eingang 1308a und einem zweiten Eingang 1308b und einem Ausgang 1308c auf, welcher Ausgang 1308c mit dem Voltmeter 1306 gekoppelt ist, welcher erste Eingang 1308a mit dem ersten Source-/Drain-Anschluss 1305b des Mess-Transistors 1305 koppelbar ist und welcher Differenzverstärker derart eingerichtet ist, dass an seinem Ausgang 1308c die Differenz zwischen zwei an den beiden Eingängen (dem ersten, invertierten Eingang 1308a und dem zweiten, nicht invertierten Eingang 1308b) angelegten elektrischen Signalen bereitstellbar ist. Ferner weist die Kalibrier-Einrichtung ein zwischen den ersten Source-/Drain-Anschluss 1305b des Mess-Transistors 1305 und den zweiten Eingang 1308b des Differenzverstärkers 1308 geschaltetes Abtast-Halte-Glied 1309 auf. Die Kalibrier-Einrichtung ist derart eingerichtet, dass in einem ersten Betriebszustand in das Abtast-Halte-Glied 1309 ein von dem physikalischen Parameter des Sensor-Elements (das heißt der Schwellenspannung des Mess-Transistors 1305) abhängiges Sensor-Signal einprägbar ist und dem zweiten Eingang 1308b des Differenzverstärkers 1308 bereitstellbar ist. Ferner ist die Kalibrier-Einrichtung derart eingerichtet, dass in einem zweiten Betriebszustand dem ersten Eingang 1308a des Differenzverstärkers 1308 ein für den physikalischen Parameter des Sensor-Elements charakteristisches Signal bereitstellbar ist. Die Kalibrier-Einrichtung ist ferner derart eingerichtet, dass in dem Ausgang 1308c des Differenzverstärkers 1308 ein von dem Wert des physikalischen Parameters des Sensor-Elements unabhängiges Sensor-Signal, eine elektrische Spannung, bereitstellbar ist, wodurch die Veränderung des Wertes des physikalischen Parameters zumindest teilweise kompensiert ist.

Ferner weist die Biosensor-Schaltkreis-Anordnung 1303 auf einen ersten Schalt-Transistor 1310 und einen zweiten Schalt-Transistor 1311. Die Sensor-Elektrode 1304 ist mit dem ersten Source-/-Drain-Anschluss des zweiten Schalt-Transistors 1311 gekoppelt, und der andere Source-/Drain-Anschluss des zweiten Schalt-Transistors 1311 ist mit dem Gate-Anschluss 1305a des Mess-Transistors 1305 und mit dem ersten Source-/Drain-Anschluss des ersten Schalt-Transistors 1310 gekoppelt. Der zweite Source-/Drain-Anschluss des ersten Schalt-Transistors 1310 ist mit der zweiten Zeilen-Leitung 1301b gekoppelt, und der Gate-Anschluss des ersten Schalt-Transistors 1310 ist mit der dritten Zeilen-Leitung 1301c gekoppelt. Ferner ist der Gate-Anschluss des zweiten Schalt-Transistors 1311 mit der vierten Zeilen-Leitung 1301d gekoppelt. Die Biosensor-Schaltkreis-Anordnung 1303 weist einen dritten Schalt-Transistor 1312 auf, dessen erster Source-/Drain-Anschluss mit dem ersten Source-/Drain-Anschluss 1305b des Mess-Transistors 1305 gekoppelt ist. Der Gate-Anschluss des dritten Schalt-Transistors 1312 ist mit der ersten Zeilen-Leitung 1301a gekoppelt, und der zweite Source-/Drain-Anschluss des dritten Schalt-Transistors 1312 ist mit einem elektrischen Kreuzungspunkt 1313 gekoppelt, der mit einem Anschluss eines Lastelements 1314 gekoppelt ist, an dessen anderen Anschluss eine Versorgungsspannung 1315 angelegt ist. Ferner ist der elektrische Kreuzungspunkt 1313 über einen Verstärker 1316 sowohl mit dem invertierten ersten Eingang 1308a des Differenzverstärkers 1308 als auch mit dem Abtast-Halte-Glied 1309 gekoppelt.

Im Weiteren wird das Prinzip der korrelierten Doppelabtastung (Correlated Double Sampling) erklärt, das beispielsweise in [4] beschrieben ist. Gemäß diesem Konzept ist das Dämpfen von Rauschanteilen und das Unterdrücken des Offset-Anteils ermöglicht. Dabei wird häufig der Eingang eines Verstärkers mit einer zu messenden Signalquelle gekoppelt. Am Ausgang des Verstärkers wird dann das verstärkte Signal plus einem Offsetsignal des Verstärkers gemessen und abgespeichert. In einer nächsten Phase wird der Verstärker mit einer geeigneten Referenzquelle gekoppelt. An dem Ausgang des Verstärkers liegt dann nur noch der Offsetanteil an. Mittels Differenzbildung der beiden Ausgangsspannungen ist der Offsetanteil des Verstärkers eliminierbar, so dass das von dem Offset des Verstärkers freie Signal erhalten wird.

Um das Sensor-Array 1300 mit dem Konzept des Correlated Double Sampling zu betreiben, werden die Zeilen von den Biosensor-Schaltkreis-Anordnungen 1303 nacheinander ausgelesen. Zum Auslesen einer Zeile von Biosensor-Schaltkreis-Anordnungen 1303 wird an die zugehörige erste Zeilen-Leitung 1301a ein derartiges elektrisches Signal angelegt, dass dadurch der dritte Schalt-Transistor 1312 leitend wird. Dadurch ist der erste Source-/-Drain-Anschluss 1305b des Mess-Transistors 1305 mit der Ausleseschaltung im oberen Bereich des Sensor-Arrays 1300 aus Fig.13 gekoppelt. Die eigentliche Messung erfolgt in zwei Phasen:

In der ersten Phase wird an die vierte Zeilen-Leitung 1301d ein derartiges elektrisches Signal angelegt, dass dadurch der zweite Schalt-Transistor 1311 leitend wird. Wird infolge eines Sensor-Ereignisses an der Sensor-Elektrode 1304 das Potential der Sensor-Elektrode 1304 verändert, so bilden die Helmholtz-Schicht an der Sensor-Elektrode 1304 und die Gate-Kapazität des Mess-Transistors 1305 einen Spannungsteiler. Es ist in diesem Zusammenhang anzumerken, dass parallel zu der Helmholtz-Schicht im elektrischen Ersatzschaltbild ein ohmscher Widerstand geschaltet sein kann, der die elektrische Leitfähigkeit des Elektrolyten repräsentiert. Als Helmholtz-Schicht wird eine oberhalb einer elektrisch geladenen Elektrode sich ausbildende Schichtenfolge von Schichten mit alternierend elektrisch geladenen Partikel bezeichnet. Anschaulich erfolgt eine Sortierung der Ionen in mehreren Schichten, so dass beispielsweise in der unmittelbaren Umgebung einer positiv geladenen Elektrode eine Schicht mit negativ geladenen Ionen befindlich ist, gefolgt von einer etwas weiter von der Elektrode entfernten Schicht von positiven Ionen, dann wieder einer Schicht von negativen Ionen, usw. Die Definiertheit der Schichten nimmt mit zunehmenden Abstand von der Elektrodenoberfläche ab. Eine solche Helmholtz-Schicht kann als Kapazität aufgefasst werden. Unter der Voraussetzung, dass die Kapazität einer Helmholtz-Schicht deutlich größer als die Kapazität an dem Gate-Anschluss 1305a des Mess-Transistors 1305 ist, fällt annähernd die gesamte Spannung am Gate-Anschluss 1305a des Mess-Transistors 1305 ab. Der Mess-Transistor 1305 wird in dieser Konfiguration in Source-Schaltung betrieben, eine Änderung des Potentials an dem Gate-Anschluss 1305a bewirkt eine um einen bestimmten Faktor verstärkte Änderung des Potentials an dem ersten Source-/Drain-Anschluss 1305b. Dieser Verstärkungsfaktor hängt von dem Produkt aus der Steilheit der Transistorkennlinie des Mess-Transistors 1305 und dem Wert des Widerstands ab. Diese Spannungsänderung wird über den Verstärker 1316 außerhalb der Biosensor-Schaltkreis-Anordnung 1303, wobei für jede Spalten-Leitung 1302 jeweils ein gemeinsamer Verstärker 1316 ausgebildet ist, verstärkt. Sowohl der Mess-Transistor 1035 als auch der Verstärker 1316 weisen einen Offset auf, der sich zu dem Sensor-Signal addiert. Die Ausgangsspannung des Verstärkers 1316 wird in dem Abtast-Halte-Glied 1309 abgespeichert.

Nun wird das an die vierte Zeilen-Leitung 1301d angelegte elektrische Signal entfernt, so dass der zweite Schalt-Transistor 1311 sperrt.

In der zweiten Phase der Messung wird an die dritte Zeilen-Leitung 1301c ein derartiges elektrisches Signal angelegt, dass der erste Schalt-Transistor 1310 leitet. Dann ist die an der zweiten Zeilen-Leitung 1301b angelegte Referenzspannung an den Gate-Anschluss 1305a des Mess-Transistors 1305 angelegt. In diesem Fall liegt an dem Ausgang des Verstärkers 1316 nur noch der oben beschriebene Offset des Mess-Transistors 1305 bzw. des Verstärkers 1316 selbst an. An dem nicht-invertierten Eingang 1308b des Differenzverstärkers 1308 liegt daher das Sensor-Signal plus dem Offsetanteil aus der ersten Messphase an, wohingegen an dem invertierten ersten Eingang 1308a des Differenzverstärkers 1308 allein der Offsetanteil aus der zweiten Messphase anliegt. Das Differenzsignal zwischen den Eingängen 1308a, 1308b des Differenzverstärkers 1308 ist daher das reine Sensor-Signal ohne den Offsetanteil. Dadurch ist die Veränderung des Werts des physikalischen Parameters des Sensor-Elements der Biosensor-Schaltkreis-Anordnung 1303 kompensiert.

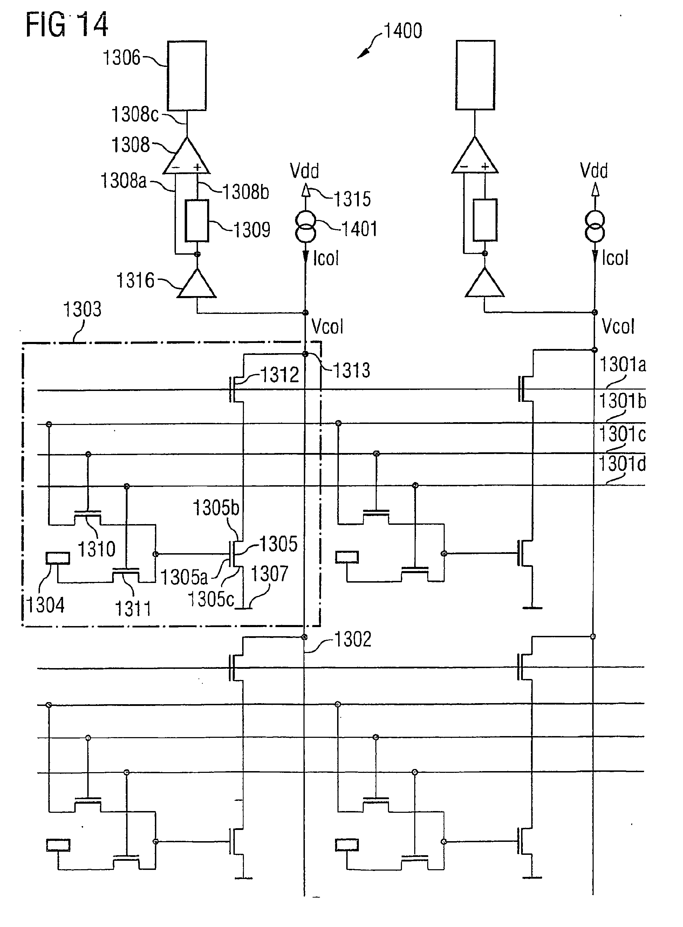

Im Weiteren wird Bezug genommen auf **Fig.14** und ein dreizehntes bevorzugtes Ausführungsbeispiel des erfindungsgemäßen Sensor-Arrays beschrieben.

Das in Fig.14 gezeigte Sensor-Array 1400 unterscheidet sich von dem in Fig.13 gezeigten Sensor-Array 1300 dadurch, dass das Lastelement 1314, ein elektrischer Widerstand, durch eine Konstantstromquelle 1401 ersetzt ist. Das Prinzip der korrelierten Doppelabtastung (Correlated Double Sampling) ist bei dem Sensor-Array 1400 analog wie bei dem Sensor-Array 1300, wobei die Verstärkung der Änderung der Gate-Spannung an dem Mess-Transistor 1305 nun aus dem Quotienten von Steilheit und Ausgangsleitwert des Mess-Transistors 1305 resultiert.

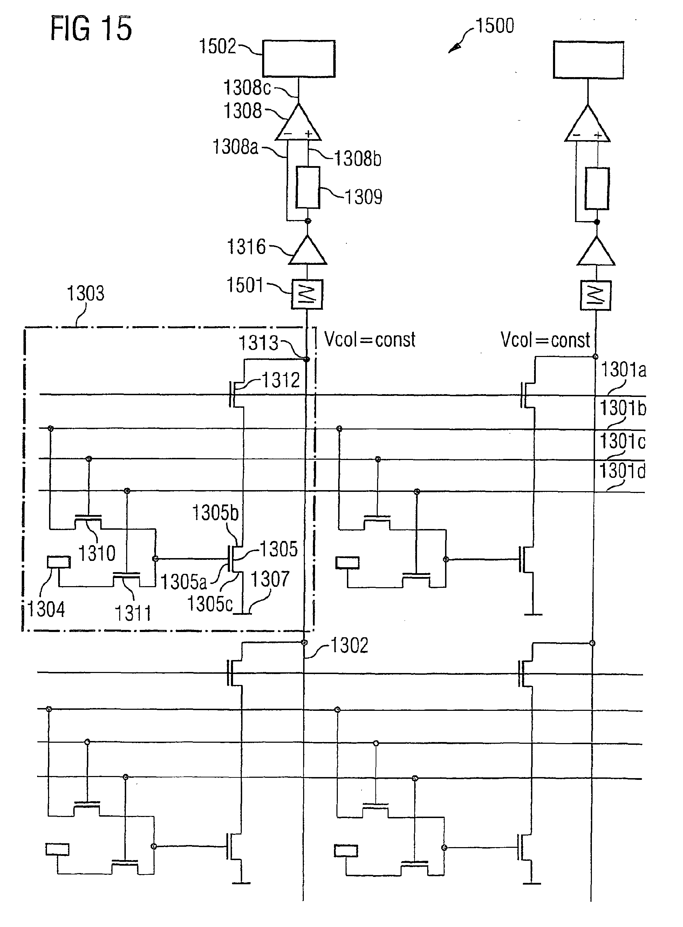

In **Fig.15** ist ein Sensor-Array 1500 gemäß einem vierzehnten Ausführungsbeispiel der Erfindung gezeigt.

Das Sensor-Array 1500 stellt andere Abwandlung des Sensor-Arrays 1300 aus Fig.13 dar. Bei dem Ausführungsbeispiel von Fig.15 sind das Lastelement 1314 und eine Versorgungsspannung 1315 entbehrlich, allerdings ist zwischen den Knoten 1313 und dem Verstärker 1316 ein Strom-Spannungs-Wandler 1501 geschaltet. Der Strom-Spannungs-Wandler 1501 ist gemeinsam für alle Biosensor-Schaltkreis-Anordnungen 1303 einer Spalten-Leitung 1302 ausgebildet.

Mit anderen Worten wird gemäß dem in Fig.15 gezeigten Ausführungsbeispiel des erfindungsgemäßen Sensor-Arrays 1500 mittels des Mess-Transistors 1305 im Strom- anstelle eines Spannungs-Signals ausgelesen. Dabei wird die elektrische Spannung $V_{col}$ auf den Spalten-Leitungen 1302 konstant gehalten, das heißt, es müssen parasitäre Kapazitäten nicht umgeladen werden, und die Schaltung kann schneller ausgelesen werden. Die Änderung des Stromflusses durch den ersten Source-/Drain-Anschluss 1305b des Mess-Transistors 1305 wird mittels des Strom-Spannungs-Wandlers 1501 in eine elektrische Spannung umgewandelt. Auch gemäß dem in Fig.15 gezeigten Ausführungsbeispiel wird die Differenz aus dem verstärkten Messsignal und dem verstärkten Referenzsignal gebildet, wobei letzteres den Offset des Mess-Transistors 1305 infolge einer Veränderung der Schwellenspannung widerspiegelt, und es wirken sich auch in diesem Fall die Offsetgrößen nicht auf das Ausgangssignal des Differenzverstärkers 1308 an dem Ausgang 1308c aus. Das Sensor-Spannungssignal kann beispielsweise mittels eines Voltmeters 1502 erfasst werden.

Es ist zu betonen, dass das Prinzip der korrelierten Doppelabtastung (CDS) auch mittels einer komplexeren Verstärker-Einrichtung beispielsweise auf Basis von Differenzstufen etc. realisiert werden kann.

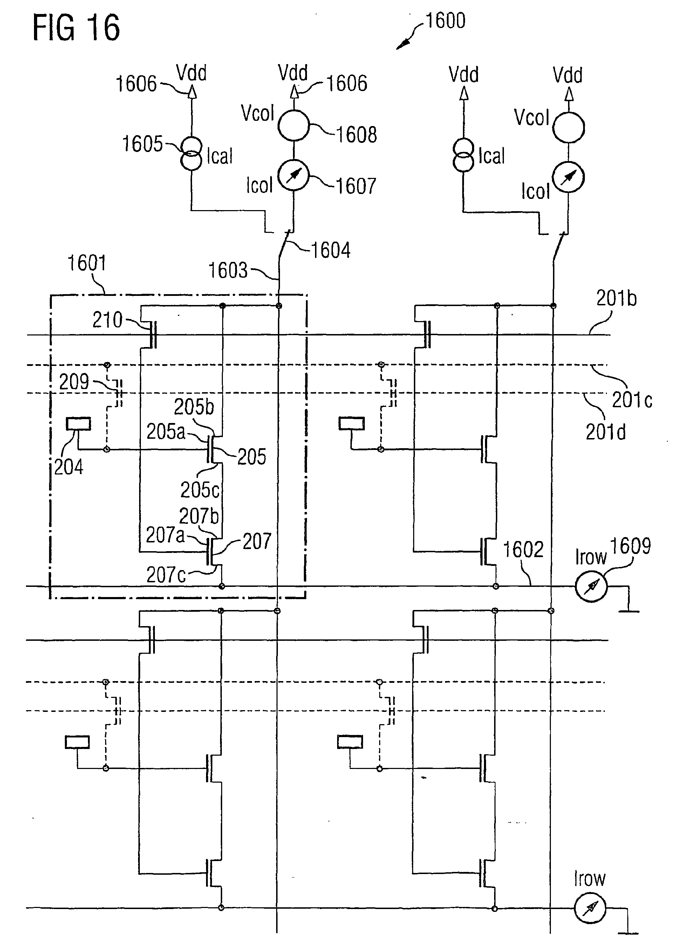

Die bezugnehmend auf in Fig.2 bis Fig.15 beschriebenen Ausführungsbeispiele der erfindungsgemäßen Sensor-Arrays betreffen jeweils ein Szenario, bei dem selektiv eine einzelne Biosensor-Schaltkreis-Anordnung ausgewählt wird, um deren Sensor-Signal zu erfassen. Im Weiteren werden bezugnehmend auf Fig.16 bis Fig.19 Ausführungsbeispiele des erfindungsgemäßen Sensor-Arrays beschrieben, bei denen Summenströme von Einzelsensor-Strömen von Biosensor-Schaltkreis-Anordnungen einer Zeilen-Leitung bzw. einer Spalten-Leitung erfasst werden.

In **Fig.16** ist ein Sensor-Array 1600 mit einer Mehrzahl von in Kreuzungsbereichen von Zeilen-Leitungen 201b, 201c, 201d bzw. 1602 und Spalten-Leitungen 1603 im Wesentlichen matrixförmig angeordneten und mit den Zeilen- und Spalten-Leitungen 201b, 201c, 201d, 1602, 1603 verschalteten Biosensor-Schaltkreis-Anordnungen 1601 gezeigt.

Die Biosensor-Schaltkreis-Anordnungen 1600 sind ausgestaltet wie die in Fig.2 gezeigten Biosensor-Schaltkreis-Anordnungen 203 mit dem Unterschied, dass gemäß dem in Fig.16 gezeigten Ausführungsbeispiel eine erste Zeilen-Leitung 201a entbehrlich ist, da gemäß diesem Ausführungsbeispiel ein Summenstrom mehrerer, entlang einer Zeile bzw. Spalte angeordneter Biosensor-Schaltkreis-Anordnungen 1601 erfasst wird. Ein Auswählen einer Biosensor-Schaltkreis-Anordnung einer Spalten-Leitung 1603 ist daher in Fig.16 nicht erforderlich. Statt dessen ist eine Summenstrom-Zeilen-Leitung 1602 ausgebildet, die mit den zweiten Source-/Drain-Anschlüssen 207c des Kalibrier-Transistors 207 einer Zeile von Biosensor-Schaltkreis-Anordnungen 1601 gekoppelt ist. Entlang einer Summenstrom-Spalten-Leitung 1603 summieren sich die einzelnen Sensor-Strom-Signale der Biosensor-Schaltkreis-Anordnungen 1601 der zugehörigen Spalte an einem Schalter 1604 in einem Endabschnitt einer jeden Spalten-Leitung 1603 auf. Mit dem Schalter 1604 ist ein Anschluss einer Konstantstromquelle 1605 gekoppelt, an deren anderen Anschluss eine Versorgungsspannung 1606 angelegt ist. Ferner ist der Schalter 1604 mit einem ersten Amperemeter 1607 gekoppelt, das wiederum mit einer Spannungsquelle 1608 gekoppelt ist, an deren einem Anschluss das Potential einer Versorgungsspannung 1606 angelegt ist. Ferner ist an einem Endabschnitt jeder Summenstrom-Spalten-Leitung 1602 ein zweites Amperemeter 1609 angeordnet.

Bei dem in Fig.16 gezeigten Sensor-Array 1600 wird also nicht eine bestimmte Biosensor-Schaltkreis-Anordnung 1601 pro Spalten-Leitung 1603 ausgewählt, wie in den zuvor beschriebenen Ausführungsbeispielen, sondern statt dessen werden Summenströme von mehreren Biosensor-Schaltkreis-Anordnungen 1601 entlang einer Summenstrom-Zeilen-Leitung 1602 oder entlang einer Summenstrom-Spalten-Leitung 1603 erfasst und ausgewertet. Um Parameterschwankungen des Mess-Transistors 205 in unterschiedlichen Biosensor-Schaltkreis-Anordnungen 1601 zu kompensieren, werden die Kalibrier-Transistoren 207 in den Strompfad geschaltet, wobei auf die Gate-Anschlüsse 207a der Kalibrier-Transistoren 207 in einer Kalibrierphase eine elektrische Ladung zum Kompensieren einer Veränderung des Wertes des physikalischen Parameters (Schwellen spannung des Messtransistors) eines Sensor-Elements (bzw. eises Mess-Transistors 205) aufgebracht wird.

In einer Kalibrierphase wird der Schalter 1604 so gelegt, dass die Summenstrom-Spalten-Leitung 1603 mit der Konstantstromquelle 1605 gekoppelt ist. Das heißt, dass ein vordefinierter Strom $I_{cal}$ in eine Biosensor-Schaltkreis-Anordnung 1601 einer Spalten-Leitung 1603 eingeprägt wird, welche Biosensor-Schaltkreis-Anordnung 1601 durch ein elektrisches Signal an der zweiten Zeilen-Leitung 201b ausgewählt wird, mittels welchen Signals bewirkt wird, dass der zweite Schalt-Transistor 210 leitet. Bei einer vorgegebenen elektrischen Spannung an der Summenstrom-Zeilen-Leitung 1602 sind die zweiten Source-/Drairi-Anschlüsse 207c der Kalibrier-Transistoren 207b einer Zeile von Biosensor-Schaltkreis-Anordnungen 1601 gekoppelt, und es stellt sich an dem Gate-Anschluss 207a des Kalibrier-Transistors 207 eine Spannung ein, durch welche die zu kalibrierende Biosensor-Schaltkreis-Anordnung 1601 in einen Zustand versetzt wird, genau den eingeprägten Strom zu führen. Wiederum bewirkt der Kalibrier-Transistor 207 eine Source-Gegenkopplung des Mess-Transistors 205. Hat sich an dem Gate-Anschluss 207a eine Kalibrier-Ladung eingestellt, so wird das elektrische Signal an der zweiten Zeilen-Leitung 201b, mittels welchen Signals zuvor der zweite Schalt-Transistor 210 leitend gemacht wurde, abgeschaltet, so dass der zweite Schalt-Transistor 210 sperrt. Auf der Gate-Kapazität des Kalibrier-Transistors 207 verbleibt jedoch die während der Kalibrierphase aufgebrachte Ladung, so dass das elektrische Potential an dem Gate-Anschluss 207a des Kalibrier-Transistors 207 unverändert bleibt. Dadurch ist die zugehörige Biosensor-Schaltkreis-Anordnung kalibriert.

Während des Messbetriebs wird der Schalter 1604 in die in Fig.16 gezeigte Stellung, das heißt in die zu der während der Kalibrierphase eingestellten Stellung komplementären Stellung gebracht und an keiner der zweiten Zeilen-Leitungen 201b liegt ein elektrisches Signal, das den zweiten Schalt-Transistor 210 leitend machen würde. Änderungen des Potentials an der Sensor-Elektrode 204 V(E) führen zu einer Änderung des Stroms durch den ersten Source-/Drain-Anschluss 205b des Mess-Transistors 205 und daher zu einer Änderung des Beitrages einer Biosensor-Schaltkreis-Anordnung 1601 zu den Summenströmen in den zugeordneten Summenstrom-Zeilen-Leitung 1602 bzw. Summenstrom-Spalten-Leitung 1603.

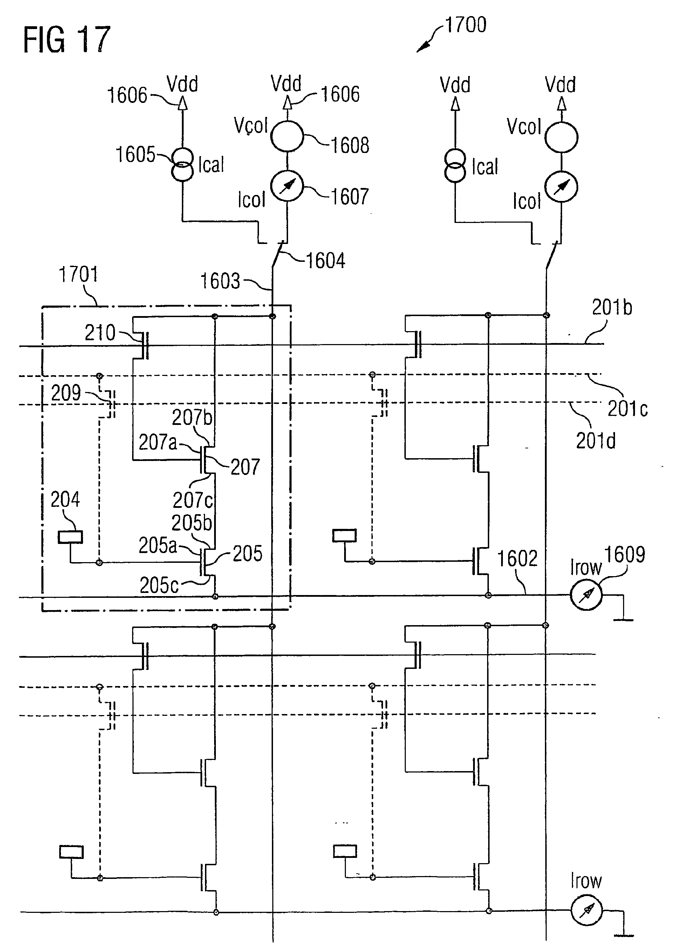

In **Fig.17** ist ein Sensor-Array 1700 gemäß einem sechzehnten Ausführungsbeispiel der Erfindung gezeigt.

Das Sensor-Array 1700 unterscheidet sich von dem in Fig.16 gezeigten Sensor-Array 1600 lediglich dadurch, dass die Biosensor-Schaltkreis-Anordnung 1701 nicht wie in Fig.16 als Source-Gegenkopplung ausgestaltet ist, sondern entsprechend der in Fig.4 gezeigten Weise, nämlich derart, dass der Kalibrier-Transistor 207 als Source-Folger ausgebildet ist. Mit anderen Worten erfolgt im Falle von Fig.17 die Kalibrierung nicht über eine Source-Gegenkopplung des Mess-Transistors 205 durch den Kalibrier-Transistor 207, sondern in diesem Falle ist der Kalibrier-Transistor 207 ein Element zum Einstellen des Potentials des ersten Source-/Drain-Anschlusses 205b des Mess-Transistors 205.

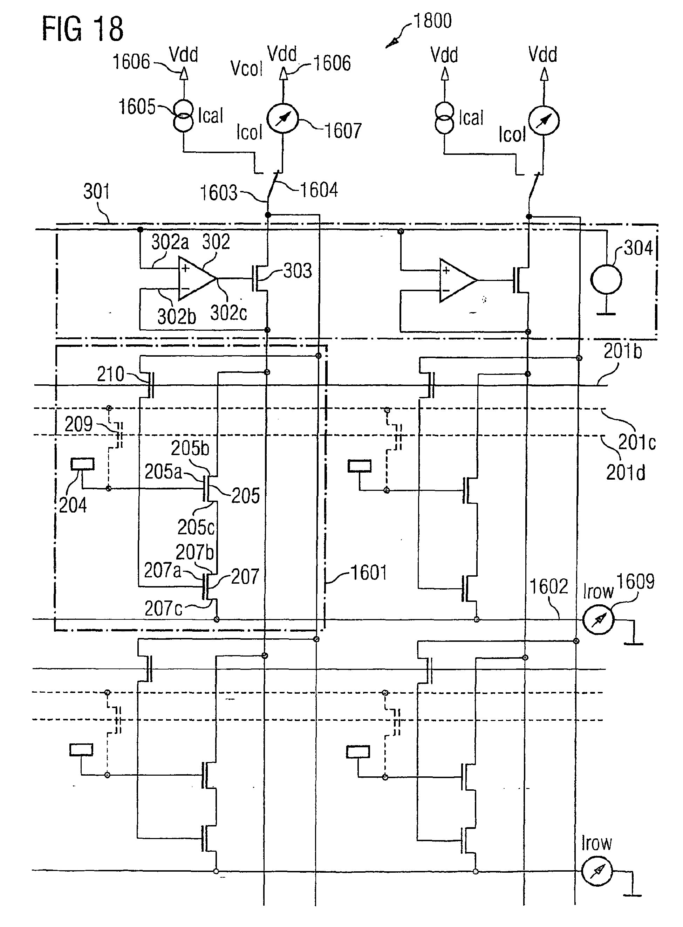

In **Fig.18** ist ein Sensor-Array 1800 gemäß einem siebzehnten Ausführungsbeispiel der Erfindung gezeigt. Das Sensor-Array 1800 entspricht weitgehend dem in Fig.16 gezeigten Sensor-Array 1600, weist jedoch zusätzlich eine Potentialsteuer-Einrichtung 301 auf, die oben bezugnehmend auf Fig.3 detailliert beschrieben ist. Mittels der Potentialsteuer-Einrichtung 301 kann das elektrische Potential der signalführenden Spalten-Leitungen 1603 sowohl während der Kalibrierphase als auch während der Messphase konstant gehalten werden.

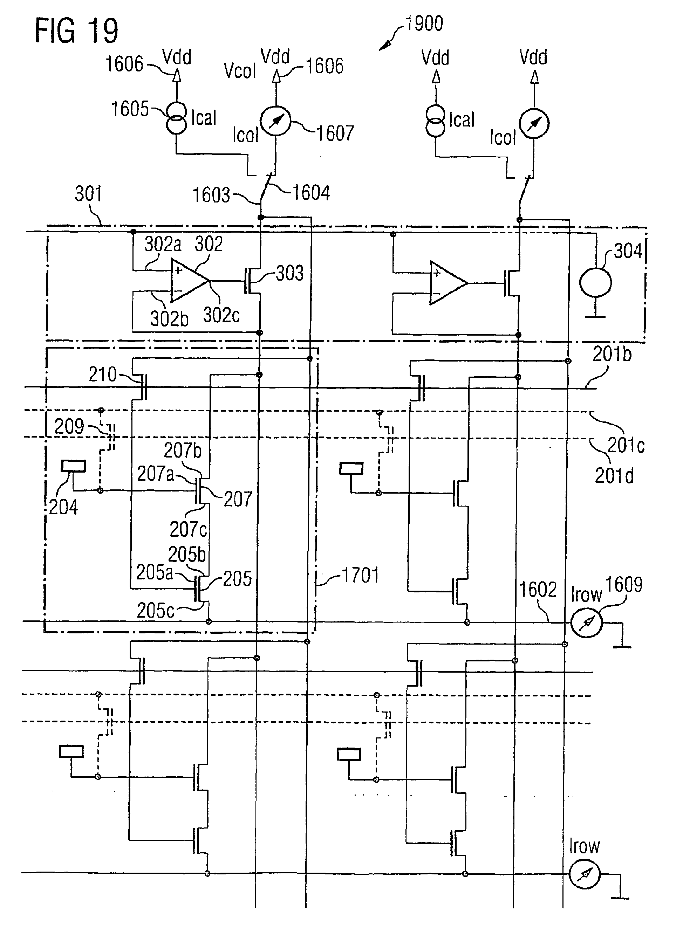

In **Fig.19** ist ein Sensor-Array 1900 gemäß einem achtzehnten Ausführungsbeispiel der Erfindung gezeigt.

Das in Fig.19 gezeigte Ausführungsbeispiel entspricht dem in Fig.17 gezeigten Ausführungsbeispiel, wobei zusätzlich eine Potentialsteuer-Einrichtung 301 zum Konstanthalten des elektrischen Potentials der stromführenden Spalten-Leitung 1603 bereitgestellt ist.

Das Auswerten von Summenstrom-Signalen entsprechend der Sensor-Arrays von Fig.16 bis Fig.19 erfolgt beispielsweise unter Verwendung einer im Weiteren beschriebenen Korrelationsrechnung. Zunächst wird für zumindest einen Teil der Summenstrom-Zeilen-Leitungen 1602 und der Summenstrom-Spalten-Leitungen 1603 der jeweilige Summenstrom der jeweiligen Zeilen-Leitung bzw. Spalten-Leitung erfasst, welcher Summenstrom Einzel-Ströme der entlang einer der zugehörigen Leitung angeordneten Biosensor-Schaltkreis-Anordnungen enthält. Da ein Sensor-Ereignis in einer Biosensor-Schaltkreis-Anordnung in einem Kreuzungsbereich einer Zeilen-Leitung und einer Spalten-Leitung in diesen beiden Leitungen korreliert auftritt, kann aus den Summenströmen auf diejenige Biosensor-Schaltkreis-Anordnungen geschlossen werden, an denen ein Sensor-Ereignis erfolgt ist. Insbesondere können zu diesem Zweck die

**EP 1 438 571 B1**

zeitabhängigen Summenströme der Zeilen-Leitungen und der Spalten-Leitungen Fourier-transformiert werden, die Fourier-transformierten Summenströme jeweils einer Zeilen-Leitung und jeweils einer Spalten-Leitung paarweise miteinander multipliziert werden und das Produkt einer Fourier-Rücktransformation unterzogen werden. Aus den Fourier-rücktransformierten Stromprodukten ist eine mögliche Korrelation zwischen einem Summenstrom-Signal einer Zeilen -Leitung und einer Spalten-Leitung ermittelbar, und es ist ermittelbar, ob an der Biosensor-Schaltkreis-Anordnung in dem jeweiligen Kreuzungsbereich der für die Korrelationsrechnung verwendeten Zeilen- und Spalten-Leitung ein Sensor-Ereignis stattgefunden hat oder nicht.

In diesem Dokument sind folgende Veröffentlichungen zitiert:

[1] Thomas, CA et al. (1972) "A miniature microelectrode array to monitor the bioelectric activity of cultured cells" Exp.Cell.Res. 74:61-66

[2] Gross, GW et al. (1995) "The use of neuronal networks on multielectrode arrays as biosensors" Biosensors&Bioelectronics 10:553-567

[3] Berdondini, L et al. "High-Density MEA for Electrophysiological Activity Imaging of Neuronal Networks" Proc. ICECS 2001, 1239-1242, September 2001

[4] Enz, CC et al. (1996) "Circuit techniques for reducing the effects of op-amp imperfections: autozeroing, correlated double sampling, and chopper stabilization", Proceedings of the IEEE 84(11):1584ff

[5] DE 43 20 881 A1

Bezugszeichenliste

| | |
|---|---|
| 100 | elektrisch adressierbares MEA |
| 101 | Glas-Substrat |
| 102 | Begrenzungswand |
| 103 | aktiver Sensorbereich |
| 104 | Sensor-Felder |
| 105 | elektrische Zuleitungen |
| 106 | Kontaktflächen |
| 200 | Sensor-Array |
| 201a | erste Zeilen-Leitung |
| 201b | zweite Zeilen-Leitung |
| 201c | dritte Zeilen-Leitung |
| 201d | vierte Zeilen-Leitung |
| 202 | Spalten-Leitung |
| 203 | Biosensor-Schaltkreis-Anordnung |
| 204 | Sensor-Elektrode |
| 205 | Mess-Transistor |
| 205a | Gate-Anschluss |
| 205b | erster Source-/Drain-Anschluss |
| 205c | zweiter Source-/Drain-Anschluss |
| 206 | Amperemeter |
| 207 | Kalibrier-Transistor |
| 207a | Gate-Anschluss |
| 207b | erster Source-/Drain-Anschluss |
| 207c | zweiter Source-/Drain-Anschluss |
| 208 | Masse-Potential |
| 209 | erster Schalt-Transistor |
| 210 | zweiter Schalt-Transistor |
| 211 | dritter Schalt-Transistor |
| 212 | erster elektrischer Knoten |
| 213 | Konstantstromquelle |
| 214 | Versorgungsspannung |

| | |
|---|---|
| 215 | erster Schalter |
| 216 | erster Verstärker |
| 300 | Sensor-Array |
| 301 | Potentialsteuer-Einrichtung |
| 302 | Operationsverstärker |
| 302a | nicht-invertierter Eingang |
| 302b | invertierter Eingang |
| 302c | Ausgang |
| 303 | vierter Schalt-Transistor |
| 304 | erste Spannungsquelle |
| 400 | Sensor-Array |
| 401 | Biosensor-Schaltkreis-Anordnung |
| 500 | Sensor-Array |
| 600 | Sensor-Array |
| 601 | zweiter Schalter |
| 602 | Operationsverstärker |
| 602a | nicht-invertierter Eingang |
| 602b | invertierter Eingang |
| 602c | Ausgang |
| 603 | Lastelement |
| 604 | Voltmeter |
| 605 | zweite Spannungsquelle |
| 700 | Sensor-Array |
| 800 | Sensor-Array |
| 801 | Lastelement |
| 900 | Sensor-Array |
| 901 | p-MOS-Kalibrier-Transistor |
| 901a | Gate-Anschluss |
| 901b | erster Source-/Drain-Anschluss |
| 901c | zweiter Source-/Drain-Anschluss |
| 902 | Biosensor-Schaltkreis-Anordnung |
| 1000 | Sensor-Array |
| 1001a | erste Zeilen-Leitung |
| 1001b | zweite Zeilen-Leitung |
| 1002a | erste Spalten-Leitung |
| 1002b | zweite Spalten-Leitung |
| 1003 | Biosensor-Schaltkreis-Anordnung |
| 1004 | Sensor-Elektrode |
| 1005 | Mess-Transistor |
| 1005a | Gate-Anschluss |
| 1005b | erster Source-/Drain-Anschluss |
| 1005c | zweiter Source-/Drain-Anschluss |
| 1006 | erstes Amperemeter |
| 1007 | Kalibrier-Transistor |
| 1007a | Gate-Anschluss |
| 1007b | erster Source-/Drain-Anschluss |
| 1007c | zweiter Source-/Drain-Anschluss |
| 1008 | erste Konstantstromquelle |
| 1009a | erster Schalt-Transistor |
| 1009b | zweiter Schalt-Transistor |
| 1010 | erster Hilfs-Transistor |
| 1011 | Bias-Spannungsquelle |
| 1012 | elektrischer Knoten |
| 1013 | zweites Amperemeter |
| 1014 | erster Schalter |
| 1015 | zweiter Schalter |
| 1016 | erster Stromspiegel-Transistor |
| 1017 | zweiter Stromspiegel-Transistor |

| | |
|---|---|
| 1018 | Versorgungsspannung |
| 1019 | dritter Schalt-Transistor |
| 100 | Sensor-Array |
| 1101 | Biosensor-Schaltkreis-Anordnung |
| 1102 | zweite Konstantstromquelle |
| 1103 | elektrischer Ausgangsknoten |
| 1200 | Sensor-Array |
| 1201 | Biosensor-Schaltkreis-Anordnung |
| 1202 | Spalten-Leitung |
| 1203 | zusätzliche Spalten-Leitung |
| 1300 | Sensor-Array |
| 1301a | erste Zeilen-Leitung |
| 1301b | zweite Zeilen-Leitung |
| 1301c | dritte Zeilen-Leitung |
| 1301d | vierte Zeilen-Leitung |
| 1302 | Spalten-Leitung |
| 1303 | Biosensor-Schaltkreis-Anordnung |
| 1304 | Sensor-Elektrode |
| 1305 | Mess-Transistor |
| 1305a | Gate-Anschluss |
| 1305b | erster Source-/Drain-Anschluss |
| 1305c | zweiter Source-/Drain-Anschluss |
| 1306 | Voltmeter |
| 1307 | Masse-Potential |
| 1308 | Differenzverstärker |
| 1308a | erster Eingang |
| 1308b | zweiter Eingang |
| 1308c | Ausgang |
| 1309 | Abtast-Halte-Glied |
| 1310 | erster Schalt-Transistor |
| 1311 | zweiter Schalt-Transistor |
| 1312 | dritter Schalt-Transistor |
| 1313 | 1313 elektrischer Kreuzungspunkt |
| 1314 | Lastelement |
| 1315 | Versorgungsspannung |
| 1316 | Verstärker-Element |
| 1400 | Sensor-Array |
| 1401 | Konstantstromquelle |
| 1500 | Sensor-Array |
| 1501 | Strom-Spannungs-Wandler |
| 1502 | Voltmeter |
| 1600 | Sensor-Array |
| 1601 | Biosensor-Schaltkreis-Anordnung |
| 1602 | Summenstrom-Zeilen-Leitung |
| 1603 | Summenstrom-Spalten-Leitung |
| 1604 | Schalter |
| 1605 | Konstantstromquelle |
| 1606 | Versorgungsspannung |
| 1607 | erstes Amperemeter |
| 1608 | Spannungsquelle |
| 1609 | zweites Amperemeter |
| 1700 | Sensor-Array |
| 1701 | Biosensor-Schaltkreis-Anordnung |
| 1800 | Sensor-Array |
| 1900 | Sensor-Array |

**Patentansprüche**

1. Schaltkreis-Anordnung (203)

   • mit einem Substrat;
   • mit einem in oder auf einem Oberflächen-Bereich des Substrats ausgebildeten Sensor-Element mit einem physikalischen Parameter, das mit einer zu untersuchenden Substanz gekoppelt ist, wobei die Art der Kopplung einen ohmschen Anteil aufweist, wobei das Sensor-Element eine mit der zu untersuchenden Substanz gekoppelte elektrisch leitfähige Sensor-Elektrode (204) aufweist, wobei das Sensor-Element einen Mess-Transistor (205) aufweist, dessen Gate-Anschluss (205a) mit der elektrisch leitfähigen Sensor-Elektrode gekoppelt ist und wobei der physikalische Parameter die Schwellenspannung des Mess-Transistors ist;
   • mit einer in oder auf dem Substrat ausgebildeten Kalibrier-Einrichtung, die derart eingerichtet ist, dass mit ihr eine individuelle Abweichung des Werts des physikalischen Parameters des Sensor-Elements von einem Referenzwert zumindest teilweise kompensierbar ist.

2. Schaltkreis-Anordnung nach Anspruch 1,
   die eine Einrichtung zum Erfassen eines ein erfolgtes Sensor-Ereignis charakterisierenden elektrischen Parameters aufweist, die mit einem ersten Source-/Drain-Anschluss des Mess-Transistors gekoppelt ist.

3. Schaltkreis-Anordnung nach Anspruch 2,
   bei der die Kalibrier-Einrichtung derart eingerichtet ist, dass mit ihr das an den ersten oder einen zweiten Source-/-Drain-Anschluss des Mess-Transistors angelegte elektrische Potential derart steuerbar ist, dass durch sie ein von einem Sensor-Ereignis bewirktes Sensor-Signal des Sensor-Elements auf einen Wert einstellbar ist, der von dem Wert des physikalischen Parameters des Sensor-Elements unabhängig ist.

4. Schaltkreis-Anordnung nach Anspruch 3,
   bei der die Kalibrier-Einrichtung derart eingerichtet ist, dass mit ihr das an dem ersten Source-/Drain-Anschluss des Mess-Transistors anliegende elektrische Potential steuerbar ist.

5. Schaltkreis-Anordnung nach Anspruch 4,
   bei der an den zweiten Source-/Drain-Anschluss des Mess-Transistors ein erstes elektrisches Referenz-Potential anlegbar ist und bei der die Kalibrier-Einrichtung einen Kalibrier-Transistor mit einem ersten und einem zweiten Source-/Drain-Anschluss aufweist, welche Source-/Drain-Anschlüsse zwischen den ersten Source-/Drain-Anschluss des Mess-Transistors und die Einrichtung zum Erfassen eines elektrischen Parameters geschaltet sind, und an dessen Gate-Anschluss ein derartiges elektrisches Signal anlegbar ist, dass das an den ersten Source-/Drain-Anschluss des Mess-Transistors anlegbare elektrische Potential derart einstellbar ist, dass die individuelle Abweichung des Werts des physikalischen Parameters des Sensor-Elements von einem Referenzwert zumindest teilweise kompensierbar ist.

6. Schaltkreis-Anordnung nach Anspruch 4,
   bei der die Kalibrier-Einrichtung derart eingerichtet ist, dass mit ihr das an dem zweiten Source-/Drain-Anschluss des Mess-Transistors anliegende elektrische Potential steuerbar ist.

7. Schaltkreis-Anordnung nach Anspruch 6,
   bei welcher der erste Source-/Drain-Anschluss des Mess-Transistors mit der Einrichtung zum Erfassen eines elektrischen Parameters gekoppelt ist, und bei der die Kalibrier-Einrichtung einen Kalibrier-Transistor aufweist, der einen mit dem zweiten Source-/Drain-Anschluss des Mess-Transistors gekoppelten ersten Source-/Drain-Anschluss und einen zweiten Source-/Drain-Anschluss aufweist, an den ein zweites elektrisches Referenz-Potential anlegbar ist, und an dessen Gate-Anschluss ein derartiges elektrisches Signal anlegbar ist, dass das an den zweiten Source-/Drain-Anschluss des Mess-Transistors anlegbare elektrische Potential derart einstellbar ist, dass die individuelle Abweichung des Werts des physikalischen Parameters des Sensor-Elements von einem Referenzwert zumindest teilweise kompensierbar ist.

8. Schaltkreis-Anordnung nach Anspruch 6,
   bei der die Kalibrier-Einrichtung aufweist:

   • einen Kalibrier-Transistor;
   • eine erste Konstantstromquelle, die mit jeweiligen zweiten Source-/Drain-Anschlüssen der zueinander parallel geschalteten Mess- und Kalibrier-Transistoren gekoppelt ist, zum Bereitstellen einer vorgebbaren elektrischen Stromstärke;

• eine Stromspiegel-Schaltung, die mit jeweiligen ersten Source-/Drain-Anschlüssen der zueinander parallel geschalteten Mess- und Kalibrier-Transistoren gekoppelt ist, die derart verschaltet ist, dass mit ihr zum zumindest teilweisen Kompensieren der individuellen Abweichung des Werts des physikalischen Parameters von einem Referenzwert das elektrische Potential am Gate-Anschluss des Kalibrier-Transistors derart einstellbar ist, dass in Abwesenheit eines Sensor-Ereignisses die Stromflüsse zwischen den beiden Source-/Drain-Anschlüssen des Mess-Transistors und des Kalibrier-Transistors gleich sind.

**9.** Schaltkreis-Anordnung nach Anspruch 6,
bei welcher an den ersten Source-/Drain-Anschluss des Mess-Transistors ein drittes elektrisches Referenz-Potential angelegt ist und bei der die Kalibrier-Einrichtung aufweist:

• einen Kalibrier-Transistor mit einem ersten und einem zweiten Source-/Drain-Anschluss;
• eine zweite Konstantstromquelle, die mit den jeweiligen zweiten Source-/Drain-Anschlüssen der zueinander parallel geschalteten Mess- und Kalibrier-Transistoren gekoppelt ist, zum Bereitstellen einer vorgebbaren elektrischen Stromstärke;
• eine dritte Konstantstromquelle, die mit dem ersten Source-/Drain-Anschluss des Kalibrier-Transistors gekoppelt ist, zum Bereitstellen einer weiteren vorgebbaren elektrischen Stromstärke, welche dritte Konstantstromquelle derart verschaltet ist, dass mit ihr zum zumindest teilweisen Kompensieren der individuellen Abweichungung des Werts des physikalischen Parameters von einem Referenzwert die an den Anschlüssen der Transistoren anlegbaren Potentiale derart einstellbar sind, dass in Abwesenheit eines Sensor-Ereignisses die Stromflüsse zwischen den beiden Source-/Drain-Anschlüssen des Mess-Transistors und des Kalibrier-Transistors gleich sind.

**10.** Schaltkreis-Anordnung nach Anspruch 2,
bei der die Kalibrier-Einrichtung derart eingerichtet ist, dass mit ihr ein von einem Sensor-Ereignis bewirktes Sensor-Signal des Sensor-Elements unter Verwendung des Prinzips der korrelierten Doppelabtastung in einen Wert umwandelbar ist, der von dem Wert des physikalischen Parameters des Sensor-Elements unabhängig ist.

**11.** Schaltkreis-Anordnung nach Anspruch 10,
bei der an einen zweiten Source-/Drain-Anschluss des Mess-Transistors ein viertes elektrisches Referenz-Potential anlegbar ist, und bei der die Kalibrier-Einrichtung

• eine elektrische Subtrahier-Einrichtung mit zwei Eingängen und einem Ausgang aufweist, welcher Ausgang mit der Einrichtung zum Erfassen eines elektrischen Parameters gekoppelt ist, welcher erste Eingang mit dem ersten Source-/Drain-Anschluss des Mess-Transistors gekoppelt ist, und welche elektrische Subtrahier-Einrichtung derart eingerichtet ist, dass an ihrem Ausgang die Differenz zwischen zwei an den beiden Eingängen angelegten elektrischen Signalen bereitstellbar ist;
• ein zwischen den ersten Source-/Drain-Anschluss des Mess-Transistors und den zweiten Eingang der elektrischen Subtrahier-Einrichtung geschaltetes Abtast-Halte-Glied aufweist;
• derart eingerichtet ist, dass

○ in einem ersten Betriebszustand in das Abtast-Halte-Glied ein von dem physikalischen Parameter des Sensor-Elements abhängiges.Sensor-Signal einprägbar ist und dem zweiten Eingang der elektrischen Subtrahier-Einrichtung bereitstellbar ist;
○ in einem zweiten Betriebszustand dem ersten Eingang der elektrischen Subtrahier-Einrichtung ein für den physikalischen Parameter des Sensor-Elements charakteristisches Signal bereitstellbar ist;
○ an dem Ausgang der elektrischen Subtrahier-Einrichtung ein von dem Wert des physikalischen Parameters des Sensor-Elements unabhängiges Sensor-Signal bereitstellbar ist, wodurch die individuelle Abweichung des Werts des physikalischen Parameters von einem Referenzwert zumindest teilweise kompensiert ist.

**12.** Schaltkreis-Anordnung nach einem der Ansprüche 2 bis 11, bei welcher der elektrische Parameter

• eine elektrische Spannung oder
• ein elektrischer Strom

ist.

13. Schaltkreis-Anordnung nach einem der Ansprüche 1 bis 12, bei der die Sensor-Elektrode eines oder eine Kombination der Materialien

  • Titan
  • Titannitrid
  • Gold und
  • Platin aufweist.

14. Schaltkreis-Anordnung nach einem der Ansprüche 1 bis 13, die ein Verstärker-Element zum Verstärken eines Sensor-Signals aufweist.

15. Schaltkreis-Anordnung nach einem der Ansprüche 1 bis 14, die eine Schalt-Einrichtung aufweist, die derart eingerichtet ist, dass mit dieser das Sensor-Element wahlweise mit einem fünften elektrischen Referenz-Potential gekoppelt oder von diesem entkoppelt ist, um das Sensor-Element vor einer Schädigung zu schützen und/oder um an das Sensor-Element ein definiertes elektrisches Potential anzulegen.

16. Schaltkreis-Anordnung nach einem der Ansprüche 1 bis 15, bei der das Substrat ein Silizium-Substrat ist.

17. Schaltkreis-Anordnung nach einem der Ansprüche 1 bis 16, bei der die Art der Kopplung zwischen dem Sensor-Element und der zu untersuchenden Flüssigkeit einen kapazitiven Anteil aufweist.

18. Sensor-Array mit einer Mehrzahl von in Kreuzungsbereichen von Zeilen- und Spalten-Leitungen im Wesentliche matrixförmig angeordneten und mit den Zeilen- und Spalten-Leitungen verschalteten Schaltkreis-Anordnungen nach einem der Ansprüche 1 bis 17.

19. Sensor-Array nach Anspruch 18,
bei dem zumindest ein Teil der Schaltkreis-Anordnungen ein mit der jeweils zugehörigen Zeilen-Leitung und/oder Spalten-Leitung gekoppeltes Auswahl-Element zum Auswählen der jeweiligen Sensor-Anordnung aufweist, um ein Sensor-Signal des Sensor-Elements der ausgewählten Schaltkreis-Anordnung zu erfassen und/oder um bei der ausgewählten Schaltkreis-Anordnung die individuelle Abweichung des Werts des physikalischen Parameters zumindest teilweise zu kompensieren und/oder um an das Sensor-Element der ausgewählten Schaltkreis-Anordnung das fünfte elektrische Potential anzulegen.

20. Sensor-Array nach Anspruch 18 oder 19,
bei dem zumindest ein Teil der einer jeweiligen Zeilen- und/oder Spalten-Leitung zugeordneten Schaltkreis-Anordnungen

  • eine gemeinsame Einrichtung zum Erfassen eines ein erfolgtes Sensor-Ereignis charakterisierenden elektrischen Parameters,
  • eine gemeinsame Konstantstromquelle,
  • eine gemeinsame Schalt-Einrichtung,
  • ein gemeinsames Referenz-Potential,
  • einen gemeinsamen Strom-Spannungs-Wandler,
  • einen gemeinsamen Analog-/Digital-Wandler,
  • einen gemeinsamen Stromspiegel,
  • eine gemeinsame Subtrahier-Einrichtung,
  • ein gemeinsames Abtast-Halte-Glied, und/oder
  • einen gemeinsamen Verstärker,

aufweist.

21. Sensor-Array nach einem der Ansprüche 18 bis 20,
bei dem zumindest ein Teil der Zeilen- und/oder Spalten-. Leitungen jeweils eine Einrichtung zum Erfassen eines ein erfolgtes Sensor-Ereignis charakterisierenden elektrischen parameters aufweist, wobei das Sensor-Array derart eingerichtet ist, dass mittels der einer jeweiligen Zeilen- oder Spalten-Leitung zugeordneten Einrichtung zum Erfassen eines elektrischen Parameters

• ein Sensor-Signal genau einer Sensor-Anordnung der jeweiligen Zeilen- oder Spalten-Leitung,
• oder eine Summe von Sensor-Signalen von zumindest einem Teil der Sensor-Anordnungen der jeweiligen Zeilen- oder Spalten-Leitung

erfassbar ist.

22. Sensor-Array nach einem der Ansprüche 18 bis 21,
bei dem zumindest ein Teil der Spalten-Leitungen mit einer Potentialsteuer-Einrichtung gekoppelt sind, welche Potentialsteuer-Einrichtung derart eingerichtet ist, dass sie das elektrische Potential der zugehörigen Spalten-Leitung auf einem im Wesentlichen konstanten Wert hält.

23. Biosensor-Array mit einem Sensor-Array nach einem der Ansprüche 18 bis 22.

**Claims**

1. A circuit arrangement (203) comprising:

   a substrate;
   a sensor element formed in or on a surface region of the substrate with a physical parameter, which is coupled to a substance to be examined, the type of coupling having a resistive component, the sensor element having an electrically conductive sensor electrode (204) that is coupled to the substance to be examined, the sensor element having a measuring transistor (205), the gate terminal (205a) of which being coupled to the electrically conductive sensor electrode, and the physical parameter being the threshold voltage of the measuring transistor;
   a calibration device formed in or on the substrate, said calibration device being set up such that it is used to at least partly compensate for an individual deviation of the value of the physical parameter of the sensor element from a reference value.

2. The circuit arrangement as claimed in claim 1,
comprising a device for detecting an electrical parameter characterizing an effected sensor event, the device being coupled to a first source/drain terminal of the measuring transistor.

3. The circuit arrangement as claimed in claim 2,
wherein the calibration device is set up such that it is used to control the electrical potential applied to the first or a second source/drain terminal of the measuring transistor in such a way that it can set a sensor signal of the sensor element, said sensor signal being caused by a sensor event, to a value which is independent of the value of the physical parameter of the sensor element.

4. The circuit arrangement as claimed in claim 3,
wherein the calibration device is set up such that it is used to control the electrical potential present at the first source/ drain terminal of the measuring transistor.

5. The circuit arrangement as claimed in claim 4,
wherein a first electrical reference potential can be applied to the second source/drain terminal of the measuring transistor, and wherein the calibration device has a calibration transistor having a first and a second source/drain terminal, which are connected between the first source/drain terminal of the measuring transistor and the device for detecting an electrical parameter, and wherein to the gate terminal thereof, an electrical signal can be applied such that the electrical potential which can be applied to the first source/drain terminal of the measuring transistor can be set such that the individual alteration of the value of the physical parameter of the sensor element from a reference value can at least partly be compensated for.

6. The circuit arrangement as claimed in claim 4,
wherein the calibration device is set up such that it is used to control the electrical potential present at the second source/drain terminal of the measuring transistor.

7. The circuit arrangement as claimed in claim 6,
wherein the first source/drain terminal of the measuring transistor is coupled to the device for detecting an electrical parameter, and wherein the calibration device has a calibration transistor having a first source/drain terminal, which is coupled to the second source/drain terminal of the measuring transistor, and a second source/drain terminal, to which a second electrical reference potential can be applied, and to the gate terminal of which an electrical signal can be applied such that the electrical potential which can be applied to the second source/drain terminal of the measuring transistor can be set such that the individual alteration of the value of the physical parameter of the sensor element from a reference value can at least partly be compensated for.

**8.** The circuit arrangement as claimed in claim 6,
wherein the calibration device comprises:

• a calibration transistor;
• a first constant-current source, which is coupled to respective second source/drain terminals of the measuring and calibration transistors that are connected in parallel with one another, for the provision of a predeterminable electrical current intensity;
• a current mirror circuit, which is coupled to respective first source/drain terminals of the measuring and calibration transistors that are connected in parallel with one another, which is connected such that it is used to set, for at least partly compensating for the individual alteration of the value of the physical parameter of a reference value, the electrical potential at the gate terminal of the calibration transistor such that, in the absence of a sensor event, the current flows between the two source/drain terminals of the measuring transistor and of the calibration transistor are identical.

**9.** The circuit arrangement as claimed in claim 6,
wherein a third electrical reference potential is applied to the first source/drain terminal of the measuring transistor, and wherein the calibration device comprises:

a calibration transistor having a first and a second source/ drain terminal;
a second constant-current source, which is coupled to the respective second source/drain terminals of the measuring and calibration transistors that are connected in parallel with one another, for the provision of a predeterminable electrical current intensity;
a third constant-current source, which is coupled to the first source/drain terminal of the calibration transistor, for the provision of a further predeterminable electrical current intensity, the third constant-current source being connected such that it is used to set, for at least partly compensating for the individual alteration of the value of the physical parameter from a reference value, the potentials that can be applied to the terminals of the transistors such that, in the absence of a sensor event, the current flows between the two source/drain terminals of the measuring transistor and of the calibration transistor are identical.

**10.** The circuit arrangement as claimed in claim 2,
wherein the calibration device is set up such that it is used to convert a sensor signal of the sensor element, said sensor signal being brought about by a sensor event, using a principle of correlated double sampling to a value which is independent of the value of the physical parameter of the sensor element.

**11.** The circuit arrangement as claimed in claim 10,
wherein a fourth electrical reference potential can be applied to a second source/drain terminal of the measuring transistor and wherein the calibration device comprises:

• an electrical subtraction device having two inputs and an output, which is coupled to the device for detecting an electrical parameter, a first one of the two inputs is coupled to the first source/drain terminal of the measuring transistor, and the electrical subtraction device is set up such that a difference between two electrical signals applied to the two inputs can be provided at its output;
• a sample-and-hold element connected between the first source/drain terminal of the measuring transistor and the second input of the electrical subtraction device; and
• wherein the calibration device is set up such that:

• in a first operating state, a sensor signal dependent on the physical parameter of the sensor element can be impressed into the sample-and-hold element and can be provided to the second input of the electrical subtraction device;
• in a second operating state, a signal which is characteristic of the physical parameter of the sensor element can be provided to the input of the electrical subtraction device;
• a sensor signal independent of the value of the physical parameter of the sensor element can be provided at the output of the electrical subtraction device, as a result of which the individual alteration of the value of the physical parameter from a reference value is at least partly compensated for.

**12.** The circuit arrangement as claimed in one of claims 2 to 11, wherein the electrical parameter is

- an electrical voltage or
- an electric current.

**13.** The circuit arrangement as claimed in one of claims 1 to 12, wherein the sensor electrode has one or a combination of the materials

- titanium
- titanium nitride
- gold and
- platinum.

**14.** The circuit arrangement as claimed in one of claims 1 to 13, comprising an amplifier element for amplifying a sensor signal.

**15.** The circuit arrangement as claimed in one of claims 1 to 14, comprising a switching device set up such that it is used optionally to couple the sensor element to a fifth electrical reference potential or to decouple it from the latter, in order to protect the sensor element from damage and/or to apply a defined electrical potential to the sensor element.

**16.** The circuit arrangement as claimed in one of claims 1 to 15, wherein the substrate is a silicon substrate.

**17.** The circuit arrangement as claimed in one of claims 1 to 16, wherein a type of coupling between the sensor element and the liquid to be examined has a capacitive component.

**18.** A sensor array having a plurality of circuit arrangements as claimed in one of claim 1 to 17, wherein said circuit arrangements are arranged essentially in matrix form in crossover regions of row and column lines and being connected up to the row and column lines.

**19.** The sensor array as claimed in claim 18, wherein at least a portion of the circuit arrangements have a selection element - coupled to the respectively associated row line and/or column line - for selection of the respective sensor arrangement to detect a sensor signal of the sensor element of the selected circuit arrangement and/or, in the case of the selected circuit arrangement, at least partly to compensate for the individual alteration of the value of the physical parameter and/or to apply the fifth electrical potential to the sensor element of the selected circuit arrangement.

**20.** The sensor array as claimed in one of claims 18 or 19, wherein at least a portion of the circuit arrangements assigned to a respective row line and/or column line comprises

- a common device for detecting an electrical parameter that characterizes an effected sensor event,
- a common constant-current source,
- a common switching device,
- a common reference potential,
- a common current-voltage converter,
- a common analog-digital converter,
- a common current mirror,
- a common subtraction device,
- a common sample-and-hold element,
- a common amplifier.

**21.** The sensor array as claimed in one of claims 18 to 20, wherein at least a portion of row lines and/or column lines respectively comprises a device for detecting an electrical parameter that characterizes an effected sensor event, wherein the sensor array is set up such that the device for detecting an electrical parameter assigned to a respective line or column line can be used for detecting

• a sensor signal of exactly one sensor arrangement of the respective line or column line,

• or a sum of sensor signals of at least a portion of the sensor arrangements of the respective line or column line.

**22.** A sensor array as claimed in one of claims 18 to 21,
wherein at least a portion of the column lines is coupled to a potential control device, which is set up such that it maintains the electrical potential of the respective column line at an essentially constant value

**23.** A biosensor array having a sensor array as claimed in one of claims 18 to 22.

**Revendications**

**1.** Montage (203)

• comprenant un substrat ;
• comprenant un élément de capteur, qui est formé dans ou sur une partie de surface du substrat et qui a un paramètre physique qui est couplé à une substance à rechercher, le type du couplage ayant une partie ohmique, l'élément de capteur comportant une électrode (204) de capteur, conductrice de l'électricité, couplée à la substance à rechercher, l'élément de capteur comportant un transistor (205) de mesure, dont la borne (205a) de grille est couplée à l'électrode de capteur conductrice de l'électricité et le paramètre physique est la tension de seuil du transistor de mesure ;
• comprenant un dispositif d'étalonnage formé dans ou sur le substrat et tel que, par lui, un écart individuel de la valeur du paramètre physique de l'élément de capteur à une valeur de référence, peut être compensé au moins en partie.

**2.** Montage suivant la revendication 1,
qui comporte un dispositif de détection d'un paramètre électrique caractérisant un évènement de capteur qui se produit, ce dispositif étant couplé à une première borne de source/drain du transistor de mesure.

**3.** Montage suivant la revendication 2,
dans lequel le dispositif d'étalonnage est tel que le potentiel électrique appliqué à la première ou à une deuxième borne de source/drain du transistor de mesure peut être réglé, de manière à ce qu'un signal de capteur de l'élément de capteur provoqué par un évènement de capteur puisse être réglé, par lui, à une valeur qui est indépendante de la valeur du paramètre physique de l'élément de capteur.

**4.** Montage suivant la revendication 3,
dans lequel le dispositif d'étalonnage est tel que le potentiel électrique s'appliquant à la première borne de source/drain du transistor de mesure peut être réglé par lui.

**5.** Montage suivant la revendication 4,
dans lequel un premier potentiel de référence électrique peut être appliqué à la deuxième borne de source/drain du transistor de mesure et dans lequel le dispositif d'étalonnage comporte un transistor d'étalonnage ayant une première et une deuxième bornes de source/drain, lesquelles bornes de source/drain sont montées entre la première borne de source/drain du transistor de mesure et le dispositif de détection d'un paramètre électrique et à sa borne de grille peut être appliqué un signal électrique tel que le potentiel électrique qui peut s'appliquer à la première borne de source/drain du transistor de mesure peut être réglé, de manière à ce que l'écart individuel de la valeur du paramètre physique de l'élément de capteur à une valeur de référence peut être compensé au moins en partie.

**6.** Montage suivant la revendication 4,
dans lequel le dispositif d'étalonnage est tel que le potentiel électrique s'appliquant à la deuxième borne de source/drain du transistor de mesure peut être réglé par lui.

**7.** Montage suivant la revendication 6,
dans lequel la première borne de source/drain du transistor de mesure est couplée au dispositif de détection d'un paramètre électrique et dans lequel le dispositif d'étalonnage comporte un transistor d'étalonnage, qui a une première borne de source/drain couplée à la deuxième borne de source/drain du transistor de mesure et une deuxième borne de source/drain à laquelle peut être appliquée un deuxième potentiel de référence électrique et à la borne de grille duquel peut être appliquée un signal électrique tel que le potentiel électrique pouvant s'appliquer à la deuxième borne de source/drain du transistor de mesure peut être réglé, de manière à pouvoir compenser au moins en partie l'écart individuel de la valeur du paramètre physique de l'élément de capteur à une valeur de référence.

8. Montage suivant la revendication 6,
dans lequel le dispositif d'étalonnage comprend :

• un transistor d'étalonnage ;
• une première source de courant constant, qui est couplée à respectivement des deuxièmes bornes de source/drain, des transistors de mesure et d'étalonnage montés en parallèle l'un avec l'autre, pour disposer d'une intensité de courant électrique pouvant être prescrite ;
• un circuit de miroir de courant, qui est couplé à des premières bornes de source/drain respectives, des transistors de mesure et d'étalonnage montés entre eux en parallèle, le circuit de miroir de courant étant tel que, par lui, pour compenser au moins en partie l'écart individuel de la valeur du paramètre physique à une valeur de référence, le potentiel électrique à la borne de grille du transistor d'étalonnage peut être réglé, de manière à ce que, en l'absence d'un événement de capteur, les flux de courant entre les deux bornes de source/drain du transistor de mesure et du transistor d'étalonnage sont égaux.

9. Montage suivant la revendication 6,
dans lequel à la première borne de source/drain du transistor de mesure est appliqué un troisième potentiel de référence électrique et dans lequel le dispositif d'étalonnage comprend :

• un transistor d'étalonnage ayant une première et une deuxième bornes de source/drain ;
• une deuxième source de courant constant, qui est couplée au deuxième borne de source/drain respective des transistors de mesure et d'étalonnage montés entre eux en parallèle, pour mettre à disposition une intensité de courant électrique pouvant être prescrite.
• une troisième source de courant constant, qui est couplée à la première borne de source/drain du transistor d'étalonnage, pour mettre à disposition une autre intensité de courant électrique pouvant être prescrite, laquelle troisième source de courant constant est montée de manière à ce que, par elle, pour compenser au moins en partie l'écart individuel de la valeur du paramètre physique à une valeur de référence, les potentiels, pouvant être appliqués aux bornes des transistors, peuvent être réglés de manière à ce que, en l'absence d'un évènement de capteur, les flux de courant entre les deux bornes de source/drain du transistor de mesure et du transistor d'étalonnage soient égaux.

10. Montage suivant la revendication 2,
dans lequel le dispositif d'étalonnage est tel que, par lui, un signal de capteur de l'élément de capteur provoqué par un élément de capteur, peut être transformé en utilisant le principe du double échantillonnage corrélé en une valeur qui est indépendante de la valeur du paramètre physique de l'élément de capteur.

11. Montage suivant la revendication 10,
dans lequel à une deuxième borne de source/drain du transistor de mesure peut être appliquée un quatrième potentiel de référence électrique et dans lequel le dispositif d'étalonnage comporte

• un dispositif électrique de soustraction comprenant deux entrées et une sortie, laquelle sortie est couplée au dispositif de détection d'un paramètre électrique, laquelle première entrée est couplée à la première borne de source/drain du transistor de mesure et lequel dispositif de soustraction est tel qu'il peut être mis à disposition, à sa sortie, la différence entre deux signaux électriques appliqués aux deux entrées ;
• un élément d'interrogation et de maintien monté entre la première borne de source/drain du transistor de mesure et la deuxième entrée du dispositif électrique de soustraction ;
• tel que

○ dans un premier état de fonctionnement, un signal de capteur dépendant du paramètre physique de l'élément de capteur, peut être injecté dans l'élément d'interrogation et de maintien et peut être mis à disposition de la deuxième entrée du dispositif électrique de soustraction ;
○ dans un deuxième état de fonctionnement, un signal caractéristique du paramètre physique de l'élément de capteur peut être mis à disposition de la première entrée du dispositif électrique de soustraction ;
○ un signal de capteur indépendant de la valeur du paramètre physique de l'élément de capteur peut être mis à disposition à la sortie du dispositif électrique de soustraction, de sorte que l'écart individuel de la valeur du paramètre physique à une valeur de référence soit compensé au moins en partie.

**12.** Montage suivant l'une des revendications 2 à 11,
dans lequel le paramètre électrique est

    • une tension électrique ou
    • un courant électrique.

**13.** Montage suivant l'une des revendications 1 à 12,
dans lequel l'électrode de capteur comprend l'un ou une combinaison des matériaux

    • titane,
    • nitrure de titane,
    • or et
    • platine

**14.** Montage suivant l'une des revendications 1 à 13, qui comporte un élément amplificateur, pour amplifier un signal de capteur.

**15.** Montage suivant l'une des revendications 1 à 14, qui comporte un dispositif de commutation, tel que, par lui, l'élément de capteur peut être couplé, au choix, à un cinquième potentiel de référence électrique ou en être découplé, pour protéger l'élément de capteur d'un endommagement et/ou pour appliquer à l'élément de capteur un potentiel électrique défini.

**16.** Montage suivant l'une des revendications 1 à 15,
dans lequel le substrat est un substrat en silicium.

**17.** Montage suivant l'une des revendications 1 à 16,
dans lequel le type du couplage entre l'élément de capteur et le liquide à rechercher comporte une partie capacitive.

**18.** Réseau de capteurs comprenant une multiplicité de conducteurs de ligne et de colonne dans des parties de croisement de montages disposés sensiblement en forme de matrice et câblés par les conducteurs de ligne et de colonne, suivant l'une des revendications 1 à 17.

**19.** Réseau de capteurs suivant la revendication 18,
dans lequel au moins une partie des montages comporte un élément de sélection couplé à respectivement le conducteur de ligne associé/ou le conducteur de colonne associé, pour sélectionner le dispositif de capteur respectif, afin de détecter un signal de capteur de l'élément de capteur du montage sélectionné et/ou, afin de compenser au moins en partie, pour le montage sélectionné, l'écart individuel de la valeur du paramètre physique et/ou afin d'appliquer à l'élément de capteur du montage sélectionné, le cinquième potentiel électrique.

**20.** Réseau de capteurs suivant la revendication 18 ou 19,
dans lequel au moins une partie des montages associés à un conducteur respectif de ligne et/ou de colonne comporte

    • un dispositif commun de détection d'un paramètre électrique caractérisant un évènement de capteur qui se produit,
    • une source de courant constante commun,
    • un dispositif de commutation commun,
    • un potentiel de référence commun,
    • un convertisseur courant-tension commun,
    • un convertisseur analogique/numérique commun,
    • un miroir de courant commun,
    • un dispositif commun de soustraction,
    • un élément d'interrogation et de maintien commun et/ou
    • un amplificateur commun.

**21.** Réseau de capteurs suivant l'une des revendications 18 à 20,
dans lequel au moins une partie des conducteurs de ligne et/ou de colonne comporte respectivement un dispositif de détection d'un paramètre électrique caractérisant un évènement de capteur qui se produit, le réseau de capteurs étant tel qu'au moyen du dispositif associé à un conducteur respectif de ligne ou de colonne et destiné un paramètre électrique, il est possible de détecter

  • un signal de capteur, précisément d'un dispositif de capteur du conducteur de ligne ou de colonne respectif,
  • ou une somme de signaux de capteur d'au moins une partie des dispositifs de capteur du conducteur respectif de ligne ou de colonne.

22. Réseau de capteurs suivant l'une des revendications 18 à 21,
dans lequel au moins une partie des conducteurs de colonne sont couplés à un dispositif de commande de potentiel, lequel dispositif de commande de potentiel est tel qu'il maintient le potentiel électrique du conducteur de colonne associé à une valeur sensiblement constante.

23. Réseau de biocapteurs, comprenant un réseau de capteurs suivant l'une des revendications 18 à 22.

FIG 1    Stand der Technik

# FIG 2

# FIG 3

# FIG 4

# FIG 5

FIG 6

FIG 7

FIG 8

# FIG 9

## FIG 10

# FIG 11

# FIG 12

# FIG 13

FIG 14

# FIG 15

1502

1308c

1308

1308a    1308b

1309

1316

1501

1303

1313    1312

1305b

1310    1305

1305a    1307

1304    1311    1305c

1302

Vcol=const

1301a

1301b

1301c

1301d

1500

Vcol=const

# FIG 16

## FIG 17

# FIG 18

FIG 19

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0175462 A
- US 5602467 A

- DE 4320881 A1

**In der Beschreibung aufgeführte Nicht-Patentliteratur**

- **Krause, M et al.** Extended gate electrode arrays for extracellular signal recordings. *Sensor and Actuators B,* vol. 70, 101-107
- **Bauman, W. H. et al.** Microelectronic sensor system for microphysiological application on living cells. *Sensors and Actuators B,* 1999, vol. 55, 77-89
- **Thomas, CA et al.** A miniature microelectrode array to monitor the bioelectric activity of cultured cells. *Exp.Cell.Res.,* 1972, vol. 74, 61-66
- **Gross, GW et al.** The use of neuronal networks on multielectrode arrays as biosensors. *Biosensors&Bioelectronics,* 1995, vol. 10, 553-567

- **Berdondini, L et al.** High-Density MEA for Electrophysiological Activity Imaging of Neuronal Networks. *Proc. ICECS 2001,* September 2001, 1239-1242
- **Enz, CC et al.** Circuit techniques for reducing the effects of op-amp imperfections: autozeroing, correlated double sampling, and chopper stabilization. *Proceedings of the IEEE,* 1996, vol. 84 (11), 1584ff